# EUROPEAN PATENT SPECIFICATION

(11) **EP 4 431 604 B1**
(45) Date of publication and mention of the grant of the patent: **11.11.2026**
(21) Application number: 24186809.0
(22) Date of filing: 15.04.2020
(51) Int. Cl.: C12N 15/10

(54) **MULTIPLEX ASSEMBLY OF NUCLEIC ACID MOLECULES**
MULTIPLEXANORDNUNG VON NUKLEINSÄUREMOLEKÜLEN
ASSEMBLAGE MULTIPLEX DE MOLÉCULES D'ACIDES NUCLÉIQUES

(30) Priority: 15.04.2019 GB 201905303
(43) Date of publication of application: 18.09.2024
(62) Document number(s) of the earlier application(s) in accordance with Art. 76 EPC: 20719413.5 / 3 956 445
(73) Proprietor: Thermo Fisher Scientific GENEART GmbH, 93059 Regensburg (DE)
(72) Inventor: KUHN, Phillip, Regensburg (DE); HOFMEISTER, Thomas, Regensburg (DE); NETUSCHIL, Nikolai, Regensburg (DE); PUETZSCHLER, Joern, Regensburg (DE); KONOVALOVA, Tatiana, Regensburg (DE)
(74) Representative: Ludwig, Christine Andrea

(56) References cited:
- WO-A1-2006/044956
- WO-A1-2018/022972
- WO-A1-2020/001783
- WO-A2-2008/054543
- US-A1- 2009 305 233
- US-A1- 2013 059 296

## Description

### FIELD OF THE INVENTION

The present disclosure generally relates to compositions and methods for the synthesis of nucleic acid molecules. Provided, as examples, are compositions and methods for high throughput synthesis and multiplex assembly of nucleic acid molecules, in many instances, with high sequence fidelity. Disclosed compositions and methods allow, in part, for miniaturization, parallelization, high throughput production and scaling, and cost reduction in gene synthesis workflows.

### BACKGROUND OF THE INVENTION

Over the years gene synthesis has become more cost effective and efforts to develop high throughput synthesis platforms and miniaturize certain workflows offers new applications and market opportunities

With progress in genetic engineering, a need for the generation of larger nucleic acid molecules has developed. In many instances, nucleic acid assembly methods start with the synthesis of relatively short nucleic acid molecules (*e.g.,* chemically or enzymatically synthesized oligonucleotides), followed by the generation of double-stranded nucleic acid fragments or sub-assemblies (*e.g.,* by annealing and elongating and/or ligating multiple overlapping oligonucleotides), and often proceeds to build larger assemblies such as genes, operons or even functional biological pathways (*e.g.,* by ligation, enzymatic elongation, recombination or a combination thereof).

Next generation gene synthesis techniques benefit from miniaturization of oligonucleotide synthesis and the ability to perform successive reaction steps. Further, reagent usage may be decreased, which currently contributes significantly to costs, by several orders of magnitude as compared to standard controlled pore glass (CPG) bead based oligonucleotide synthesis methods.

High throughput and small scale gene synthesis can be achieved by using microarray or chip-based oligonucleotide synthesis systems, which are capable of producing 10,000 to 1 million oligonucleotides per chip. The oligonucleotides are then released from the chip (*e.g.,* by elution, cleavage or amplification) and assembled into larger nucleic acid fragments. However, since all oligonucleotides are typically synthesized on the same common support, they can only be recovered as a highly complex pool of sequences. Hence, the oligonucleotide density on a chip may be limited (*e.g.,* to thousands of different sequences per chip) to balance yield per oligonucleotide and complexity. Furthermore, in a complex pool of oligonucleotides the probability for cross-hybridizations between oligonucleotides belonging to different assembly products increases thereby limiting the success rate of correctly assembled nucleic acid fragments. Different protocols were developed to handle the complexity of chip-derived oligonucleotide pools for downstream assembly workflows. However, the current approaches are difficult to scale, error prone and inefficient.

WO2018/022972A1 relates to devices and methods for multiplex nucleic acid assembly. US2009/305233A1 relates to methods, compositions, and kits for polynucleotide assembly.

The present invention seeks to overcome these weaknesses by providing improved compositions, methods and rules for multiplex nucleic acid assembly and processing to facilitate production workflows and optimize scalability of high throughput gene synthesis.

### SUMMARY OF THE INVENTION

The present invention is defined by the appended claims. The present disclosure relates, in part, to compositions and methods for high throughput synthesis and assembly of nucleic acid molecules. Particular aspects of the disclosure include methods and rules for designing, grouping and pooling of nucleic acid molecules for efficient multiplex assembly, amplification and subsequent processing and analysis.

In a first aspect the present disclosure provides methods for multiplex assembly of two or more predefined nucleic acid fragments in multiple reaction compartments comprising the following steps: (a) designing a plurality of oligonucleotide sequences together comprising the sequences of the two or more predefined nucleic acid fragments and synthesizing the oligonucleotides to obtain a plurality of single-stranded oligonucleotides, (b) selectively retrieving a first and a second and optionally one or more further sub-set of the plurality of single-stranded oligonucleotides, wherein the oligonucleotides of each sub-set are components of two or more predefined nucleic acid fragments, and wherein all oligonucleotides belonging to one of the two or more predefined nucleic acid fragments share a region of sequence complementarity with at least one other oligonucleotide belonging to the same nucleic acid fragment, (c) pooling the oligonucleotides of the first sub-set into a first reaction compartment, the second sub-set into a second reaction compartment and optionally pooling the one or more further sub-sets into one or more further reaction compartments, and (d) simultaneously assembling under suitable conditions the two or more nucleic acid fragments in the first, second and optionally further reaction compartments. In some instances, the two or more predefined nucleic acid fragments may comprise at least three or more predefined nucleic acid fragments.

In some instances, the plurality of single-stranded oligonucleotides is synthesized on a common carrier such as an array or a microchip. The single-stranded oligonucleotides may be bound to individual supports wherein each individual support may be located at an addressable position of the common carrier. Further, some or all of the plurality of oligonucleotides may be synthesized using electrochemically generated acid or photochemically generated acid. Further, some or all of the plurality of oligonucleotides may be synthesized using phosphoramidite chemistry or may be synthesized enzymatically. In some instances, the oligonucleotides may be provided phosphorylated. Additionally, the one or more supports may be beads and the plurality of oligonucleotides may be synthesized on such beads. In some instances, the oligonucleotides may be released from the pooled supports and optionally separated from the supports prior to assembly. Thus, in some aspects, the selectively retrieving and pooling of sub-sets of oligonucleotides comprises selectively retrieving and pooling individual supports to which the oligonucleotides are bound and releasing the oligonucleotides from the pooled supports and separating the oligonucleotides from the supports prior to assembling the two or more nucleic acid fragments.

In some instances, the sequences of the two or more (*e.g.,* from about two to about twenty, from about three to about twenty, from about four to about twenty, from about eight to about forty, from about ten to about sixty, etc.) nucleic acid fragments assembled in the first and/or second and optionally further reaction compartments may not be complementary or identical to the sequences of any other nucleic acid fragment simultaneously assembled in the same reaction compartment. Also, at least one of the two or more nucleic acid fragments assembled from the first sub-set of oligonucleotides may have a sequence region that is complementary to at least one of the two or more nucleic acid fragments assembled from a second or further sub-set of oligonucleotides.

Some or all of the assembled nucleic acid fragments may comprise terminal linker regions with universal primer binding sites and/or a restriction enzyme cleavage sites. In particular, the oligonucleotides that form termini of the two or more nucleic acid fragments may each comprise a linker region at one end whereas the oligonucleotides that form internal regions of the two or more nucleic acid fragments may not comprise such linker region. In instances, at least a portion of the oligonucleotides that form termini of the two or more nucleic acid fragments may comprise and assembly tag, wherein the assembly tag has a length and nucleotide or base composition designed to (i) adjust the length of one or more fragments in a pool and/or (ii) adjust the GC content of one or more fragments in a pool.

Additionally, the first and second and optionally further reaction compartments may be wells of a microwell plate or may be vessels, tubes, fluidic chambers, droplets or cells. Also, the number of different reaction compartments may be between 50 and 500 or between 10 and 100. Furthermore, the two or more nucleic acid fragments may be assembled in a reaction volume of between about 0.1 pl and about 10 pl, between about 0.01 µl and about 10 µl, between about 0.1 µl and about 1,000 µl or between about 0.5 µl and about 50 µl

In some instances, the number of oligonucleotides pooled into the first and/or second or optionally further reaction compartments may be between from about 10 and to about 500 (*e.g.,* from about 10 to about 500 oligonucleotides, from about 50 to about 500 oligonucleotides, from about 75 to about 500 oligonucleotides, from about 100 to about 500 oligonucleotides, from about 10 to about 400 oligonucleotides, from about 40 to about 400 oligonucleotides, from about 70 to about 350 oligonucleotides, etc.).

In some instances, each oligonucleotide of the plurality of oligonucleotides may be provided or synthesized at an amount of between about 10 fmol and about 10 pmol. In some aspects the oligonucleotides are synthesized at sufficient amounts on porous supports of a common carrier and are not amplified prior to the pooling and/or assembly. In some instances the number of different oligonucleotides in a sub-set required to assemble a single nucleic acid fragment is between 5 and 30 or is between 6 and 200. Similarly, the number of nucleic acid fragments simultaneously assembled in a single assembly reaction or in the first and/or second or further reaction compartment may be between 2 and 10, or between 3 and 20, or between about 2 and about 400, or between about 4 and about 200, or between about 10 and about 100. Also each nucleic acid fragment may be assembled from at least 3 overlapping oligonucleotides or may be assembled from 5 to about 30 overlapping oligonucleotides.

In some aspects, the number of different predefined oligonucleotides in a sub-set may be between about 10 and about 500. Additionally, the number of nucleic acid fragments simultaneously assembled in each reaction compartment can be between about 2 and about 400, or between about 5 and about 200, or between about 10 and about 100 or between 3 and 20, and the total number of nucleic acid fragments assembled in the first, second and optionally further reaction compartments can be between 6 and 100, or between 50 and 500 or between 100 and 1,000.. Further, each assembled nucleic acid fragment may be between about 100 and about 5,000 base pairs or between about 200 and about 600 base pairs in length. In some instances the total number of nucleic acid fragments assembled in the first, second and optionally further reaction compartments may be between 6 and 100, or between 50 and 500 or between 100 and 1,000. Further, the two or more nucleic acid fragments may be assembled in a reaction volume of between about 0.1 pl and about 10 pl, between about 0.01 µl and about 10 µl, between about 0.1 µl and about 1,000 µl or between about 0.5 µl and about 50 µl.

Additionally, the two or more nucleic acid fragments assembled in first and second and optionally further assembly reactions or reaction compartments may be grouped to minimize cross-hybridization between oligonucleotides belonging to different nucleic acid fragments, or may be grouped according to fragment GC content and/or fragment length. In some instances the two or more fragments assembled in the same assembly reaction or reaction compartment may not deviate by more than 2%, more than 5%, more than 10%, more than 15% or more than 20% in length and/or may not deviate by more than 1%, more than 2%, more than 3%, more than 4%, more than 5%, more than 10% or more than 20% in GC content.

Further, the two or more nucleic acid fragments assembled in first and second and optionally further assembly reactions or reaction compartments may be grouped according to fragment similarity. In aspects, the two or more nucleic acid fragments assembled in the same reaction compartment are non-similar, wherein similarity of two nucleic acid fragments is determined based on a pairwise alignment of the fragment sequences, division of the aligned sequences into matching blocks and irrelevant blocks and counting of the irrelevant blocks.

In some aspects a method is provided for pooling or distributing oligonucleotides for assembling S fragments into Pₘₐₓ reaction compartments wherein such method comprises the steps of (i) determining whether two fragments harmonize taking into account fragment length, fragment GC content and fragment similarity, (ii) defining an undirected graph with nodes, wherein each node represents a fragment S and wherein an edge between two nodes is present if the two respective fragments do not harmonize; (iii) coloring the nodes of the undirected graph with as few colors P as possible, wherein each color represents a reaction compartment and wherein each color is used at most Sₘₐₓ times, (iv) determining the amount of used colors P; (v) assigning the Sₘₐₓ fragments to Pₘₐₓ reaction compartments, wherein all fragments S with the same color are assigned to the same reaction compartment, and (vi) pooling the oligonucleotides into the assigned reaction compartments.

In some instances assembled nucleic acid fragments or a portion thereof may be purified from one or more reaction mixtures.

The disclosure also includes computerized methods, as well as computer programs, for oligonucleotide sequence design for multiplex nucleic acid assembly of desired nucleic acid molecules. In a first aspect such method may comprise the following steps: (i) dividing *in silico* the desired nucleic acid molecule into a plurality of fragments, (ii) generating *in silico* multiple sets of oligonucleotide sequences for each fragment, wherein each set of oligonucleotide sequences comprises a plurality of forward sequences and a plurality of reverse sequences, wherein forward and reverse sequences are intended to hybridize in a predetermined order, (iii) assigning *in silico* a score to each pair of oligonucleotide sequences within the multiple sets of oligonucleotide sequences generated *in silico,* wherein the score indicates the probability of hybridization between two oligonucleotides, (iv) determining *in silico* a total score for each set of oligonucleotide sequences by (a) summing up *in silico* the individual scores assigned to all pairs of oligonucleotide sequences which are intended to hybridize in a predetermined order and subtracting the individual scores assigned to all pairs of oligonucleotide sequences which are not intended to hybridize in said order, or by (b) selecting the maximum scores of all pairs of oligonucleotide sequences that are not intended to hybridize, and (v) selecting *in silico* for each fragment at least one set of oligonucleotide sequences from the plurality of generated sets of oligonucleotide sequences that together yield the highest total score according to step (iv) (a) or together yield the lowest total score according to step (iv) (b).

In a second aspect such method may comprise the following steps: (i) generating *in silico,* multiple sets of oligonucleotide sequences for a predefined nucleic acid sequence, wherein each set of oligonucleotide sequences comprises a plurality of forward sequences and a plurality of reverse sequences, wherein forward and reverse sequences are intended to hybridize in a predetermined order, (ii) assigning *in silico,* a score to each pair of oligonucleotide sequences within the multiple sets of oligonucleotide sequences generated *in silico,* wherein the score indicates the probability of hybridization between two oligonucleotides, (iii) determining *in silico* a total score for each set of oligonucleotide sequences by (a) summing up the individual scores assigned to all pairs of oligonucleotide sequences which are intended to hybridize in a predetermined order and subtracting the individual scores assigned to all pairs of oligonucleotide sequences which are not intended to hybridize in said order, or by (b) selecting the maximum scores of all pairs of oligonucleotide sequences that are not intended to hybridize, and (iv) dividing *in silico,* the desired nucleic acid molecule into a plurality of oligonucleotide sets, each oligonucleotide set defining a fragment of the desired nucleic acid sequence, wherein each oligonucleotide set is selected based on the highest total score determined according to step (iii) (a) or based on the lowest total score determined according to step (iii) (b).

Such methods may further comprise assigning *in silico,* a score to each combination of selected sets of oligonucleotide sequences within the pool of selected sets, wherein the score indicates the probability of cross-hybridization between forward and/or reverse oligonucleotides of a first set of oligonucleotide sequences with forward and/or reverse oligonucleotides of a second set of oligonucleotide sequences, and selecting two or more sets of oligonucleotide sequences with a desired score for simultaneous assembly of two or more fragments in the same reaction compartment.

The disclosure further comprises computer program products for use in conjunction with a computer system, including computer program products comprising a non-transitory computer readable storage medium and a computer program mechanism embedded therein, the computer program mechanism comprising instructions for performing a computerized method for optimizing oligonucleotide sequence design for multiplex nucleic acid assembly as set out herein.

The two or more nucleic acid fragments may be assembled in the two or more reaction compartments by polymerase chain reaction or ligase chain reaction. Further, the assembled fragments may be amplified using universal primers binding to universal primer binding sites in the linker regions or fragment termini. Alternatively, the assembled nucleic acid fragments may further comprise a specific primer binding site at one or both ends optionally flanking the universal primer binding sites and may be amplified using specific primers binding to the specific primer binding sites therein. Where present, specific primer binding sites may differ in all nucleic acid fragments assembled in the same reaction compartment. However, the same one or more specific primer binding sites may be present in nucleic acid fragments assembled in different reaction compartments. Also, primers for amplification of two or more specific nucleic acid fragments from the same reaction compartment may be provided at different concentrations.

The present disclosure also provides methods for combining and processing of nucleic acid assembly products from multiple assembly pools. In some aspects such method may comprise combining at least a portion of a first assembly pool comprising one or more nucleic acid fragments of a first reaction compartment and at least a portion of a second assembly pool comprising one or more nucleic acid fragments of a second reaction compartment and optionally portions of further assembly pools comprising one or more nucleic acid fragments of further reaction compartments to obtain a mixture of assembled nucleic acid fragments from multiple assembly pools.

In some aspects the combining step may comprise (i) providing a target reaction compartment, (ii) assigning a dilution factor to each assembly pool, wherein the dilution factor indicates the degree of dilution of an assembly pool in the target reaction compartment and wherein the dilution factor is determined based on one or more sequence parameters of the one or more nucleic acid fragments present in a pool, (iii) optionally determining the concentration of the nucleic acid fragments in each assembly pool and optionally adjusting the nucleic acid concentration in one or more of the assembly pools to obtain equimolar nucleic acid solutions, and (iv) combining a first quantity of the first assembly pool and at least a second quantity of the second assembly pool into the target reaction compartment, wherein the first and at least second quantities are determined based on the dilution factors assigned to the first and at least second assembly pools. In many instances the sequence parameters for determining dilution factors are selected from one or more of fragment length, GC content, complex DNA regions, number of distinct nucleic acid fragments per assembly pool, nucleic acid concentration, and/or number of screening repetitions.

Additionally, some or all of the nucleic acid fragments may be purified at various steps of the process. Also, nucleic acid fragments may be subject to one or more error correction and/or selection steps during various steps of the process as set out herein.

Methods disclosed herein may further comprise: tagging and optionally amplifying at least a portion of the nucleic acid fragments, sequencing at least a portion of the nucleic acid fragments, and analyzing the sequencing reads to identify one or more error-free nucleic acid fragment. In some instances, tagging of nucleic acid fragments may be achieved by polymerase chain reaction using primers that comprise a tag region and a fragment-compatible region. Appended tags may comprise a sequencing adaptor and/or one or more barcodes.

Such methods may further comprise: detecting that one or more nucleic acid fragments contain one or more errors or are not represented or underrepresented by the analyzed sequencing reads, identifying the one or more reaction compartments in which the one or more desired nucleic acid fragments were assembled, and selectively amplifying the one or more nucleic acid fragments from the one or more reaction compartments using fragment-specific primers. In some aspects the methods may further comprise retrieving one or more error-free nucleic acid fragments. Further, two or more of the error-free nucleic acid fragments may be combined and assembled into one or more multi-fragment assembly products as set out herein.

Additionally, the analyzed sequencing reads may be used to determine the representation and correctness of assembled nucleic acid fragments and for optimizing and/or adjusting the steps of grouping and pooling the first, second and optionally further sub-sets of oligonucleotides.

### BRIEF DESCRIPTION OF THE DRAWINGS

The novel features of the invention are set forth in the appended claims. A better understanding of the features and advantages of the present disclosure will be obtained by reference to the following detailed description that sets forth illustrative examples, in which the principles of the disclosure are utilized, and the accompanying drawings of which:

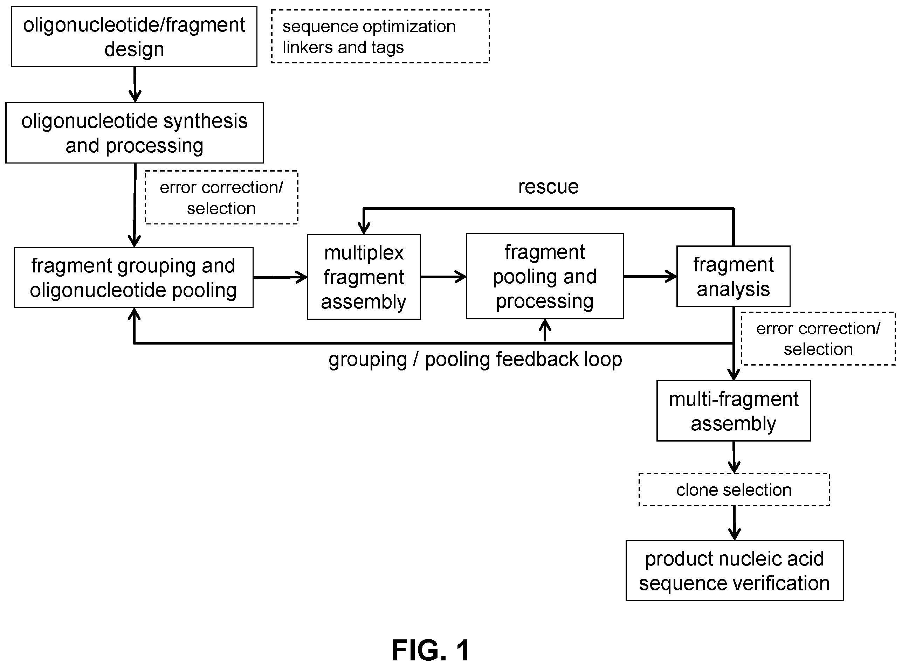

**FIG. 1** is a schematic of a workflow of various aspects of the disclosure. In this workflow, once a desired nucleic acid molecule is contemplated, a design path is set up for the production of this nucleic acid molecule. The first step will typically comprise the design of the sequence and how it is to be generated. A target sequence may be broken down into smaller fragments, *e.g*. nucleic acid fragments that may be assembled from chemically synthesized oligonucleotides. Nucleic acid fragment and oligonucleotide design may include sequence optimization as well as attachment of flanking regions (*e.g*., tags and linkers) for downstream processing. Following synthesis and processing oligonucleotide sub-sets may be selectively pooled for multiplex assembly of nucleic acid fragments in multiple pools according to specific rules. Assembled nucleic acid fragments may then be combined for further processing and analyzed. Analysis results may be used to further optimize rules for grouping and pooling oligonucleotides and nucleic acid fragments. Error correction and/or selection of oligonucleotides and nucleic acid fragments may occur at one or more points along the workflow. Correct nucleic acid fragments may be retrieved and further combined to for multi-fragment assembly to obtain longer nucleic acid molecules. A rescue pathway may be employed if no correct fragment can be identified. The desired nucleic acid molecule may then be cloned and sequence-verified.

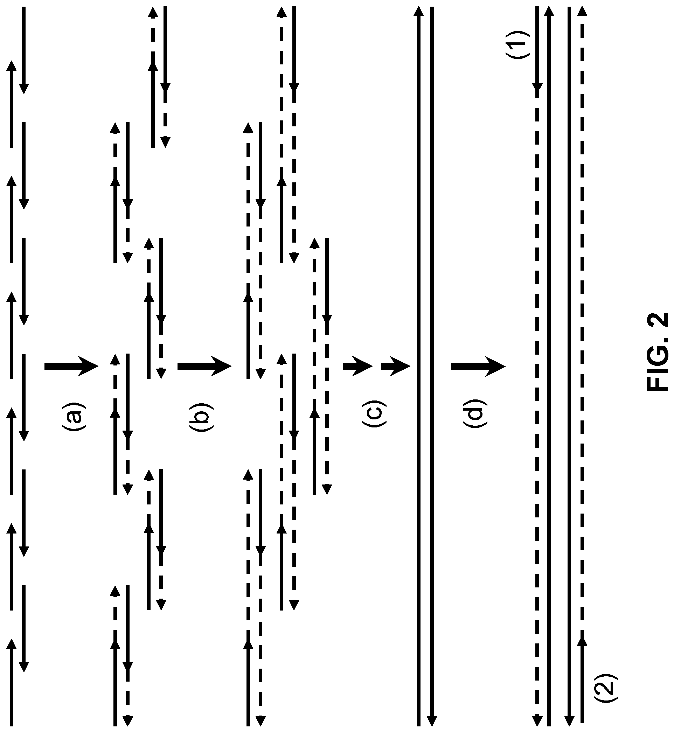

**FIG. 2** is a schematic of a PCR-based process for assembling nucleic acid molecules. (a) Overlapping forward and reverse oligonucleotides are extended in the first cycle round. (b) Extended assembly products anneal to each other and are further extended in the second cycle round. (c) Further extensions take place in subsequent rounds and full-length product accumulates. Two terminal oligonucleotides (1) and (2) can also be universal and applied in excess within the PCR mix to allow a controlled amplification of assembly products.

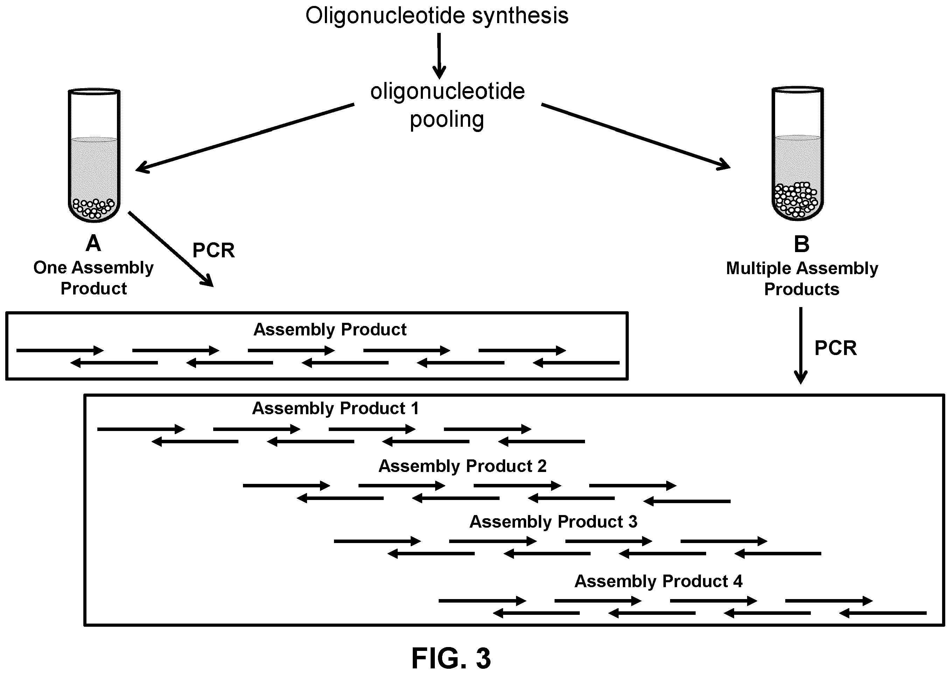

**FIG. 3** is a schematic representation of multiplex nucleic acid assembly workflows of the disclosure. (A) shows beads in a vessel, where oligonucleotides on these beads are released into solution and are then used for assembly of a single nucleic acid fragment. (B) shows beads in a vessel, where oligonucleotides from these beads are components of more than one assembly product (*e.g*., multiple fragments). These oligonucleotides released into a single pool for multiplex assembly (*e.g*., by PCR) are presented directly under (B).

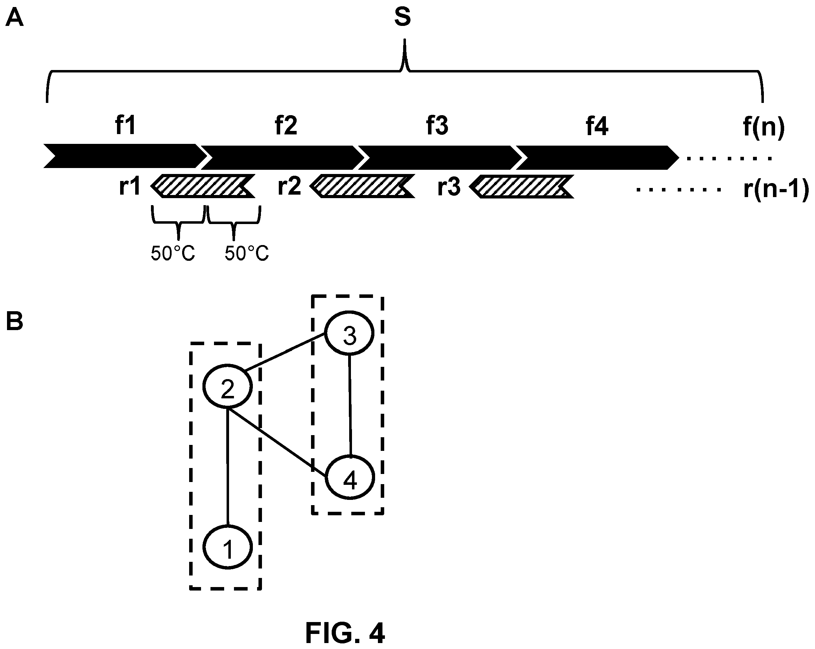

**FIG. 4** shows a bioinformatics approach for multiplex-compatible oligonucleotide design. (A) shows a set of oligonucleotides comprising overlapping forward and reverse oligonucleotides for assembly of fragment "S". (B) shows a graph of oligonucleotide sets 1 to 4, each set represented by a node with connecting lines between nodes ("edges") indicating suitability for multiplexing. Two exemplary cliques (oligonucleotide sets 1 and 2, and sets 3 and 4, respectively) are indicated by dotted boxes, together covering the entire graph.

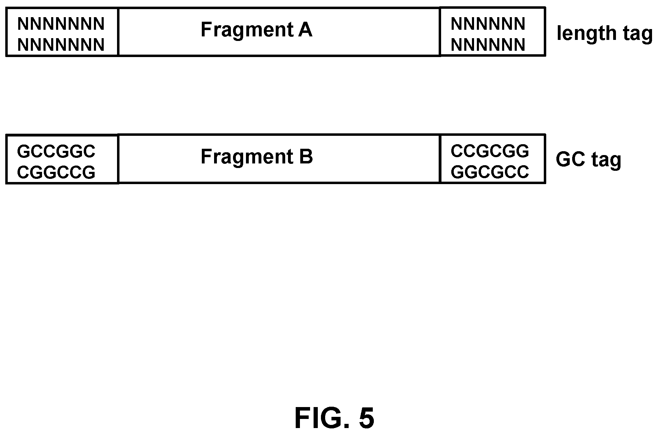

**FIG. 5** is an exemplary representation of two nucleic acid fragments comprising terminal assembly tags that do not form part of the final nucleic acid products. Fragment A comprises a "length" tag" at each end to adjust fragment length and fragment B comprises a "GC" tag at each end to compensate for a low fragment GC content. The individual composition of a length tag (wherein bases are represented by multiple "Ns") may be determined taking additional sequence parameters into account.

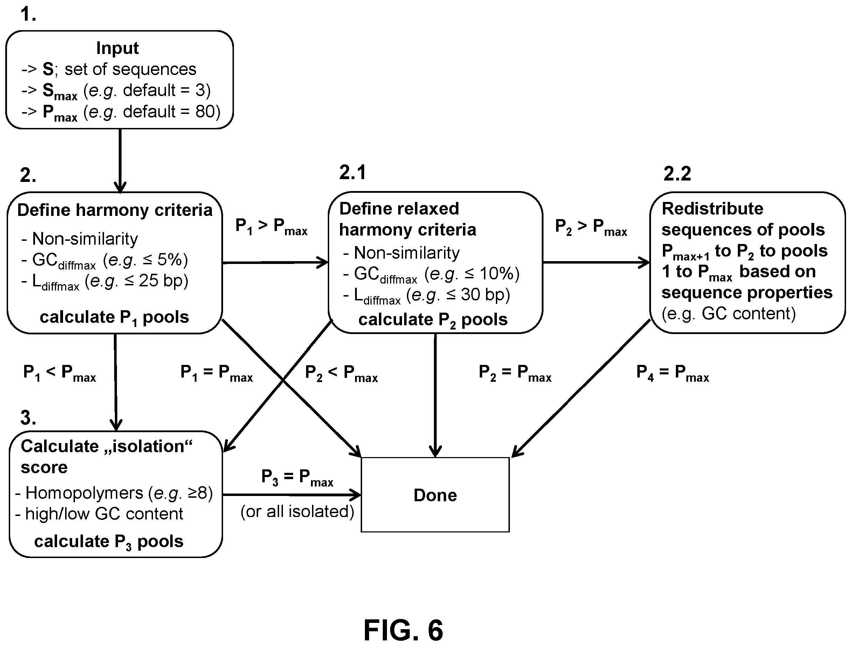

**FIG. 6** is a schematic of a method for distributing sub-sets of oligonucleotides into a predefined number of pools or reaction compartments according to sequence harmony criteria for multiplex fragment assembly.

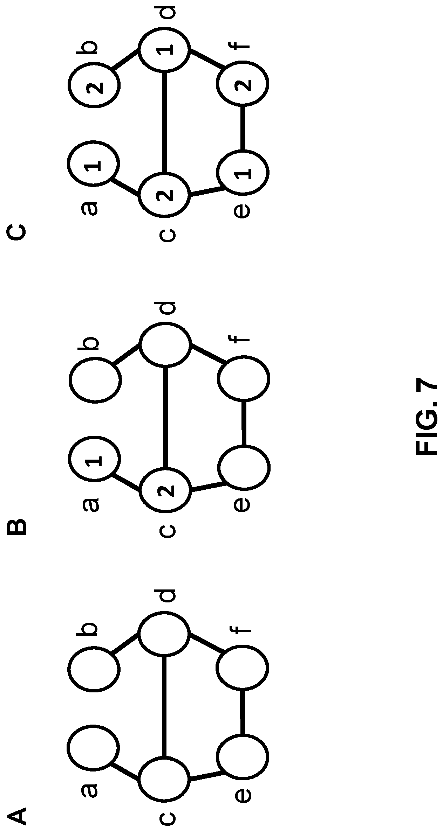

**FIG. 7** is a schematic of a "coloring" approach using an undirected graph for assigning oligonucleotide sub-sets representing nucleic acid fragments into multiple pools or reaction compartments.

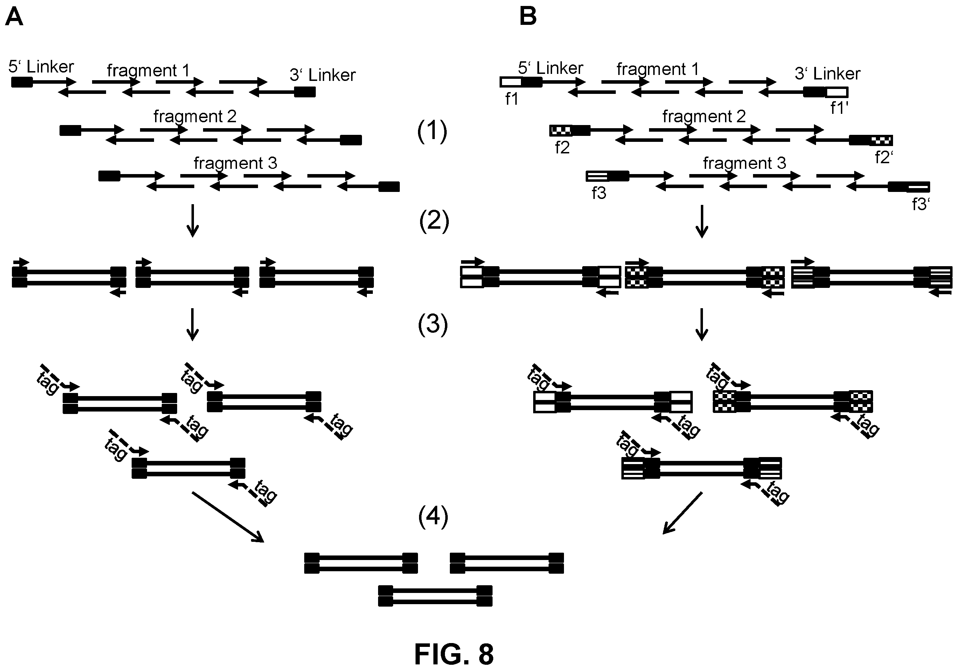

**FIG. 8** is a schematic illustration of multiplex fragment assembly and amplification using either universal (A) or fragment-specific (B) primers.

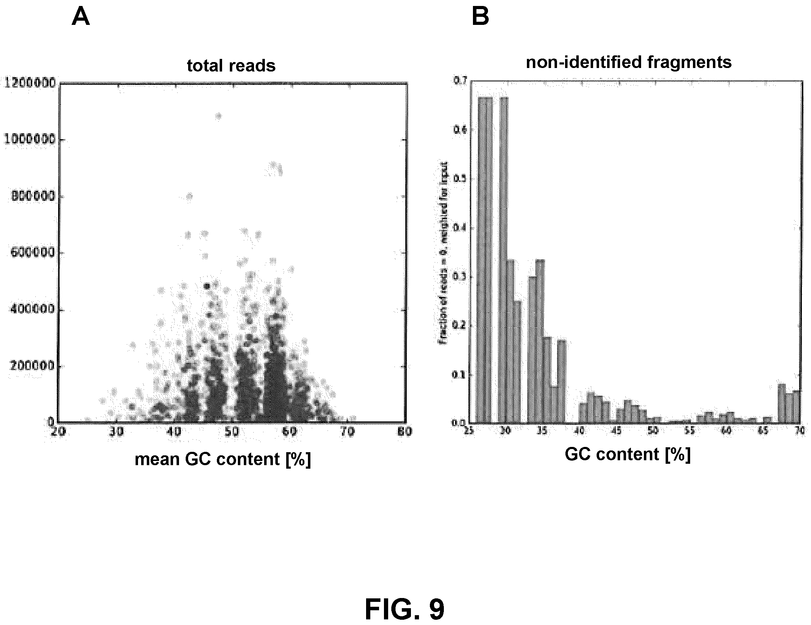

**FIG. 9** shows an analysis of total sequencing reads obtained from a mixture of nucleic acid fragments assembled in multiplex reactions. (A) shows the distribution of total reads according to fragment GC content and (B) shows the number of non-identified fragments in relation to fragment GC content.

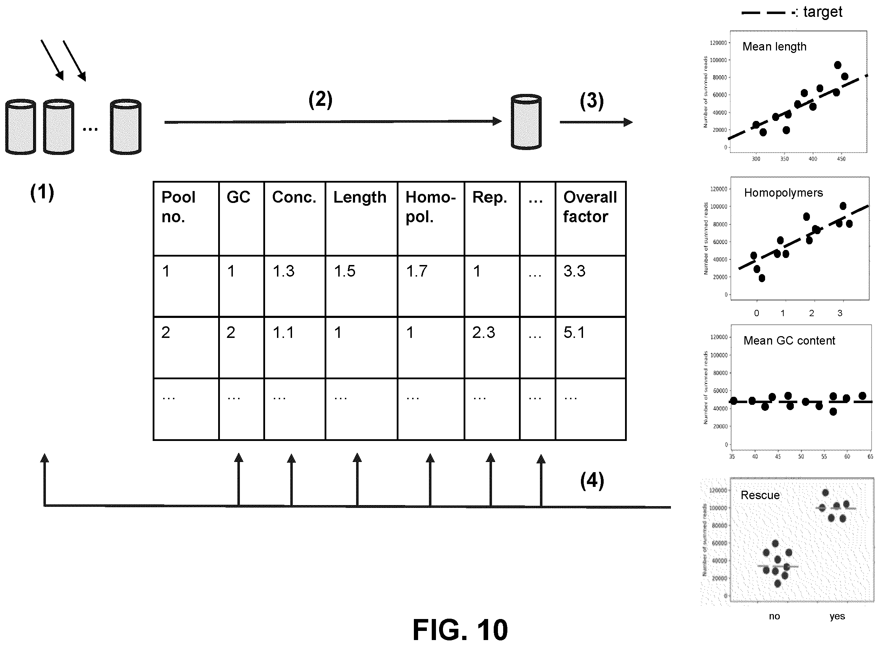

**FIG. 10** is a schematic representation of a workflow for optimized pooling of nucleic acid assembly products from multiple assembly pools for combined downstream processing. Assembled fragments are pooled in different quantities according to dilution factors assigned to the various multiplex assembly pools that take into account nucleic acid fragment sequence parameters. Fragments are processed and sequenced and total reads are analyzed according to various parameters including mean fragment length (A), number of homopolymers (B), mean GC content (C), and number of repetitions required to rescue/obtain a desired nucleic acid fragment (D). Production results are then used to further optimize and fine-tune pooling factors.

### DETAILED DESCRIPTION

### Definitions

The term "nucleic acid" or nucleic acid molecule" as used herein refers to a covalently linked sequence of nucleotides or bases (*e.g*., ribonucleotides for RNA and deoxyribonucleotides for DNA but also include DNA/RNA hybrids where the DNA is in separate strands or in the same strands) in which the 3' position of the pentose of one nucleotide is joined by a phosphodiester linkage to the 5' position of the pentose of the next nucleotide. Unless further specified, nucleic acid molecules may be single- or double-stranded or partially double-stranded and may appear in linear or circularized form in a supercoiled or relaxed formation with blunt or sticky ends and may contain "nicks". Nucleic acid molecules may be composed of completely complementary single strands or of partially complementary single strands forming at least one mismatch of bases. Nucleic acid molecules may comprise chemically, enzymatically, or metabolically modified forms of nucleotides or combinations thereof. Chemically synthesized nucleic acid molecules may refer to nucleic acids typically less than or equal to 200 nucleotides long (*e.g.,* between 5 and 200, between 10 and 150, between 15 and 100, or between 20 and 50 nucleotides in length), whereas enzymatically synthesized nucleic acid molecules may encompass smaller as well as larger nucleic acid molecules as described elsewhere herein. Enzymatic synthesis of nucleic acid molecules may include stepwise processes using enzymes such as polymerases, ligases, exonucleases, endonucleases, recombinases, terminal transferases, deacetylases, esterases or the like or a combination thereof. Thus, the disclosure provides, in part, compositions and combined methods relating to the enzymatic *de novo* synthesis of nucleic acid molecules as well as the enzymatic assembly of chemically synthesized nucleic acid molecules.

A nucleic acid molecule has a "5'-terminus" and a "3'-terminus" because nucleic acid molecule phosphodiester linkages occur between the 5' carbon and 3' carbon of the pentose ring of the substituent mononucleotides. The end of a nucleic acid molecule at which a new linkage would be to a 5' carbon is its 5' terminal nucleotide. The end of a nucleic acid molecule at which a new linkage would be to a 3' carbon is its 3' terminal nucleotide. A terminal nucleotide or base, as used herein, is the nucleotide at the end position of the 3'- or 5'-terminus. A nucleic acid molecule region, even if internal to a larger nucleic acid molecule (*e.g.,* a sequence region within a nucleic acid molecule), also can be said to have 5'- and 3'-ends or termini (wherein ends and termini are used interchangeably herein). Nucleic acid molecule also refers to short nucleic acid molecules, often referred to as, for example, primers or probes. Also, the terms "5'-" and "3'-" refer to strands of nucleic acid molecules. Thus, a linear, single-stranded nucleic acid molecule will have a 5'-terminus and a 3'-terminus. However, a linear, double-stranded nucleic acid molecule will have a 5'-terminus and a 3'-terminus for each strand. Thus, for nucleic acid molecules that encode proteins, for example, the 3'-terminus of the sense strand may be referred to.

The term "oligonucleotide", as used herein, refers to DNA and RNA, and to any other type of nucleic acid molecule that is an N-glycoside of a purine or pyrimidine base but will typically be DNA. Oligonucleotides are thus a subset of nucleic acid molecules and may be single-stranded or double-stranded. Oligonucleotides (including primers as described below) may be referred to as "forward" or "reverse" to indicate the direction in relation to a given nucleic acid sequence. For example, a forward oligonucleotide may represent a portion of a sequence of the first strand of a nucleic acid molecule (*e.g.,* the "sense" strand), whereas a reverse oligonucleotide may represent a portion of a sequence of the second strand (*e.g.,* "antisense" strand) of said nucleic acid molecule or *vice versa.* In many instances, a set of oligonucleotides used to assemble longer nucleic acid molecules will comprise both forward and reverse oligonucleotides capable of hybridizing to each other via complementary regions. Oligonucleotides that are components of larger nucleic acid molecules and can be assembled through complementary overlaps are also referred to as "assembly oligonucleotides" herein. Oligonucleotides are typically less than 300 nucleotides, more typically less than 200 nucleotides in length. Thus, "primers" will generally fall into the category of oligonucleotide. In many instances, oligonucleotides are built *de novo* (*i.e.* in the absence of a template) from stepwise addition of single building blocks or bases. The building blocks may be added chemically or enzymatically. Oligonucleotides disclosed herein can be prepared by any suitable method, including direct chemical synthesis by a method such as the phosphotriester method of Narang et al., Meth. Enzymol. 68:90-99 (1979); the phosphodiester method of Brown et al., Meth. Enzymol. 68:109-151 (1979); the diethylphosphoramidite method of Beaucage et al., Tetrahedron Letters 22:1859-1862 (1981); and the solid support method of U.S. Pat. No. 4,458,066. A review of synthesis methods of conjugates of oligonucleotides and modified nucleotides is provided in Goodchild, Bioconjugate Chemistry 1:165-187 (1990).1. For enzymatic synthesis of oligonucleotides see *e.g.* Jensen and Davis "Template-Independent Enzymatic Oligonucleotide Synthesis (TiEOS): Its History, Prospects, and Challenges", Biochemistry, 2018, 57 (12), pp 1821-1832. Oligonucleotides are typically used to construct longer nucleic acid fragments. Oligonucleotides can comprise or consist of any composition capable of being assembled, ligated, and amplified using the methods, compositions, and kits disclosed herein.

A "terminal oligonucleotide" is an oligonucleotide that comprises a terminus or terminal overhang of the nucleic acid fragment to which it corresponds. An "internal oligonucleotide" is an oligonucleotide that does not comprise a terminus or terminal overhang of a nucleic acid fragment. An internal oligonucleotide comprises two "overlaps", one of which is reverse complementary to an overlap of a first other oligonucleotide, and the other of which is reverse complementary to an overlap of a second other oligonucleotide, wherein the first and second other oligonucleotides each correspond to the strand of opposite sense to the strand to which the internal oligonucleotide corresponds. Thus, an "overlap" as used herein is a region of a first assembly oligonucleotide that is reverse complementary to an overlap (region) of a second assembly oligonucleotide, where the first and second assembly oligonucleotides both correspond to the same nucleic acid fragment.

In some instances an overlap of an assembly oligonucleotide includes a terminus of the assembly oligonucleotide. In some instances an assembly oligonucleotide may have a non-overlapping terminal region interposed between an overlap and the adjacent terminus of the assembly oligonucleotide. In some examples where an assembly oligonucleotide comprises two overlaps, the two overlaps may be contiguous one with the other or there may be a region interposed between the two overlaps. In some instances where an assembly oligonucleotide comprises two overlaps, the two overlaps are contiguous one with the other and together make up the entire sequence of an assembly oligonucleotide. Overlaps may be of any length operable for hybridization as described elsewhere herein. In various examples, overlaps are sequence adjusted or optimized for preferential hybridization of their exact reverse complements. In some examples, one or more assembly oligonucleotides may comprise an overlap with a terminal regional of a target vector or other nucleic acid molecule.

An "assembly oligonucleotide pool" is a mixture comprising four or more single stranded oligonucleotide species (4, 5, 6, 7, 8, 9, 10, 11, 12, 13, 14, 15, 16, 17, 18, 19, 20, or more) that is sufficient to provide all oligonucleotides for assembly of at least one nucleic acid fragment. An assembly oligonucleotide pool may comprise sufficient oligonucleotides for a single, or a plurality (2, 3, 4, 5, or more) of nucleic acid fragment and/or one or more polynucleotides of interest (*e.g.,* 2, 3, 5, 10, 50, 100, 500, 1000, etc.). Assembly oligonucleotide pools can be provided in any form, including without limitation frozen, in solution, or bound to a substrate such as, by way of non-limiting example only, a microarray, microfluidics array, or solid phase material of a column. Oligonucleotide pools may, in addition to the oligonucleotides that are components of assembly products, comprise other oligonucleotides, including without limitation oligonucleotides that result from errors in oligonucleotide synthesis present in the assembly oligonucleotide pool.

Further, oligonucleotides may be modified after synthesis. For example, oligonucleotides may be labelled or may be phosphorylated to facilitate ligation of adjacent oligonucleotides in an assembly reaction.

Where appropriate, the term oligonucleotide may refer to a primer or probe and these terms may be used interchangeably herein.

Whereas probes may be typically used to detect at least partially complementary nucleic acid molecules, primers are often referred to as starter nucleic acid molecules for enzymatic assembly reactions. Thus, the term "primer", as used herein, refers to a short nucleic acid molecule capable of acting as a point of initiation of nucleic acid synthesis under suitable conditions. Such conditions include those in which synthesis of a primer extension product complementary to a nucleic acid strand is induced in the presence of different nucleoside triphosphates (*e.g.,* A, C, G, T and/or U) and an agent for extension (for example, a DNA polymerase or reverse transcriptase) in an appropriate buffer and at a suitable temperature. A primer is generally composed of single-stranded DNA but can be provided as a double-stranded molecule for specific applications (*e.g.,* blunt end ligation). Optionally, a primer can be naturally occurring or synthesized using chemical synthesis of recombinant procedures. The appropriate length of a primer depends on the intended use of the primer but typically ranges from about 6 to about 200 nucleotides, including intermediate ranges, such as from about 10 to about 50 nucleotides, from about 15 to about 35 nucleotides, from about 18 to about 75 nucleotides and from about 25 to about 150 nucleotides. The design of suitable primers for the amplification of a given target sequence is well known in the art and described in the literature (see for example OLIGOPERFECT^{™} Designer, Thermo Fisher Scientific). Primers can incorporate additional features which allow for the detection or immobilization of the primer but do not alter the basic property of the primer, that of acting as a point of initiation of DNA synthesis.

In some examples, a primer includes a detectable moiety or label. The label can generate, or cause to generate, a detectable signal. In some examples, the detectable signal can be generated from a chemical or physical change (*e.g.,* heat, light, electrical, pH, salt concentration, enzymatic activity, or proximity events). In some examples, the label can include compounds that are luminescent, photoluminescent, electroluminescent, bioluminescent, chemiluminescent, fluorescent, phosphorescent or electrochemical. In some examples, the label can include compounds that are fluorophores, chromophores, radioisotopes, haptens, affinity tags, atoms or enzymes.

Primers may contain an additional nucleic acid sequence at the 5' end which does not hybridize to the target nucleic acid, but which facilitates cloning or detection of the amplified product. The region of the primer that is sufficiently complementary to the template or target nucleic acid molecule to hybridize is typically located in the 3' region of a primer and referred to herein as the hybridizing or complementary region, or target-specific region. The primer can also include a region that is designed to exhibit minimal hybridization to a portion of the template nucleic acid molecule (*e.g.,* a non-target specific sequence in the 5' region of the primer). For example, the primer can include at least one tag in the 5' non-target specific region. A set of primers (optionally including tags) used in the same amplification reaction may have melting temperatures (Tm) that are substantially the same, where the melting temperatures are within about 10-5 °C of each other, or within about 5-2 °C of each other, or within about 2-0.5 °C of each other, or less than about 0.5 °C of each other.

Adaptors may be used for example, to graft a nucleic acid molecule to a support (*e.g.,* bead, flowcell, bottom of a well or array of reaction sites). In some examples, an adaptor can have any length, including fewer than 10 bases in length, or about 10-20 bases in length, or about 20-50 bases in length, or about 50-100 bases in length, or longer. An adaptor can have any combination of blunt end(s) and/or sticky end(s). In some examples, at least one end of the adaptor can be compatible with at least one end of a nucleic acid molecule. In some examples, a compatible end of the adaptor can be joined to a compatible end of a nucleic acid molecule. In some examples, the adaptor can have a 5' or 3' overhang end. In some examples, the adaptor can include an oligo-dA, oligo-dT, oligo-dC, oligo-dG or oligo-U sequences or can include one or more inosine residues. Adaptors are often used for conducting a sequencing reaction and may therefore be included in tags attached to nucleic acid molecules prior to sequencing.

The term "barcode", as used herein, refers to a nucleic acid segment that may be used as a component of a nucleic acid molecule to identify this nucleic acid molecule. For example, a nucleic acid molecule may have at its 5' terminus a nucleotide region that identifies it as coming from a particular sample or source. Barcodes may also be used to identify individual nucleic acid molecules that result in the generation of specific assembly or amplification products (*e.g.,* an assembly product that does not contain errors). Barcodes may be used, for example, to determine the effects of "PCR heterogeneity". Typically, individual nucleic acid molecules or assembly products to be analyzed are connected (*e.g.,* via ligation or PCR) to a library of diverse (degenerate or defined) barcodes at an early step in a workflow. This connection is typically done in a manner such that each nucleic acid molecule in a sample (or in a diluted portion thereof) is statistically connected to a different barcode that remains associated with it during amplification processes. The origin of amplified nucleic acid molecules may then be traced back to starting nucleic acid molecules (or a container comprising such nucleic acid molecules). This is typically done by sequencing of nucleic acid molecules present after amplification.

Barcodes may be of any number or lengths but they will typically be long enough such that they can be readily identified (*e.g.,* by hybridization, by sequencing, etc.) but short enough so that they do not interfere with processes related to the workflows they are used in. Barcode length may also depend on the read lengths of sequencing platforms used. Barcodes will typically be from about 5 to about 50 nucleotides (or base pairs). In many instances, a barcode may have a length that is sufficient to allow for specific binding of a barcode-specific primer such as, *e.g*., between about 10 and about 30 bases.

Where barcodes are used in dial-out procedures (*i.e.* to specifically amplify and retrieve a sequence-verified or error-free nucleic acid fragment from a mixed pool) they may be designed to (i) allow for sufficient distinction between all barcodes even if sequencing errors occur (*e.g.,* where a sequencing-based mutation in a first barcode would result in a sequence that is identical with the sequence of a second barcode), and to (ii) allow for specific retrieval ("dialing-out") of a desired molecule carrying an individual barcode (*e.g.,* by using a set of barcode primers, wherein each primer only hybridizes to one specific barcode). Sufficiently distinct barcodes or barcode specific primers may be designed using "edit distance" (also referred to as "Levenshtein distance") or "Hamming distance" parameters known in the art.

Like oligonucleotides or primers, barcodes may be synthesized on a microchip or array in microscale amounts using standard chemistry, enzymatic synthesis, or electro- or photochemistry as disclosed elsewhere herein.

The terms "complementary" or "complementarity", as used herein, refer to the natural binding of nucleic acid molecules (primers, oligonucleotides or polynucleotides etc.) under permissive salt and temperature conditions by base pairing. For example, the sequence "A-G-T" binds to the complementary sequence "T-C-A." Complementarity between two single-stranded molecules may be "partial," such that only some of the nucleic acids bind, or it may be "complete," such that total complementarity exists between the single-stranded molecules. The degree of complementarity between nucleic acid strands has significant effects on the efficiency and strength of the hybridization between the nucleic acid strands. This is of particular importance in amplification reactions, which depend upon binding between nucleic acids strands. Complementary regions between nucleic acid molecules such as oligonucleotides may also be referred to as "overlaps" or "overlapping" regions.

As used herein the term "overlap" or "overlapping" refers to a sequence homology or sequence identity shared by a portion of two or more oligonucleotides.

The term "homologous" or "homology", as used herein, refers to a degree of identity. Nucleic acid sequences may be partially or completely homologous (identical). A partially homologous sequence is one that at least partially inhibits a completely complementary sequence from hybridizing to a target nucleic acid and is referred to using the functional term "substantially homologous".

The term "percent identical" refers to percentage of sequence identity between two amino acid sequences or between two nucleotide sequences. Identity can each be determined by comparing a position in each sequence which may be aligned for purposes of comparison. For example, sequences can be identical on a nucleotide-by-nucleotide basis for nucleic acids or amino acid-by-amino acid basis for polypeptides over a window of comparison. Percent identity can be calculated, for example, by comparing two optimally aligned sequences over a comparison window, determining the number of positions at which the identical nucleotides or amino acids occurs in both sequences to yield the number of matched positions, dividing the number of matched positions by the total number of positions in the comparison window, and multiplying the result by 100 to yield the percentage of sequence identity. Methods to calculate the percentage of sequence identity are known to those of skill in the art. When an equivalent position in the compared sequences is occupied by the same base or amino acid, then the molecules are identical at that position; when the equivalent site occupied by the same or a similar amino acid residue (*e.g.,* similar in steric and/or electronic nature), then the molecules can be referred to as homologous (similar) at that position. Expression as a percentage of homology, similarity, or identity refers to a function of the number of identical or similar amino acids at positions shared by the compared sequences.

A "region of homology" refers to a portion or segment of a sequence that has substantial identity with a portion of another sequence, *e.g.,* portions of the sequences of two nucleic acid fragments, more generally portions of the sequences of two polynucleotides or nucleic acid molecules. An internal region of homology means that the homologous sequence portion does not encompass either the termini of the nucleic acid (*e.g.,* the 5' and 3' terminal-most sequences with reference to a single stranded nucleic acid fragment or the ends of a double stranded nucleic acid fragment with reference to one strand). The degree of homology between the internal sequence portions is sufficiently high to permit hybridization between complementary strands of the sequence portions under conditions suitable for nucleic acid assembly as described herein. For example, the regions of internal homology may comprise at least ab out 70%, 75%, 80%, 85%, 90%, 95%, 96%, 97%, 98%, 99% or 100% sequence identity. The region of internal homology may span at least about 10, 15,20, 25, 30,40, 50, 60, 70, 80, 90, 100, or more, consecutive nucleic acid residues. In an example, the region of internal homology spans at least the length of one or more oligonucleotides. The term "sequence homology" as used herein refers to the proportion of base matches between two nucleic acid sequences or the proportion of amino acid matches between two amino acid sequences. When sequence homology is expressed as a percentage, *e.g.,* 50%, the percentage denotes the proportion of matches over the length of a desired sequence as compared to another sequence. Gaps (in either of the two sequences) are permitted to maximize matching; gap lengths of 15 bases or less are usually used, 6 bases or less are used more frequently, with 2 bases or less used even more frequently.

The term "hybridization", as used herein, refers to any process by which a strand of nucleic acid binds with an at least partially complementary strand through base pairing. Hybridization and the strength of hybridization (for example, the strength of the association between the nucleic acids) is impacted by such factors as the degree of complementary between the nucleic acids, stringency of the conditions involved, the Tₘ of the formed hybrid, and the G:C ratio within the nucleic acids.

As defined herein "multiplex assembly" refers to selective and non-random assembly of two or more nucleic acid fragments within a sample or reaction compartment from sets of overlapping or complementary oligonucleotides, wherein each set of oligonucleotides comprises multiple oligonucleotides that together comprise the sequence of one predefined nucleic acid fragment in that sample or compartment. In some instances the "plexy" or "plex" of a given multiplex assembly refers to the number of different nucleic acid fragments that are simultaneously assembled in a single assembly reaction (*i.e.* the same reaction compartment). In some instances however, the plexy" or "plex" may also refer to the number of different nucleic acid fragments that are assembled in parallel in multiple assembly reactions (*i.e.* in different compartments). Multiplex assembly in a single compartment may comprise between about 3 and about 20, between about 3 and about 50, between about 5 and about 100 or between about 10 and about 1,000 assembly reactions. Multiplex assembly in separate compartments may comprise between about 2 and about 2,000, between about 5 and about 500, between about 10 and about 100 assembly reactions conducted in parallel.

The term "gene" or "gene sequence", as used herein generally refers to a nucleic acid sequence that encodes a discrete cellular product. In many instances, a gene or gene sequence includes a DNA sequence that comprises an open reading frame (ORF) and can be transcribed into mRNA which can be translated into polypeptide chains, transcribed into rRNA or tRNA or serve as recognition sites for enzymes and other proteins involved in DNA replication, transcription and regulation. These genes include, but are not limited to, structural genes, immunity genes, regulatory genes and secretory (transport) genes etc. However, as used herein, "gene" refers not only to the nucleotide sequence encoding a specific protein, but also to any adjacent 5' and 3' non-coding nucleotide sequence involved in the regulation of expression of the protein encoded by the gene of interest. These non-coding sequences include terminator sequences, promoter sequences, upstream activator sequences, regulatory protein binding sequences, and the like. In many instances, a gene is assembled from shorter oligonucleotides or nucleic acid fragments.

The terms "fragment", "segment", or "component" or similar terms as used herein in connection with a nucleic acid molecule or sequence either refer to a product or intermediate product obtained from one or more process steps (*e.g.,* synthesis, assembly, amplification, tagging etc.), or refer to a portion, part or template of a longer or modified nucleic acid product to be obtained by one or more process steps (*e.g.,* assembly, amplification, construction, cloning etc.). In some instances, a nucleic acid fragment may represent both, an assembly product (*e.g.,* assembled from multiple oligonucleotides) and a starting compound for higher order assembly (*e.g.,* a gene assembled from multiple fragments).

The term "full-length" as used herein in connection with a nucleic acid molecule or sequence refers to the "desired" length of a product nucleic acid molecule or sequence to be obtained by a given process step (*e.g.,* the product of an assembly reaction or of multiple subsequent assembly reactions). The term full-length is therefore not limited to the length of the final or last nucleic acid product generated in a workflow or by a series of method steps, but refers, in some instances, to the desired length of an intermediate product of a particular process step. For example, a full-length oligonucleotide may be one that does not have truncations as a result of chemical synthesis.

The term "vector", as used herein refers to any nucleic acid molecule capable of transferring genetic material into a host organism. The vector may be linear or circular in topology and includes but is not limited to plasmids, viruses, bacteriophages. The vector may include amplification genes, enhancers or selection markers and may or may not be integrated into the genome of the host organism.

The term "plasmid", as used herein refers to a vector that is able to be genetically modified to insert one or more nucleic acid molecules (*e.g.,* assembly products). Plasmids will typically contain one or more region that renders it capable of replication in at least one cell type.

The term "amplification", as used herein, relates to the production of additional copies of a nucleic acid molecule. Amplification as used herein is often carried out using polymerase chain reaction (PCR) technologies well known in the art (*see, e.g.,* Dieffenbach, C. W. and G. S. Dveksler (1995) PCR Primer, a Laboratory Manual, Cold Spring Harbor Press, Plainview, N.Y.) but may also be carried out by other means including isothermal amplification methods such as, *e.g*., transcription mediated amplification, strand displacement amplification, rolling circle amplification, loop-mediated isothermal amplification, helicase-dependent amplification, single primer isothermal amplification or recombinase polymerase amplification (*see, e.g.,* Fakruddin et al., "Nucleic acid amplification: Alternative methods of polymerase chain reaction", J. Pharm Bioallied Sci, 2013, v.5(4), 245-252; or Gill and Ghaemi, "Nucleic acid isothermal amplification technologies: a review", Nucleosides Nucleotides Nucleic Acids. 2008 27(3), 224-43).

The term "multiplex amplification" refers to selective and non-random simultaneous amplification of two or more nucleic acid fragments within the same sample or reaction compartment. Multiplex amplification is typically conducted by PCR-based methods in the presence of one or more pairs of terminal primers capable of hybridizing to specific primer binding sites in the target nucleic acid molecules. In some instances the "plexy" or "plex" of a given multiplex amplification refers to the number of different nucleic acid molecules that are simultaneously amplified in a single reaction (*i.e.* the same reaction compartment). Multiplex amplification in a single compartment may comprise between about 3 and about 20, between about 3 and about 50, between about 5 and about 100 or between about 10 and about 1,000 assembly reactions.

The term "adjacent", as used herein, generally refers to a position in a nucleic acid molecule immediately 5' or 3' to a reference region. The term adjacent may also be used for oligonucleotides that can be positioned next to each other in a given nucleic acid strand during an assembly reaction.

The term "transition", when used in reference to the nucleotide sequence of a nucleic acid molecule, refers to a point mutation that changes a purine nucleotide to another purine (A ↔ G) or a pyrimidine nucleotide to another pyrimidine (C ↔ T).

The term "transversion", when used in reference to the nucleotide sequence of a nucleic acid molecule, refers to a point mutation involving the substitution of a (two ring) purine for a (one ring) pyrimidine or a (one ring) pyrimidine for a (two ring) purine.

The term "indel", as used herein, refers to the insertion or deletion of one or more bases in a nucleic acid molecule.

The term "sequence fidelity", as used herein refers to the level of sequence identity of a nucleic acid molecule as compared to a reference sequence. Full identity being 100% identical over the full-length of the nucleic acid molecules being scored for sequence identity. Sequence fidelity can be measure in a number of ways, for example, by the comparison of the actual nucleotide sequence of a nucleic acid molecule to a desired nucleotide sequence (*e.g.,* a nucleotide sequence that one wishes to be used to generate a nucleic acid molecule). Another way sequence fidelity can be measured is by comparison of sequences of two nucleic acid molecules in a reaction mixture. In many instances, the difference on a per base basis will be, on average, the same.

The term "error correction", as used herein refers to changes in the nucleotide sequence of a nucleic acid molecule to alter a defect. These defects can be mismatches, insertions, deletions and/or substitutions. Defects can occur when a nucleic acid molecule that is being generated (*e.g.,* by chemical or enzymatic synthesis) is intended to contain a particular base at a location but a different base is present at that location.

The term "transformation", as used herein, describes a process by which an exogenous nucleic acid molecule enters and changes a recipient cell. It may occur under natural or artificial conditions using various methods well known in the art. Transformation may rely on any known method for the insertion of foreign nucleic acid sequences into a prokaryotic or eukaryotic host cell. The method is selected based on the host cell being transformed and may include, but is not limited to, viral infection, electroporation, lipofection, and particle bombardment. Such "transformed" cells include stably transformed cells in which the inserted nucleic acid is capable of replication either as an autonomously replicating plasmid or as part of the host chromosome. They also include cells that transiently express the inserted DNA or RNA for limited periods of time.

The terms "microchip", "chip", "synthesis chip", or similar variations thereof as used herein will refer to an electronic computer chip on which oligonucleotide synthesis can occur.

The term "microarray" or "array", as used herein refers to an array of distinct polynucleotides or oligonucleotides arrayed on a substrate, such as paper, nylon or any other type of membrane, filter, chip, glass slide, or any other suitable solid support. In certain instances a microchip may represent a specific example of a microarray.

The terms "multiwell plate", "microplate", "microwell plate", "plate" or similar variations thereof refer to a two-dimensional array of multiple wells located on a substantially flat surface. Multiwell plates can comprise any number of wells of any width or depth. In certain instances, a multiwell plate may be configured as a microchip. For example, when a material with well-like structures is overlaid onto a microchip.

The term "reaction compartment" refers to any subdivided region or space that is suitable for conducting chemical or biological reactions such that the reactants are separated from reactants in a second region by a physical barrier. A reaction compartment may include for example a container, a well, a tube, a vessel, a channel, a chamber, a groove, a pore, a droplet, a cell etc.

Depending on reaction conditions a reaction compartment may have any shape, size or dimension. For example, a reaction compartment may have a width, height or diameter of between about 10 µm and about 100 mm, or between about 100 µm and about 10 mm, or between about 500 µm and about 1 mm, etc.

Also, a reaction compartment may be defined by its reaction volume. In some instances the reaction volume may be between about 0.01 µl and about 1,000 µl or between about 0.1 µl and about 1,000 µl or between about 0.5 µl and about 500 µl or between about 0.1 µl and about 100 µl or between about 0.5 µl and about 50 µl or between 0.1 and about 10 µl. The number of reaction compartments may also vary and may depend on the number of reactions that may be performed or handled in parallel (*e.g*. assisted by automated procedures). Thus, depending on various factors including efficiency, parallelization of automation, the number of compartments can be adjusted to accommodate the desired scale. In some instances the number of compartments may be between 3 and 1,000, between 50 and 500 or between 10 and 100. In some instances the compartments may be wells of a microwell plate and the number of wells may be 6, 12, 24, 48, 96, 96, 384 or 1536.

In some instances, a prokaryotic or eukaryotic cell (*e.g.,* a yeast cell, an *E. coli* cell, a mammalian cell, an engineered cell) may be used as reaction compartment in methods disclosed herein. In some instances, a cellular compartment may comprise a reaction volume of between about 0.1 pl and about 10 pl.

The term "solid support", as used herein refers to a porous or non-porous material on which polymers such as oligonucleotides or nucleic acid molecules can be synthesized and/or immobilized. As used herein "porous" means that the material contains pores which may be of non-uniform or uniform diameters (for example in the nm range). In such porous materials, the reaction may take place within the pores. The support can have any one of a number of shapes, such as pin, strip, plate, disk, rod, fiber, bends, cylindrical structure, planar surface, concave or convex surface or a capillary or column. The support can be a particle, including bead, microparticles, nanoparticles and the like. The support can be a non-bead type particle (*e.g.,* a filament) of similar size. The support can have variable widths and sizes. For example, sizes of a bead which may be used in the practice of aspects of the disclosure may vary widely but include beads with diameters between 0.01 µm and 100 µm, between 1.0 µm and 100 µm, between 0.5 µm and 50 µm, between 1.0 µm and 10 µm, between 5 µm and 40 µm, or between 10 µm and 80 µm.

The support can be hydrophobic or capable of binding a molecule via hydrophobic interaction. The support can be hydrophilic or capable of being rendered hydrophilic and includes inorganic powders such as silica, magnesium sulfate, and alumina, cellulosic materials. Supports may be synthetic or modified naturally occurring polymers, such as nitrocellulose, carbon, cellulose acetate, polyvinyl chloride, polyacrylamide, cross linked dextran, agarose, polyacrylate, polyethylene, polypropylene, poly (4-methylbutene), polystyrene, polymethacrylate, poly(ethylene terephthalate), nylon, poly(vinyl butyrate), polyvinylidene difluoride (PVDF) membrane, glass, controlled pore glass, magnetic controlled pore glass, magnetic or non-magnetic beads, ceramics, metals, and the like; either used by themselves or in conjunction with other materials.

The support can be immobilized or located at an addressable position of a carrier such as, *e.g.,* a multiwell plate or a microchip. The support can be loose, fragmentable or particulate (such as, *e.g.,* a resin material or a bead in a well) or can be reversibly immobilized or linked to the carrier (*e.g.,* by cleavable chemical bonds or magnetic forces etc.). For example, a plurality of nucleic acids having a desired sequence (such as a specific type of oligonucleotide) may be synthesized or provided on individual supports in an array device or common carrier where each support is located at a uniquely addressable position such that one or more individual supports and specific nucleic acids bound thereto can be selectively retrieved from defined positions of the array or carrier.

As used herein an "addressable position" generally refers to a location within an array or carrier that may be readily identified so that a nucleic acid molecule located at a specific position may be individually processed at or retrieved from the one or more specific position of said array or carrier. In some instances an array or carrier may comprise multiple individual supports (such as beads) located at distinct addressable positions (such as individual wells of a multiwell plate or a microchip) with each support carrying a specific type of oligonucleotide (an oligonucleotide having a desired base composition and/or length and/or sequence). In some instances the carrier is a microchip with multiple wells, each well configured to receive a single support. Single supports may be retrieved as disclosed for example in PCT Publication WO 2016/094512.

In many instances, a support used in methods or compositions of the disclosure will be one where individual nucleic acid molecules are synthesized on separate or discrete areas to generate features (*i.e.,* locations containing individual nucleic acid molecules) on the support. In some examples, the size of the defined feature is chosen to allow formation of a microvolume droplet or reaction volume on the feature, each droplet or reaction volume being kept separate from each other. As described herein, features are typically, but need not be, separated by interfeature spaces to ensure that droplets or reaction volumes or between two adjacent features do not merge. Interfeatures will typically not carry any nucleic acid molecules on their surface and will correspond to inert space. In some examples, features and interfeatures may differ in their hydrophilicity or hydrophobicity properties. In some examples, features and interfeatures may comprise a modifier. In one example of the disclosure the feature is a well or microwell or a notch. Nucleic acid molecules may be covalently or non-covalently attached to the surface or deposited or synthesized or assembled on the surface.

### Overview

The disclosure relates, in part, to compositions and methods for the preparation of nucleic acid molecules. While the disclosure has numerous aspects and variations associated with it, some of these aspects and variations of applicability of the technology may be related to one or more process steps represented in the exemplary workflow shown in FIG. 1.

FIG. 1 shows a workflow directed to compositions and methods for designing and generating a desired product nucleic acid (*e.g.,* a gene). In this workflow, the first step is designing the desired nucleic acid molecule and, in most instances, oligonucleotides and intermediate fragments that will be used in the workflow to generate the desired product nucleic acid molecule. The designing step typically includes sequence optimization, breaking up the sequence into smaller fragments and fragments into overlapping oligonucleotides. Depending on functionality required in down-stream reactions terminal oligonucleotides may be designed to include one or more tags or linkers as specified below.

Once the design phase is completed, the oligonucleotides that make up the desired nucleic acid molecules can then be synthesized and processed for use in down-stream assembly reactions.

The next step in the workflow shown in FIG. 1 is the grouping and pooling of sets of oligonucleotides that will be used to produce the desired nucleic acid fragments. In many instances, oligonucleotides used to form the assembly product will be produced on solid supports. Preferably, the supports are loose and/or removable from a common carrier to enable individual retrieval and pooling of desired oligonucleotides. Where solid supports are particulate (e.g., beads) these solid supports may be pooled and the oligonucleotides may then be released from the supports. Then oligonucleotides that are to be used in the generation of a desired fragment may be collected prior to assembly of the fragment, or oligonucleotides may not be separated but used to assemble a plurality of different desired fragments in the same reaction compartment. The process chosen will typically be directed by the specifics of the assembly process, the synthesis platform used and the desired assembly products.

The pooling of sets of oligonucleotides into multiple compartments for multiplex fragment assembly may be fine-tuned to take into account various criteria such as length, GC content and fragment similarity.

According to the workflow in FIG. 1, once the appropriate oligonucleotides are collected, these oligonucleotides are then used to assemble multiple nucleic acid fragments through a series of assembly reactions (as exemplified in FIG. 2). Multiplex assembly reactions can be conducted in multiple reaction compartments to simultaneously assemble multiple fragments from pooled sets of oligonucleotides in each compartment (as exemplified in FIG. 3). In many instances, nucleic acid amplification reactions (*e.g.,* PCR, LCR) are used for one or more assembly steps.

Nucleic acid fragments obtained from multiple assembly reactions may then be further amplified using universal or specific primers and can be pooled for down-stream processing. Multiplex amplification can be conducted with multiple primer pairs to equalize distributions of different nucleic acid fragments in a pool. Pooled fragments can then optionally be purified, amplified, tagged and prepared for sequencing reactions and analyzed to identify correct assembly products. Optionally, where one or more nucleic acid fragments with correct sequences cannot be identified or are underrepresented in a first reaction cycle, a "rescue" step may be performed to selectively retrieve and process such fragment (*e.g.,* by selective amplification from a multiplex assembly reaction pool).

Sequencing data obtained from a "reaction cycle" (*i.e.* all steps from oligonucleotide synthesis to fragment sequence analysis) may further be analyzed for certain fragment parameters (*e.g.,* fragment length, GC content, homopolymers, similarity etc.) to determine to what extent those parameters affect assembly efficiency and correctness. Such analyses can be used to fine-tune criteria for oligonucleotide and fragment pooling thereby establishing a feedback loop for process optimization.

Following sequence analysis, error-free fragments can be retrieved and optionally assembled into larger product nucleic acids.

Also according to some examples of the workflow in FIG. 1, assembled nucleic acid molecules are cloned. Typically, assembled nucleic acid molecules are inserted into a vector, then the vector is introduced into a host cell, followed by selection to generate clonal isolates containing candidate inserts representing the desired product nucleic acid molecule. These candidates may then be sequenced to determine (1) whether the desired product nucleic acid molecule is present and, if so, (2) whether the nucleotide sequence of the desired product nucleic acid molecule is correct. Further, a sufficient number of clonal isolates will typically be generated to have a high probability that at least one of the clonal isolates is of the desired product nucleic acid molecule with the correct sequence.

In some aspects, methods are provided for the production of nucleic acid molecules having high "sequence fidelity". This high sequence fidelity can be achieved by, for example, one two or all four of the following: accurate nucleic acid synthesis, intelligent pooling of oligonucleotides for multiplex fragment assembly, selection of oligonucleotides or nucleic acid fragments having "correct" sequences, error correction, and sequence verification of product nucleic acid molecules. Components and methods for achieving high sequence fidelity will generally be used in more than one portion of workflows and each component or method may be used more than once. For example, the exemplary workflow set out in FIG. 1 shows error correction/correct sequence selection occurring at two different steps in the workflow.

Described herein are a number of components and/or methods with applicability to workflows such as those shown in FIG. 1, as well as other workflows. Thus, components and/or methods set out herein may be useful, for example, (1) in workflows such as that set out in FIG. 1, (2) in other workflows related to nucleic acid biology, (3) as "stand alone" components and/or methods, and (4) as components and/or methods that may be practiced in combination with other components and/or methods set out herein.

### Oligonucleotide and Fragment Design

### Sequence optimization

As noted above, often, one of the first steps in producing a nucleic acid molecule or protein of interest, after the molecule(s) has been identified, is nucleic acid molecule design. A number of factors go into design of the nucleic acid sequence to be synthesized and the oligonucleotides used to generate the nucleic acid molecule. These factors include one or more of the following: (1) the AT/GC content of all or part of the nucleic acid molecule (*e.g.,* the coding region), (2) the presence or absence of restriction endonuclease cleavage sites (including the addition and/or removal of restriction sites), (3) preferred codon usage for the particular protein production or host expression system that is to be employed, (4) junctions of the oligonucleotides being assembled, (5) the number and lengths of the oligonucleotides used to produce the desired nucleic acid molecule, (6) minimization of undesirable regions (*e.g.,* "hairpin" sequences, regions of sequence homology to cellular nucleic acids, repetitive sequences, inhibitory cis-acting elements, restriction enzyme cleavage sites, internal splice sites etc.) and (7) coding region flanking segments that may be used for attachment of 5' and 3' components (*e.g.,* tags, linkers, restriction endonuclease sites, universal or specific primer binding sites, sequencing adaptors or barcodes, recombination sites, etc.).

In many instances, parameters will be input into a computer and software will generate an *in silico* nucleotide sequence that balances the input parameters. The software may place "weights" on the input parameters in that, for example, what is considered to be a nucleic acid molecule that closely matches some of the input criteria may be difficult or impossible to assemble. Exemplary nucleic acid design methods are set out in U.S. Patent No. 8,224,578. As further described below, the sequence design may also take into account requirements for multiplexing of oligonucleotides belonging to different nucleic acid fragments of a product nucleic acid molecule.

One main aspect of nucleic acid molecule design is the probability that the nucleic acid molecule can be produced in a "single-run". Put another way, the probability that a particular synthesis and assembly cycle will result in the generation of the designed nucleic acid molecule. This may be estimated by the use of data derived from past synthesis and assembly cycles and characteristics of the designed nucleic acid molecules. One factor that results in some synthesis assembly failures is high or low GC content. A number of nucleic acid design parameters are set out in Fath et al., PLoS One, 6:e17596 (2011).

Further, nucleic acid molecules design factors may be considered across the length of the nucleic acid molecule or in specific regions of the molecule. For example, GC content may be limited across the length of the nucleic acid molecule to prevent synthesis "failures" resulting from specific locations within the molecule. Thus, synthesizability of the nucleic acid molecule is a characteristic of the entire nucleic acid molecule in that a regional "failure to assemble" results in the designed nucleic acid molecule not being assembled. From a regional perspective, one or more codons may be selected for optimal translation but this may conflict with, for example, region limitation of GC content.

Assembly success often involves multiple parameters and regional characteristics of the desired nucleic acid molecule. Total and regional GC content is only one example of a parameter. For example, the total GC content of a nucleic acid molecule may be 50% but the GC content in a particular region of the same nucleic acid molecule may be 75%. Thus, in many instances, GC content will be "balanced" across the entire nucleic acid molecule and may vary regionally by less than 15%, 10%, 8%, 7%, or 5% from the total GC content.

Further, the designed nucleic acid molecule may be based upon a naturally occurring nucleic acid molecule and modifications are made to the naturally occurring nucleic acid molecule to enhance synthesizability. A synthetic gene ought therefore to be optimized in relation to the codon usage and the GC content and, on the other hand, substantially avoid the problems associated with DNA motifs and sequence repeats and inverse complementary sequence repeats. These requirements cannot, however, ordinarily be satisfied simultaneously and in an optimal manner. When a limited set of codons is used, often the repetitive nature of a coding sequence increases. Thus, alteration of a coding sequence to what is considered preferred codon usage may lead to a highly repetitive sequence and a considerable difference from the desired GC content. Further, coding regions designed to have either low GC or high GC content will exacerbate this problem by limiting the codons used by base type.

The aim therefore is to reach a compromise which is as optimal as possible between satisfying the various requirements. In instances where the product nucleic acid molecule encodes a protein, the large number of amino acids in the protein leads to a combinatorial explosion of the number of possible DNA sequences which - in principle - are able to express the desired protein based on the degeneracy of the genetic code. For this reason, various computer-assisted methods have been proposed for ascertaining an optimal codon sequence. Further factors which may influence the result of expression are DNA motifs and repeats or inverse complementary repeats in the base sequence. Certain base sequences produce in a given organism certain functions which may not be desired within a coding sequence. Examples are cis-active sequence motifs such as splice sites or transcription terminators. The presence of a particular motif may reduce or entirely suppress expression or even have a toxic effect on the host organism. Sequence repeats may lead to lower genetic stability and impede the synthesis of repetitive segments owing to the risk of incorrect hybridizations. Inverse complementary repeats may lead to the formation of unwanted secondary structures at the RNA level or cruciform structures at the DNA level, which impede transcription and lead to genetic instability, or may have an adverse effect on translation efficiency.

Repetitive sequence segments may, for example, lead to low genetic stability. The synthesis of repetitive segments is also made distinctly difficult because of the risk of faulty hybridization. Therefore, the assessment of a test sequence includes whether it comprises identical or mutually similar sequence segments at various points. The presence of corresponding segments can be established for example with the aid of a variant of a dynamic programming algorithm for generating a local alignment of the mutually similar sequence segments. Such an algorithm is set out in U.S. Patent No. 8,224,578. Typically, to calculate the criterion weight relating to the repetitive elements, the individual weights of all the local alignments where the alignment weight exceeds a certain threshold value are summed. Addition of these individual weights gives the criterion weight which characterizes the repetitiveness of the test sequence.

Inverse complementary repeats present the potential formation of secondary structures and the RNA level or cruciform structures at the DNA level can be recognized on the test sequence by the presence of such inverse complementary repeats (inverse repeats). Cruciform structures at the DNA level may impede translation and lead to genetic instability. Further, the formation of secondary structures at the RNA level may have adverse effects on translation efficiency. In this connection, inverse repeats of particular importance are those which form hairpin loops or cruciform structures. Faulty hybridizations or hairpin loops may also have adverse effects in the synthesis of the former from oligonucleotides.

Typically, nucleic acid molecules will be designed to have an estimated and/or actual synthesis and assembly cycle success rate of at least 90% (*e.g.,* from about 90% to about 99.5%, from about 92% to about 99.5%, from about 93% to about 99.5%, from about 94% to about 99.5%, from about 95% to about 99.5%, from about 90% to about 98%, from about 92% to about 98%, from about 93% to about 98.5%, from about 94% to about 98.5%, etc.).

As noted herein one important feature of the coding region is codon usage. Codon usage varies with a number of factors, including the particular organism and the type of gene being expressed within that organism. For example, it has been found in both Drosophila and Caenorhabditis that codon bias plays a major role in the selection of highly expressed genes. Further, "optimal" codons typically correspond to the tRNA molecules that are most abundant in a cell. These observations support the translation selection hypothesis which states that codon usage has been shaped by selection to improve the efficiency of translation of certain proteins. Thus, protein expression levels within cells appear to be partly controlled by the codons of the particular gene. Also, expression efficiency is also thought to increase with the use of preferred codons for a particular cell type and there is evidence that low-frequency-usage codons within a coding sequence provides for genetic instruction that regulates the rate of protein synthesis. (Reviewed in Angov, "Codon usage: Nature's roadmap to expression and folding of proteins, " Biotechnol. J, 6:650-659 (2011).)

Codon usage may be adjusted across a coding region and/or in portions of coding regions to match desired codons. In many instances, this will be done while maintaining a desired level of synthesizability.

The *in silico* design of the nucleic acid in terms of synthesizability may also include "fragmentation" of the full-length nucleic acid sequence into smaller fragments that can be assembled based on single-stranded oligonucleotides. Methods and systems for automated nucleic acid synthesis design are described for example in U.S. Patent No. 7,164,992. In most instances, oligonucleotides will provide the starting point for the methods and compositions underlying the present disclosure.

### Multiplex Assembly Design

Nucleic acid fragments obtained in multiplex assembly methods described herein may be intermediates or components of larger nucleic acid molecules and may vary in length. In some instances, nucleic acid fragments may have lengths that are compatible with the read length of the selected sequencing technology. For example where Personal Genome Machine (PGM) from Ion Torrent^{™} Systems, Inc. is used as sequencing platform, the starting fragments may be between about 200 and about 700 base pairs in length, or between about 300 and about 600 base pairs in length, which reflects the read length of the platform. However, other fragment sizes may be chosen for other sequencing platforms and can be selected by the skilled person depending on the respective read length obtained by the platform.

The nucleotide sequences of oligonucleotides used to assemble nucleic acid fragments in multiplex assembly reactions should be designed to lessen cross-hybridization between oligonucleotides, fragments and, when present, primers. In particular, oligonucleotides belonging to a first fragment should not hybridize to any of the oligonucleotides belonging to a second fragment if the first and second fragments are simultaneously assembled in the same reaction compartment. In particular, the sequences of the oligonucleotides belonging to the two or more fragments assembled in a given reaction compartment should not be too similar in certain regions (as described in more detail below) or too similar with a reverse complementary oligonucleotide of another fragment to avoid cross-hybridization between oligonucleotides of different fragments during assembly. For purpose of illustration only, in an example the 10 bases of the 3' end of a first fragment must not be identical or too similar or reverse complementary identical or too similar to a terminal end of a second fragment in the same compartment.

Bioinformatic considerations for oligonucleotide designs that allow for multiplexing of fragment assembly at limited complexity according to methods of the disclosure (*e.g.,* 2-10 nucleic acid fragments of about 1 kb each assembled from about 40 oligonucleotides of length 50 per fragment) are illustrated by the following example. As described elsewhere herein, overlapping oligonucleotides may be assembled using different conformations. One approach exemplified in FIG. 4A is to design a plurality of forward oligonucleotides f(S,1) to f(S, n(S)) that represent the full-length forward strand of a given fragment S, wherein the nicks between adjacent forward oligonucleotides are bridged by a plurality of complementary reverse oligonucleotides r(S,1) to r(S, n(S)-1) that together represent a portion of the reverse strand of fragment S. (It is noted that for simplicity, the S parameter has been omitted in FIG. 4A). In such examples, less reverse oligonucleotides than forward oligonucleotides are needed expressed by "n-1". In other examples, the number of forward and reverse oligonucleotides per fragment may be equal (f(S,1) to f(S, n(S)) and r(S,1) to r(S, n(S))). In yet other examples, more reverse than forward oligonucleotides may be required (f(S,1) to f(S, n(S)) and r(S,1) to r(S, n(S)+1)). In the example of FIG. 4A, the oligonucleotides are designed such that for a predetermined temperature T (*e.g.,* T = 50°C) for each i (wherein i ∈ {1,...,n-1}), forward oligonucleotide f(i) and the 3' segment of reverse oligonucleotide r(i), and oligonucleotide f(i+1) and the 5' segment of oligonucleotide r(i) hybridize. Whereas hybridization conditions should be optimal for overlapping oligonucleotides designed to assemble into the defined conformation of fragment S (*e.g.,* hybridization of r(1) with f(1) and f(2) in FIG. 4A), any other hybridization events between f and r oligonucleotides that result in unintended assembly products are to be avoided (*e.g.,* hybridization of r(1) with f(3) or f(4) etc.). To minimize the chance for such "mis-hybridizations" to occur, hybridization events can be rated using alignment techniques or other state-of-the-art techniques. Once the sequences of the forward oligonucleotides have been defined, the sequences of suitable reverse oligonucleotides can be computed using standard methods (such as the nearest neighbor algorithm) for calculating nucleic acid hybridization temperatures (*see, e.g.,* SantaLucia (1998). "A unified view of polymer, dumbbell, and oligonucleotide DNA nearest-neighbor thermodynamics". Proc. Natl. Acad. Sci. USA. 95 (4): 1460-5*;* Rychlik et al., (1990). "Optimization of the annealing temperature for DNA amplification in vitro". Nucleic Acids Res. 18 (21): 6409-6412).

As discussed elsewhere herein, oligonucleotides produced for nucleic acid assembly are typically within a certain length range. Assume for purposes of this example that a suitable oligonucleotide length for fragment assembly may be within a range of about 36 to about 60 nucleotides. To allow for efficient assembly, length differences between all forward oligonucleotides should be bounded by a certain constant, *e.g.,* 5 (indicating the maximum length difference between oligonucleotides). Furthermore, adjacent reverse oligonucleotides must not overlap (*e.g.,* oligonucleotides r(1) and r(2) or oligonucleotides r(2) and r(3) in FIG. 4A). In spite of these constraints, a multitude of possible oligonucleotide designs will be computed for given fragment S. For purpose of illustration assume fragment S has a length of 400 nucleotides that can be divided into eight forward oligonucleotides each of exact length 50. Alternatively, the same fragment S may be divided into eight oligonucleotides of lengths 48, 50, 52, 51, 49, 50, 47, 53, or seven oligonucleotides of lengths 56, 57, 59, 56, 57, 57, 58, or nine oligonucleotides of lengths 44, 44,..., 44, 46, 46, etc. Each bundle of choices leads to a different overall set of oligonucleotides and a different mass of possible oligonucleotide mis-hybridizations. Using the well-known technique of dynamic programming (*see, e.g.,* "Introduction to Algorithms", Third Edition (2009) by Cormen *et al*.) the skilled person can generate an array representation of all possible sum partitions of given length for fragment S, and by using randomization, the skilled person can generate a large number of different oligonucleotide designs, *i.e.,* generate many partitions of the fragment length into summands that are within a certain range. In fact, even different ranges can be tested, like [36..41], [37..42], ..., [55..60]. Thus, a large number of possible oligonucleotide set designs are obtained and each set can be evaluated for possible mis-hybridizations that would make the design an "unwanted" design. The skilled person would reject such unwanted designs and rather select a "desired" oligonucleotide design for given fragment S, wherein a desired design refers to a set of forward and reverse oligonucleotide sequences that together comprise a low computed likelihood of hybridizing in an unintended order. For example, a given set of oligonucleotide sequences may be selected based on a combination of scores, where the resulting scores are correlated with the probability for mis-hybridization events.

For multiplexing two or more fragments, the exercise needs to be extended to oligonucleotides belonging to different fragments. For example, for two fragments S(1) and S(2) to be assembled from two oligonucleotide sets O(1) and O(2) in the same reaction mixture, cross-hybridization between oligonucleotides designed for S(1) assembly with oligonucleotides designed for S(2) assembly should be excluded. If the computed "mis-hybridization score" for oligonucleotides between both sets indicates "no mis-hybridization", oligonucleotide set O(1) and oligonucleotide set O(2) can be pooled into the same assembly reaction.

Thus, in one aspect the disclosure provides oligonucleotide sets O(1) to O(m), that allow for as many of a given set of fragments S(1) to S(m), to be multiplexed in a single reaction (*i.e.,* to be assembled in the same reaction mixture) without generating mis-hybridized reaction products. By increasing the number of fragments assembled in a single reaction, the overall amount of separate pooling steps and therefore the time required to pool all fragment assembly components (*e.g.,* from a synthesis chip) is significantly reduced. By way of example, if 100 fragments can be assembled in 10 multiplex reactions, a 10-fold saving of pooling time and assembly reaction mixtures is achieved.

After having chosen an oligonucleotide set with suitable assembly design (*i.e.,* comprising a "no mis-hybridization score") for each fragment, a mis-hybridization score is determined for each combination of two oligonucleotide sets to determine, whether two or more of the oligonucleotide sets can be pooled together. Such analysis can be facilitated by a computer program generating an undirected graph with a node for each oligonucleotide set as illustrated in FIG. 4B. FIG. 4B shows an undirected graph on 4 nodes 1 to 4, representing oligonucleotide sets O(1) to O(4) for assembly of fragments S(1) to S(4). An edge between two oligonucleotide sets belonging to two distinct fragments indicates that they have a desired cross-hybridization score and can be multiplexed (*i.e.,* pooled together in the same reaction mixture). In this example, oligonucleotide sets for S(1) and S(2) or oligonucleotide sets for S(2) and S(3) can be multiplexed, as there is an edge between nodes 1 and 2, and between nodes 2 and 3, whereas oligonucleotide sets for S(1) and S(4) cannot be multiplexed, as there is no edge between nodes 1 and 4. Thus, according to this exemplary illustration, a first set of oligonucleotides can be pooled with a second set of oligonucleotides if the sets are connected by an edge. More generally, a number of m sets of oligonucleotides can be pooled together if any pair of the m sets is connected by an edge, *i.e.,* they form a so-called "clique". In this example, oligonucleotide sets O(2), O(3), and O(4) represent a clique and can be pooled together to assemble fragments S(2), S(3) and S(4) in a single reaction. Any so-called clique cover (*i.e.,* a set of disjoint cliques that cover all nodes) of the undirected graph with k cliques results in a pool with k pools, so a clique cover with k as small as possible is desired. It is also understood that the clique size is bounded by the pool size. In the example of FIG. 4B, {1}, {2,3,4} is a clique cover as well as {1,2}, {3,4}. Thus, fragments S(1) to S(4) can be assembled by pooling oligonucleotide sets O(1) and O(2) together, and by separately pooling oligonucleotide sets O(3) and O(4) together (as indicated by the two dotted boxes in FIG. 4B). It is understood by a person skilled in the art that the above problem illustrated by a "clique cover" approach can likewise be reformulated as a problem using independent sets, a coloring approach (as described elsewhere herein) or other notions (see *e.g.* "Introduction to Algorithms", Third Edition (2009) by Cormen *et al*.).

In order to compute a clique cover with a small number of cliques, any approach known by the skilled person can be applied (*see, e.g.,* Gramm et al., "Data Reduction, Exact, and Heuristic Algorithms for Clique Cover, PROC. 8TH ALENEX-06, pp. 86-94, SIAM, 2006). For example, a so-called "greedy" approach (that decides which next step will provide the most obvious benefit for a complex problem) may be used for identifying a large clique, and any large clique may be removed from the graph until a suitable clique cover is obtained. Using randomization, many such covers can be generated and the most suitable one (taking assembly requirements into account) can be selected. Furthermore, to evaluate a sufficient number of possibilities, a larger number of graphs can be generated by starting with multiple different sequence designs for each oligonucleotide set, compute clique cover for the plurality of them, and obtain a large set of pooling solutions from which the one with the minimum number of pools can be selected.

In various examples, the design or selection of a set of oligonucleotides for a given fragment can be based on the probabilities of the underlying oligonucleotides to hybridize in a predetermined order. For example, a score can be assigned to every pair of oligonucleotides indicating the likelihood of such pair to hybridize. The quality of the design of an entire set of oligonucleotides may then be determined by calculating a total score for the set based on the individual scores obtained for each pair. However, the individual scores may need to be weighted because the pair scores do not indicate whether the oligonucleotides in a given set are intended to hybridize in a desired order. For example, one would avoid selecting a set of oligonucleotides that comprises oligonucleotide pairs with a high hybridization score that would likely hybridize in an undesired order. Options to determine a total score for a given set of oligonucleotides include the following: In a first embodiment, the individual scores of all oligonucleotide pairs are summed up (wherein the intended hybridizations are weighted positively and the unintended hybridizations are weighted negatively) and an oligonucleotide set together yielding the highest total score is selected. In a second embodiment one may define a threshold value that must not be exceeded by the plurality of scores of unintended hybridizations. (For example and for purposes of illustration only, one could define the probability of unintended hybridizations occurring as being below 1 in 10,000). Thus, according to the second embodiment, one would not sum up the individual scores to obtain a total score but rather determine the maximum of the scores of the "undesired" hybridizations and keep such maximum as small as possible.

A computerized method for optimizing oligonucleotide sequence design for multiplex nucleic acid fragment assembly according to methods of the disclosure may therefore comprise the following steps conducted on a computer: (i) dividing *in silico,* the desired nucleic acid molecule into a plurality of fragments, (ii) generating *in silico,* multiple sets of oligonucleotide sequences for each fragment, wherein each set of oligonucleotide sequences comprises a plurality of forward sequences and a plurality of reverse sequences, wherein the forward and reverse sequences of each set are intended to hybridize in a predetermined order, (iii) assigning *in silico* a score to each pair of oligonucleotide sequences within the multiple sets of oligonucleotide sequences generated *in silico* (including pairs of forward and reverse sequences, pairs of forward and forward sequences, and pairs of reverse and reverse sequences), wherein the score indicates the probability of hybridization between two oligonucleotides, (iv) determining *in silico* a total score for each set of oligonucleotide sequences by (a) summing up the individual scores assigned to all pairs of oligonucleotide sequences which are intended to hybridize in the predetermined order and subtracting the individual scores assigned to all pairs of oligonucleotide sequences which are not intended to hybridize in the predetermined order, or by (b) selecting the maximum scores of all pairs of oligonucleotide sequences that are not intended to hybridize, and (v) selecting for each fragment at least one set of oligonucleotide sequences from the plurality of generated sets of oligonucleotide sequences that together yield the highest total score according to step (iv) (a) or together yield the lowest total score according to step (iv) (b).

In an alternative example, the computerized method for optimizing oligonucleotide sequence design for multiplex nucleic acid fragment assembly may comprise the steps of: (i) generating *in silico,* multiple sets of oligonucleotide sequences for a desired nucleic acid sequence, wherein each set of oligonucleotide sequences comprises a plurality of forward sequences and a plurality of reverse sequences, wherein forward and reverse sequences are intended to hybridize in a predetermined order, (ii) assigning *in silico,* a score to each pair of two oligonucleotide sequences within the multiple sets of oligonucleotide sequences generated *in silico* (including pairs of forward and reverse sequences, pairs of forward and forward sequences, and pairs of reverse and reverse sequences), wherein the score indicates the probability of hybridization between two oligonucleotides, (iii) determining *in silico* a total score for each set of oligonucleotide sequences by (a) summing up the individual scores assigned to all pairs of oligonucleotide sequences which are intended to hybridize in the predetermined order and subtracting the individual scores assigned to all pairs of oligonucleotide sequences which are not intended to hybridize in the predetermined order, or by (b) selecting the maximum scores of all pairs of oligonucleotide sequences that are not intended to hybridize, (iv) dividing *in silico,* the desired nucleic acid molecule into a plurality of oligonucleotide sets, each oligonucleotide set defining a fragment of the desired nucleic acid sequence, wherein each oligonucleotide set is selected based on the highest total score determined according to step (iii) (a) or based on the lowest total score determined according to step (iii) (b).

The above alternative methods may further comprise assigning *in silico,* a score to each combination of selected sets of oligonucleotide sequences within the pool of selected sets, wherein the score indicates the probability of cross-hybridization between forward and/or reverse oligonucleotides of a first set of oligonucleotide sequences with forward and/or reverse oligonucleotides of a second set of oligonucleotide sequences, and selecting two or more sets of oligonucleotide sequences with a desired score for multiplex assembly of two or more fragments from the plurality of fragments.

The methods may further comprise synthesizing the selected sets of oligonucleotides, optionally wherein the synthesizing is conducted on a microarray or microchip.

The methods may further comprise pooling the selected two or more sets of oligonucleotide sequences and assembling the pooled sets of oligonucleotide sequences into two or more fragments.

The methods may further comprise pooling the two or more assembled fragments, and assembling the two or more assembled fragments or a fraction thereof to generate the desired nucleic acid molecule.

The disclosure further comprises a computer program product for use in conjunction with a computer system, the computer program product comprising a non-transitory computer readable storage medium and a computer program mechanism embedded therein, the computer program mechanism comprising instructions for performing a computerized method for optimizing oligonucleotide sequence design for multiplex nucleic acid fragment assembly according to one or more of the above methods.

### Linkers and Tags

Oligonucleotide and fragment design may also take into account terminal or flanking sequences such as linkers or tags.

Linkers or linker regions as used herein refer to terminal regions of nucleic acid fragments suitable for linking a nucleic acid fragment with another nucleic acid fragment or functional entity. For example a linker can comprise one or more of a primer binding site, a restriction enzyme cleavage site in any combination or order. In some instances a linker region contains one or more universal primer binding site (*i.e.* a primer binding site that is common for all or a sub-set of nucleic acid fragments). In some instances a linker region contains one or more specific primer binding sites (*i.e.* a primer binding site that is specific for an individual nucleic acid fragment or a sub-set of nucleic acid fragments). A linker region may thus be used to connect or attach another nucleic acid molecule via fusion PCR using the universal or specific primer binding sites therein (often referred to as "tagging"). For example, tags (optionally containing barcodes and/or adaptors) with a region complementary to such primer binding site in a linker region may be attached by PCR. In some examples, a linker region may further comprise one or more scissile bonds or restriction enzyme cleavage sites (*e.g.* a type IIs cleavage site) which may be the same or different in two terminal linker regions of a nucleic acid fragment. In some instances the recognition site for a type IIs cleavage site is located outside the linker region. In other instances the recognition site for a restriction enzyme is located within the linker region. The linker of a nucleic acid fragment may further comprise a region that overlaps with or has complementarity with a region of another nucleic acid fragment. For example, if a linker region is cleaved with a restriction enzyme leaving a single-stranded overhang, the overhang may hybridize to a complementary single-stranded overhang of another (cleaved) nucleic acid molecule (*e.g.* the linker region of another fragment) thereby facilitating linkage of both molecules.

Linker regions may be added to or incorporated into nucleic acid fragments by ligation or amplification or during assembly of the nucleic acid fragment. For example, oligonucleotides representing termini of a nucleic acid fragment may be designed and synthesized with linker regions at their 5' ends. During assembly (*e.g*. by PCR or ligation) the forward and reverse oligonucleotides that are components of a specific fragment hybridize in a predefined order with those carrying linker regions hybridizing at both termini. Thus, oligonucleotides that form termini of nucleic acid fragments may comprise a linker region at one end (*e.g.* the 5'-end) whereas oligonucleotides that form internal regions of nucleic acid fragments may not comprise such linker region. A linker region suitable for methods described herein can be any length, including lengths between 4 and 200 nucleotides or base-pairs, between 10 and 100 nucleotides or base-pairs, between 20 and 50 nucleotides or base-pairs, etc.

Oligonucleotides or fragments may also be designed to comprise one or more tags. A tag refers to a sequence region or nucleic acid molecule that is used for amplification, processing, selection, analysis or detection of a nucleic acid or intermediate but is in many instances not included in the desired target nucleic acid molecule.

Adding one or more tags to nucleic acid molecules for downstream processing or analysis is well established. For example restriction enzyme recognition and/or cleavage sites can be designed and added to the 5' and/or 3' end of an assembly region of a given nucleic acid fragment to allow cleavage and assembly as described elsewhere herein . (For example, a restriction enzyme recognition site may be added to the 5'end of a given sense strand of a fragment and the reverse complement of the recognition sequence may be added to the 3' end). In addition, sequencing adaptors and/or barcodes may be attached for subsequent sequencing and analysis of individual molecules or sets of molecules. Thus, tags are typically added or attached to a nucleic acid molecule to confer a certain function to the molecule that is required in a downstream workflow or processing step.

Tags may consist of or comprise one or more segments of different functionality including one or more enzyme recognition or cleavage sites, one or more barcodes and/or one or more adaptors and/or one or more linker regions.

Tags according to various embodiments may be single-stranded or double-stranded. In some examples, at least one end of a double-stranded tag is a blunt end or an overhang end, including a 5' or 3' overhang end. In some examples, a tag or a segment thereof can include a nucleotide sequence that is identical or complementary to any portion of a nucleic acid molecule. Tags suitable for methods described herein can be any length, including lengths between 2-2000 nucleotides or base-pairs, between 5 and 500 nucleotides or base-pairs, between 10 and 100 nucleotides or base-pairs, etc. In instances, a tag can be about 100-200 nucleotides or longer.

Tags or segments thereof may be removable from nucleic acid molecules after use. In some examples, a tag can include at least one scissile linkage that can be susceptible to cleavage or degradation by an enzyme or chemical compound. Optionally, the tag includes at least one base that is a substrate for a DNA glycosylase. For example, the tag may include a uracil base that can be excised with uracil N-glycosylase (UNG), upon which the backbone may be cleaved at the abasic site by formamidopyrimidine DNA glycosylase (Fpg). In some examples, the tag can include at least one phosphorothiolate, phosphorothioate, and/or phosphoramidite linkage. In some examples, a tag can include any type of restriction enzyme recognition sequence, including type I, type II, type IIs, type IIB, type III, type IV restriction enzyme recognition sequences, or recognition sequences having palindromic or non-palindromic recognition sequences.

Whereas it is well established that tags can be added or attached to nucleic acid molecules to confer a certain functional property to nucleic acids, it has been found, that one or more tags can be added to a sequence to improve sequence properties so that possible production problems (*e.g*. inefficient multiplex assembly or amplification) are avoided. Where sequences turn out to be difficult to synthesize, assemble or express, the degeneracy of the genetic code is typically used to modify the primary sequence e.g. by replacing certain codons or single bases to introduce silent mutations or change the overall GC content or repetitive elements that may interfere with production as set out above. Silent mutations are, however, not available in cases where a target nucleic acid molecule is produced based on a predefined nucleic acid sequence (*e.g.* a "wildtype sequence") and no redesign or modification of the sequence is desired. Although the method proposed herein does not require editing of the final target sequence, it is based on the possibility to redesign and modify intermediates of a target nucleic acid (*e.g.* nucleic acid fragments) thereby increasing the success rate of correct assembly reactions.

Thus, methods disclosed herein make use of "assembly tags" designed to confer certain sequence properties to a nucleic acid molecule (*e.g.* an oligonucleotide or nucleic acid fragment) to facilitate its assembly. In some instances an assembly tag may be attached to a nucleic acid molecule to change or adapt its overall sequence properties or compensate for sub-optimal base composition or sequence length. Depending on sequence requirements, an assembly tag may be attached to the 5'-end or the 3'-end or to both ends of a nucleic acid molecule. Also, tags attached to the 5' and 3' ends may be identical or may differ in length and/or base composition. For example, an assembly tag can be attached to one or both ends of a fragment to increase its assembly success rate and can be removed before a desired final nucleic acid product nucleic acid is assembled, cloned or isolated. Thus, assembly tags are preferably removable once the intermediates or sub-fragments have been assembled and do therefore not form part of the final target sequence. Removal of (assembly) tags can be achieved in many ways. In some instances, a tag may be removed from a nucleic acid fragment via cleavage (*e.g*. restriction enzyme cleavage) or may be excluded from downstream processing steps using nested PCR with primers that bind inside of terminal tags.

Assembly tags may be configured to adjust various parameters and may be attached or incorporated into fragments as described more generally for linkers or tags above (*e.g.* included in terminal oligonucleotides used for fragment assembly or appended by PCR or ligation).

It has been shown for example that fragment length is critical for the success of multiplex fragment assembly reactions. Nucleic acid fragments are generally co-assembled (in the same pool) at higher efficiency if they have a similar length. Thus, in some instances, a "length tag" may be attached to a short nucleic acid fragment to extend its length to or beyond a defined minimal length that is determined for a given fragment assembly pool. An example of a length tag is provided in Fig. 5 showing hypothetical Fragment A equipped with terminal length tags (indicated by multiple "Ns" representing random base composition). Both length tags may further comprise a restriction enzyme cleavage site or other scissile bond for removal downstream of fragment assembly.

Alternatively, a "GC tag" with a predefined GC content may be attached to a nucleic acid fragment to increase or decrease its overall GC content.

It has been observed that the overall GC content of a nucleic acid fragment affects the efficiency of its assembly in a multiplex assembly reaction. In certain instances fragments with an overall GC content close to 55% are easier to assemble or easier to handle in downstream workflows (such as sequencing) than fragments with higher or lower GC contents. In particular the GC content of fragments that are co-assembled from sets of oligonucleotides are preferably within a range of between about 40% and about 60%, or between about 50% and about 57%. In some instances, the GC content of a fragment has a GC content of at least about 40%. In some instances, the GC content is close to or about 55%. Nucleic acid fragments are generally co-assembled (in the same pool) at higher efficiency if they have a similar GC content. Furthermore, it has been found that the overall GC content of a sequence is more critical than the local GC content (*i.e.,* the GC content within a smaller sequence window). Where the overall GC content varies substantially between fragments that are to be co-assembled in the same reaction compartment, one or more "GC tags" may be added to such fragment to equilibrate or correct the overall GC content thereby increasing the efficiency of multiple fragment assembly. An example of a GC tag is provided in Fig. 5 showing hypothetical Fragment B equipped with terminal GC tags comprising a predefined GC content or base composition. Both GC tags may further comprise a restriction enzyme cleavage site or other scissile bond for removal downstream of fragment assembly.

Thus, various methods for multiplex nucleic acid fragment assembly disclosed herein include the design of assembly tags to (i) shift the overall GC content of a fragment to a desired range (*e.g*. between about 50% and about 60% total GC), and/or (ii) adjust the length of a fragment to a desired minimal length or a predefined length range. In some instances an assembly tag may be designed to achieve (i) and (ii) at the same time, *e.g.* by increasing the GC content and at the same time extending the length of a given fragment. Also, the length of a GC tag may be restricted to keep the tagged fragment inside a predefined length range.

When designing one or more assembly tags, the introduction of unwanted sequence motifs or properties that may affect assembly or downstream processes should be avoided. For example, the addition of one or more assembly tags should not result in the introduction of long homopolymers (*e.g*. stretches of identical bases of a certain length), or unwanted restriction enzyme recognition sites or other motifs that may affect or interfere with downstream processing steps.

Assembly tags may be designed in various ways. For example, an assembly tag (or a pair of tags) could be designed with a high GC content to increase the GC content of a fragment sequence that has a low overall GC content (*e.g.* a GC content of < 35% or below 40%). GC tags may be designed using different algorithms such as for example a randomized or deterministic algorithm. As an example for a deterministic approach, a list of carefully predesigned 5' and 3' tags could be generated and the best combination selected. Alternatively, randomized approaches such as evolutionary algorithms, genetic programming or ant-colony optimization are well known by those skilled in the art and can be used for assembly tag design.

In one example, an assembly tag sequence with a high GC content could be obtained by choosing a randomly generated sequence where "Gs" and "Cs" are generated with a probability of 40% each while "As" and "Ts" are generated with a probability of 10 % each. More generally, a sequence may be generated by randomly choosing bases with probabilities p(A), p(C), p(G), and p(T), wherein the probabilities p(X) are predefined. As this can be computed in high throughput fashion, thousands of such sequences may be generated and may be checked for additional requirements (*e.g*. homopolymers, cleavage sites etc.) to identify the optimal tag or pair of tags. Also, the length of the tag(s) should be chosen so that the overall fragment length is within the desired length range or does not deviate from the predetermined length range by more than 1% or more than 2% or more than 5%.

For purpose of illustration, a given fragment may have a length of 200 bases and a GC content of 40% (*i.e.,* 80 "Gs" or "Cs" and 120 "As" or "Ts"). Assuming that the optimal GC range for a fragment would be between 45% and 60% and the optimal length range would be between 250 and 500 bases, the fragment could be modified with an assembly tag to optimize its sequence properties. To increase the overall GC content, a first GC tag may be added to the 5'-end (*e.g*. upstream of a restriction enzyme recognition site embedded in a linker sequence). The 5' GC tag may have a sequence of length 50 consisting of 35 "Gs" and "Cs" and 15 "As" or "Ts". Provided the same rules are used for a 3' GC tag, the resulting tagged fragment would comprise a sequence of length 300 with 150 "Gs" or "Cs" and 150 "As" or "Ts" yielding an overall GC content of 50%. By using assembly tags of the disclosure, both the overall GC content and length range of the fragment can be shifted to a preferred range (*i.e.* 50% GC and length of 300 in this example) thereby allowing a more reliable and efficient assembly.

In many instances, oligonucleotides for multiplex fragment assembly may be designed such that all fragments assembled in the same reaction compartment comprise substantially identical 5'- and/or 3'-ends. It will be understood that "substantially identical" as used herein means that, although sequences are intended to be identical, there may be some errors in synthesized nucleic acid molecules resulting in less than 100% identity. For example, two molecules can be considered substantially identical, if the percent identity between the two molecules is at least 75%, 80%, 85%, 90%, 95%, 98%, 99%, 99.9% or greater, when optimally aligned.

Some or all fragments assembled in the same compartment may contain functional elements such as universal primer binding sites and/or restriction enzyme cleavage sites or homologous regions, allowing for simultaneous amplification with a universal pair of primers in a subsequent PCR reaction and/or simultaneous processing in a downstream assembly or cloning step. Thus, in some instances an oligonucleotide belonging to a first fragment may contain the same or substantially the same 5'end as an oligonucleotide belonging to a different second fragment. Likewise, in some instances an oligonucleotide belonging to a first fragment may have the same or substantially the same 3'end as an oligonucleotide belonging to a different second fragment. In many instances only a sub-portion of the oligonucleotides provided for multiplex assembly in a pool will comprise the same 5'- or 3' end.

In some instances, each fragment assembled from a sub-set of oligonucleotides comprises a universal primer binding site and/or a restriction enzyme cleavage site. The universal primer binding site may be used in downstream workflows for simultaneous amplification of all or a sub-set of assembled nucleic acid fragments. For example, where fragments will be tagged and prepared for sequencing as described elsewhere herein, a universal primer binding site can be used to conjugate barcodes and/or sequencing adaptors via fusion PCR. Universal primer binding sites may also be used for normalizing the fragments in a mixed pool of fragments (*e.g*., prior to a tagging step). In such instance, a set of diverse primer binding sites may be used as set out in more detail below.

### Oligonucleotide Synthesis and Processing

Oligonucleotides or nucleic acid fragments used for assembly of a desired nucleic acid molecule may be derived from a number of sources, for example, they may be cloned, derived from polymerase chain reactions, chemically or enzymatically synthesized or purchased. Oligonucleotides suitable for use in the methods disclosed herein may be obtained *e.g.,* by synthesizing on a microarray or microchip as disclosed in PCT Publications WO 2013/049227 and WO 2016/094512. In many instances, chemically synthesized nucleic acids tend to be of less than 200 nucleotides in length. PCR and cloning can be used to generate much longer nucleic acids. Further, the percentage of erroneous bases present in nucleic acids (*e.g.,* nucleic acid fragments) is, to some extent, tied to the method by which it is made. Typically, chemically synthesized nucleic acids have the highest error rate.

A number of methods for chemical synthesis of oligonucleotides are known. In many instances, oligonucleotide synthesis is performed by a stepwise addition of nucleotides to the 5'-end of the growing chain until oligonucleotides of desired length and sequence are obtained. Further, each nucleotide addition can be referred to as a synthesis cycle and often consists of four chemical reactions: (1) De-Blocking/De-Protection, (2) Coupling, (3) Capping, and (4) Oxidation. One example of chemical oligonucleotide synthesis chemical processes is set out below.

Nucleic acid synthesis typically starts with selection of synthesis components or "building blocks" used to produce oligonucleotides. Often 3'-O-(N,N-diisopropyl phosphoramidite) derivatives of nucleosides (nucleoside phosphoramidites) are used as building blocks in phosphite triester methodologies. To prevent undesired side reactions, all other functional groups present in the nucleosides are normally rendered unreactive (protected) by the attachment of protecting groups. Upon oligonucleotide synthesis completion, remaining protecting groups are typically removed to yield the desired oligonucleotides free of protein groups.

5'-hydroxyl groups are often protected by an acid-labile DMT (4,4'-dimethoxytrityl) group. Phosphite group are often protected by a base-labile 2-cyanoethyl group.

De-blocking/De-protection: DMT groups are often removed with a solution of one or more acids, such as trichloroacetic acid (TCA) or dichloroacetic acid (DCA), present in an inert solvent (dichloromethane or toluene), resulting in the solid support-bound oligonucleotide precursor bearing a free 5'-terminal hydroxyl group. A washing step typically follows.

Coupling: In an exemplary process, a solution of nucleoside phosphoramidite in acetonitrile is activated by a solution of an acidic azole catalyst (*e.g.,* 1 H-tetrazole, 2-ethylthiotetrazole, 2-benzylthiotetrazole, or 4,5-dicyanoimidazole) and then brought in contact with the solid support where free 5'-hydroxy group reacts with the activated phosphoramidite moiety of the incoming nucleoside phosphoramidite to form a phosphite triester linkage. The coupling reaction of 2'-deoxynucleoside phosphoramidites is rapid and goes to near completion in about 20 seconds. Upon the completion of the coupling, unbound reagents and by-products are typically removed by washing.

Capping: Typically, after the completion of the coupling reaction, a small percentage (generally in the range of 0.1 to 1%) of the solid support-bound 5'-OH groups is unreacted and must be permanently blocked from further chain elongation so as to prevent the formation of oligonucleotides with an internal base deletion commonly referred to as (n-1) shortmers. Unreacted 5'-hydroxy groups are, to a large extent, acetylated by the capping mixture. Capping steps are often performed by treating the solid support-bound material with a mixture of acetic anhydride and 1-methylimidazole.

Oxidation: The newly formed tricoordinated phosphite triester linkage typically formed by chemical synthesis is not natural and is relatively unstable. Treatment with reagents containing iodine and water in the presence of a weak base (*e.g.,* pyridine, lutidine, or collidine) oxidizes the phosphite triester linkage into a tetracoordinated phosphate triester, which is a protected precursor of naturally occurring phosphodiester internucleosidic linkages.

A number of variations to the oligonucleotide synthesis process may be made. For example, electrochemically generated acid (EGA) or photogenerated acid (PGA) may be used to remove the protecting group (*e.g.,* DMT) before the next amidite is added to the nucleic acid molecule attached to the solid support. In some examples, at least one proton carrier, such as 2-chloro-6-methylpyridine or diphenylamine, may be present in the solution with the EGA or PGA. The at least one proton carrier may act to reduce the effect of DNA degradation by accepting protons from the EGA or PGA, thereby adjusting the acidity of the solution.

EGA and PGA deprotection reagents and methods for generating such acids, as well as their use in oligonucleotide synthesis are set out for example in Maurer et al., "Electrochemically Generated Acid and Its Containment to 100 Micron Reaction Areas for the Production of DNA Microarrays", PLoS, Issue 1, e34 (2006), or in PCT Publications WO 2013/049227 and WO 2016/094512.

While in many instances oligonucleotides may be produced using phosphoramidite synthesis chemistry, as well as variations thereof, other methods may also be used to produce oligonucleotides, including PCR, restriction enzyme digest, exonuclease treatment, or template-independent synthesis using a nucleotidyl transferase enzyme. Exemplary methods of template-independent synthesis using a nucleotidyl transferase enzyme are set out in U.S. Patent No. 8,808,989. The nucleotidyl transferase enzyme (*e.g.,* terminal deoxynucleotidyl transferase) is used to incorporate nucleotide analogs having an unmodified 3' hydroxyl and a cleavable protecting group. Because of the protecting group, synthesis pauses with the addition of each new base, whereupon the protecting group is cleaved, leaving a polynucleotide that is essentially identical to a naturally occurring nucleotide (*i.e.,* is recognized by the enzyme as a substrate for further nucleotide addition). Other methods of enzymatic nucleic acid synthesis are disclosed *e.g.* in WO 2017/176541 or WO 2017/223517. Thus, in certain examples, the disclosure includes methods in which oligonucleotides are produced by enzymatic reaction.

Nucleotide triphosphates (*e.g.,* deoxynucleotide triphosphates) (NTPs) suitable for use with enzymatic oligonucleotide synthesis methods will have protecting groups that do not prevent the NTPs from being used by a nucleotidyl transferase as a substrate and can be efficiently removed to allow for addition to an oligonucleotide chain. Thus, in certain examples, the nucleotide addition occurs via enzymatic reaction. In some instances, EGA is generated as part of the deprotection process. Further, in certain instances, all or part of the oligonucleotide synthesis reaction may be performed in aqueous solutions. In other instances, organic solvents will be used.

During synthesis, oligonucleotides are typically attached to one or more solid support (*e.g.* a matrix, a resin, a gel, the surface of an array etc.). In standard procedures, each oligonucleotide required to assemble a longer nucleic acid molecule is typically synthesized in excess. However, in instances where oligonucleotides are synthesized on 2-dimensional microarray or chip-based platforms at small scale, oligonucleotides may need to be amplified with pool-specific or universal primers (*e.g.* off the chip or following release from the platform) to obtain sufficient amounts of oligonucleotides for subsequent assembly reactions. This has the disadvantage that (i) all oligonucleotides synthesized on the chip or array must be designed with one or more flanking primer binding and/or restriction enzyme cleavage sites for subsequent amplification and processing prior to assembly and (ii) additional steps are needed to amplify and cleave (and optionally purify) the oligonucleotides before they can be used in the assembly reaction. This can be avoided by using three-dimensional solid supports (*e.g.* a porous matrix, microparticles or beads) optionally comprising a porous surface for oligonucleotide synthesis at sufficient quantities in the pores of the support. In some examples a platform as described in PCT Publications WO 2013/049227 and WO 2016/094512 may be used for methods described herein where oligonucleotides are synthesized on porous beads in wells of a chip.

In some examples oligonucleotides are attached to one or more support via a succinyl linker. In certain examples, a universal linker may be located between the succinyl group and the oligonucleotides. Alternatively, the linker may have a specific base attached as the starting base of the oligonucleotide. In such instances, solid supports (*e.g.,* once having a dA, dT, dC, dG, or dU) are selected based upon the starting base of the oligonucleotide being synthesized.

Once the oligonucleotide synthesis has been completed, the resulting oligonucleotides are typically subjected to a series of post processing steps that may include one or more of the following: (a) cleavage of the oligonucleotides or elution from the support, (b) concentration measurement, (c) concentration adjustment or dilution of oligonucleotide solutions, often referred to as "normalization", to obtain equally concentrated dilutions of each oligonucleotide species, and, optionally, (d) pooling or mixing aliquots of two or more normalized oligonucleotide samples to obtain equimolar mixtures of all oligonucleotides required to assemble one or more specific nucleic acid molecules, wherein the aforementioned steps may be combined in different orders.

The method used to cleave or elute the oligonucleotides from a support is often specific for the linking group through which the oligonucleotide is linked to the solid support. Typically, oligonucleotides are cleaved from solid supports using, for example, gaseous ammonia, aqueous ammonium hydroxide, aqueous methylamine, or mixtures thereof. A succinyl linker may be cleaved by the use of, for example, concentrated aqueous ammonium hydroxide. The reaction is usually carried out at temperatures between 50°C and 80°C for at least one to about eight hours. In certain examples, the succinyl linker may be cleaved by the use of ammonia gas, using increased heat and pressure, such as, for example, a temperature of about 80°C, and a pressure of about 3 bars for a time of about 2 hours.

The concentration of individual oligonucleotides or mixtures of oligonucleotides can be determined by optical density measurement. Alternatively, yields of oligonucleotides immobilized on solid supports may be determined using methods as described *e.g.* in WO 2017/100283. After appropriate dilution of a required oligonucleotide solution, equal amounts or volumes of each diluted solution can be pooled for further multiplex processing, *e.g.,* by manual or automated pipetting or by using systems as described below.

In some instances, oligonucleotides will be released from synthesis supports and then pooled as unbound nucleic acid molecules for subsequent assembly steps. In some examples, oligonucleotides attached to individual or "loose" solid supports (such as microparticles or beads) may be pooled before they are released as described in more detail below. For example, the oligonucleotides are synthesized on solid supports that are located at addressable positions of a common carrier (*e.g.* a microchip) and can be selectively released by releasing the respective solid support from the carrier. Supports carrying assembly oligonucleotides for multiple nucleic acid fragments may be collected in a single compartment for further processing (*e.g.,* cleavage from the supports, washing, concentration measurement etc.). Thus, pooled oligonucleotides may be components of a single fragment to be assembled or may be components of multiple fragments to be assembled simultaneously in a single reaction compartment ("multiplex assembly") as described in more detail below.

### Selective Retrieval and Pooling of Oligonucleotides

In the practice of methods of the disclosure, populations of oligonucleotides may be generated where each population contains oligonucleotides designed to be sub-components of multiple assembly products (*e.g*. nucleic acid fragments). These oligonucleotides may be sorted to decrease the complexity of assembly reaction mixtures with the resulting oligonucleotide mixture to form a sorted oligonucleotide population. As examples, the number of oligonucleotides may be designed to be sub-components of from about 2 to about 500 (*e.g.,* from about 3 to about 500, from about 5 to about 500, from about 10 to about 500, from about 20 to about 500, from about 30 to about 500, from about 50 to about 500, from about 75 to about 500, from about 100 to about 500, from about 150 to about 500, from about 200 to about 500, from about 75 to about 250, etc.) or more nucleic acid fragments.

Further, populations of oligonucleotides may be sorted into (i) specified numbers of sorted oligonucleotide populations (*e.g.,* from about 1 to about 300, from about 5 to about 300, from about 10 to about 100, from about **1** to about 10, from about 1 to about 100, from about 10 to about 75, from about 20 to about 300, from about 25 to about 400, etc.) (ii) sorted oligonucleotide populations that contain specific numbers of different oligonucleotides (*e.g.,* from about 100 to about 2,000, from about 200 to about 2,000, from about 500 to about 2,000, from about 750 to about 2,000, from about 750 to about 3,000, etc.), (iii) sorted oligonucleotide populations that contain oligonucleotides designed for the assembly of a specified number of product nucleic acid molecules.

In some circumstances, a stepwise release or sorting of oligonucleotides obtained from a microarray or chip-based platform may be limited in terms of miniaturization, or be too time consuming to execute, and may therefore be of limited use in high throughput applications. Further, some chip synthesis platforms do not allow for selective release of individual oligonucleotides.

An issue arises with chip- or array-based oligonucleotide synthesis platforms in that, typically, the synthesized oligonucleotides are released simultaneously from the chip. This results in a highly complex mixture of oligonucleotides that are assembly components for multiple, different nucleic acid fragments. Highly complex mixtures of oligonucleotides often lead to failed assembly reactions due to unintended hybridization of the nucleic acid molecules intended to form a single assembly product.

The complexity of a population of oligonucleotides is, in part, determined by the number of different oligonucleotides present. In some instances, the number of oligonucleotides present that are designed to have different nucleotide sequences, may be from about 100 to about 1,000,000 (*e.g.,* from about 1,000 to about 1,000,000, from about 2,000 to about 200,000, from about 5,000 to about 50,000, etc.).

It has been shown that oligonucleotide pools above a certain complexity cannot be efficiently assembled in a single reaction compartment without problems. This often limits the number of different oligonucleotides that can be synthesized on a common carrier (such as a microarray or microchip) in parallel and can limit the usefulness of such high throughput oligonucleotide synthesis platforms.

One approach for addressing the complexity issue in nucleic acid assembly is to release and/or collect oligonucleotides that are components of a limited number of assembly products (*e.g.,* from about 2 to about 100, from about 3 to about 80, from about 4 to about 50, from about 2 to about 10, from about 2 to about 5, from about 1 to about 10, etc. assembly products) to keep oligonucleotide complexity minimal in assembly mixtures.

When a large number of oligonucleotides are produced, for example, on a common carrier, oligonucleotides may be released by methods such as through the cleavage of a linker. Such linkers may be cleaved, for example, photo-chemically with a laser, electrochemically, or micromechanically. A defined number of oligonucleotides may also be selectively released by amplification off the synthesis support, *e.g*., by using primers that specifically bind to primer binding sites included in each oligonucleotide. This, however, requires additional design of flanking primer binding sites for each oligonucleotide which limits synthesis capacity. In some instances, it may be desired to use a universal retrieval method that does not depend on a specific oligonucleotide sequence. This may be achieved *e.g*., by synthesizing oligonucleotides on removable solid supports on a common carrier. In a preferred setting, the solid supports are addressable such that each desired oligonucleotide can be selectively removed from the common carrier. For example, PCT Publication WO 2016/094512 describes a chip-based platform where oligonucleotides are synthesized on beads within wells. Beads carrying oligonucleotides comprising a desired sequence can be selectively released by using electrolysis and gas bubble displacement. Since multiple wells can be activated at a time, multiple beads carrying desired oligonucleotides can be retrieved simultaneously which allows efficient pooling of sub-sets of oligonucleotides required for the assembly of multiple nucleic acid fragments.

For example, depending on the number of nucleic acid fragments to be assembled, the number of beads that may be pooled may vary widely and include from about 10 to about 50, from about 50 to about 100, from about 100 to about 1,000, from about 50 to about 10,000, from about 100 to about 10,000, or from about 500 to about 10,000 individual beads.

Depending on the application and the number of nucleic acid fragments to be assembled, all of the supports of a carrier or only a subset may be retrieved and pooled. When only a subset of the supports are pooled or when the total number of supports is limited, the number of supports pooled may vary widely and include from about 10 to about 50, from about 50 to about 100, from about 100 to about 1000, from about 50 to about 10,000, from about 100 to about 10,000, or from about 500 to about 10,000 individual beads.

The released synthesis supports or beads may be deposited in any suitable container. One example of a container is the well of a microwell plate (*e.g.,* a well of a 1536 microwell plate). Examples for suitable collection devices are disclosed in PCT Publication WO 2016/094512.

In some instances, beads may be extracted and pooled using systems and devices as described, for example, in U.S. Patent Publication Nos. 2008/0281466 or 2008/0113361 or in U.S. Patent Nos. 6,887,431; 7,347,975 or 7,384,606. Alternative ways of retrieving loose supports may include picking supports with a micropipette, by suction or magnetic attraction.

In instances where magnetic beads are used as solid support, a bead picking instrument comprising, for example, a precision-controlled electro-micromagnet can be programmed and activated to extract and pool individual magnetic beads harboring synthesized nucleic acid molecules. Automation suitable use includes a precision-controlled electro-micromagnet that picks up the first bead and deposits it into a pooling well (*i.e.,* a well which contains multiple beads for collection of nucleic acid molecules sought to be used in combination). Alternatively, a micromagnet can be used which picks up the first bead and then moves in the X-Y direction to the next position, lowers down in the Z direction to pick up the second bead, back up in the Z direction to get out of the magnetic field range, moves to the third well in the X-Y direction, etc. Thus, the magnet is left "on" and the set of beads (*e.g.,* from about two to about fifty, from about ten to about fifty, from about two to about one hundred, from about ten to about one hundred, from about twenty to about eighty, etc.) is picked up and carried as a string of beads.

As a set of beads is collected, this set is then deposited into a pooling vessel. Of course, multiple sets of beads may be collected and deposited in a single pooling vessel.

In certain examples, all supports contained on a common carrier may be retrieved and pooled into a single vessel or distributed into two or more vessels. This may be achieved by selective retrieval of support-attached oligonucleotides in a step-wise manner in a predefined or random order.

In some examples, a picking or extraction device may be programmed to target only a portion of the wells of a specific array to extract and pool a predefined selection of bead- attached nucleic acid molecules. Further, the device can be programmed to extract and pool beads from the wells of two or more different plates or arrays. The picking may combine full extraction of all beads of a first carrier with selective extraction of a portion of beads obtained from a second carrier. The first and the second carrier may vary in size and dimension.

Each bead extracted by the device can then be transferred to a pooling station by moveable means of the picking device. In one embodiment the pooling station may contain a chamber with a microwell plate. In one embodiment the microwell plate may be a 1536 microwell plate. However, microwell plates or other vessels (such as columns) of other sizes and dimensions are known in the art and can be used in the current pooling methods. Defined fractions of nucleic acid molecules can be pooled in individual wells of a microwell plate wherein one pooled fraction contains all nucleic acid molecules required to assemble at least a defined fragment of a full-length construct. In one example, an individual oligonucleotide pool may contain all oligonucleotides required to assemble a predefined number of nucleic acid fragments or a full-length construct. Different oligonucleotide pools allocated to each well can be further identified using a machine readable identifier disposed on the microwell plates.

Electrostatic forces may also be used to remove beads and other supports from synthesis platforms. For example, oligonucleotide synthesis substrates (beads in this instance) may have an electrostatic charge and may be separated from association with a surface or well using an opposite charge. For example, if one or more beads have a positive charge then an electrode present underneath may be used to generate a positive charge to repel the bead and force it from the well. Magnetic charges can also be used to achieve the same purpose. Further, charges may be used to selectively remove a subset of synthesis substrates from a synthesis platform. In other instances, acoustic systems or optomechanical retrieval systems as described *e.g.* in Lee et al. (Nature Communications Vol. 6, Article number: 6073 (2015)) may be used for selective retrieval of individual supports or nucleic acid molecules.

Once a subset of oligonucleotides is released from a synthesis platform or carrier, the released oligonucleotides may be collected, optionally released from a support (*e.g.,* in instances where oligonucleotides are attached to beads) and subjected to one or more assembly reactions to generate a single assembly product or a known number of assembly products. Oligonucleotides may then be released from additional regions of the same carrier or from another carrier for assembly of additional nucleic acid molecules. For example, multiple sets of oligonucleotides corresponding to multiple fragments may be released into a single compartment or vessel (*e.g.,* a micrometer sized well). In some instances, the release and assembly of oligonucleotides may occur on different locations of the same carrier or device. For example, two or more subsets of oligonucleotides synthesized at individual areas of a common carrier may be released (selectively or collectively as described elsewhere herein) and translocated to another area of the same carrier or device for multiplex assembly in one or more reaction compartments.

For example, in a first step, all supports carrying oligonucleotides that belong to a first assembly product may be simultaneously released and pooled into a first compartment (such as a well of a multiwell plate). In a second step, all supports carrying oligonucleotides that belong to a second assembly product, may be simultaneously released and pooled into a second compartment.

The efficiency of this process may be improved in a number of ways that begin with the generation of oligonucleotide pools for the formation of more than one assembly product in a single reaction compartment (*e.g.,* a vessel, a tube, a column, a chamber, a well, a droplet, a cell etc.). For example, the oligonucleotide pool may contain oligonucleotides for the formation of multiple nucleic acid fragments. One way of doing this is illustrated in FIG. 3.

The left side of FIG. 3, Tube A, shows a schematic representation of a workflow for the release of oligonucleotides from beads for the formation of a pool, where the oligonucleotides are components of a single nucleic acid fragment, as well as the generation of the single nucleic acid fragment by PCR. The right side of FIG. 3, Tube B, shows a schematic representation of a workflow for the release of oligonucleotides from beads for the formation of a pool, where the oligonucleotides are components of four fragments, as well as the generation of the four fragments by PCR. This allows for the generation of four nucleic acid molecules (having different sequences) in a single compartment and through the same PCR cycling processes. Such processes allow for the collection of oligonucleotides that are components of more than one nucleic acid molecule. This improves the efficiency of nucleic acid molecule assembly in that more than one nucleic acid molecule may be produced without sorting of oligonucleotide components.

In some instances, the number of nucleic acid molecules represented by oligonucleotides in a pool may be so large that assembly of all of the nucleic acid molecules may not occur efficiently. In certain examples it may be desired to reduce the complexity of an oligonucleotide pool to a level that allows for simultaneous assembly of multiple fragments in a single reaction but is sufficiently low to guarantee efficient assembly without cross-hybridization events between oligonucleotides belonging to different fragments. For example, instead of successively releasing oligonucleotide pools for the assembly of 100-200 fragments, 10 to 100 pools each comprising the oligonucleotides for assembly of 3 to 10 fragments could be generated simultaneously.

To allow for pooling of equimolar amounts of oligonucleotides from different samples or supports, the initial concentration of oligonucleotides in each sample or on each support should be equal or similar. However, there may be instances where a higher amount of a first type of oligonucleotide is required and a lower amount of a second type of oligonucleotide. For example, where the first type of oligonucleotide represents a terminal region of a nucleic acid molecule to be assembled and the second type of oligonucleotide represents a central region of the nucleic acid molecule to be assembled, an assembly strategy may require a higher amount of the terminal oligonucleotide to be efficient. In such case, the first type of oligonucleotide may be provided at a higher concentration in the initial sample than the second type of oligonucleotide such that the total amount of pooled oligonucleotides reflects the difference in initial concentration between the first and second types of oligonucleotide. This can be achieved for example by pooling different amounts of supports (beads, particles) carrying a specific oligonucleotide species. In addition, the amount of oligonucleotide synthesized on each support can be quantified using fluorescent staining as described *e.g.* in PCT publication WO 2017/100283.

In some instances, each type of oligonucleotide having a desired sequence may be added to an assembly reaction at an amount of between about 10 fmol and about 10 pmol.

In many instances, the use of 3-dimensional (optionally porous) supports such as porous beads will produce sufficient amounts of oligonucleotides for fragment assembly such that no amplification of individual or pooled oligonucleotides prior to the assembly step is needed. For example, a single bead of about 20 - 50 µm in size may carry between about 10 and about 500 fmol of oligonucleotide. In some examples, beads may have a size of between about 10 µm and about 100 µm, a surface area within a range of between about 100 and 500 m²/g, and/or a porosity within a range of about 60% to about 80%. Exemplary beads that can be used in methods disclosed herein are described in PCT Publication WO 2016/094512. If higher amounts of oligonucleotides are needed for assembly of a desired nucleic acid fragment, a plurality of beads carrying the same type of oligonucleotide (*e.g*. 2, 4, 6 8 10, 20, 30 or 50 beads or more) may be pooled together. This allows for specific pooling of desired amounts of each individual oligonucleotide, in particular where different amounts of different types of oligonucleotides are needed for efficient fragment assembly. In one example, 10 beads - each bead loaded with 100 fmol of oligonucleotide - may be pooled to provide 1 pmol of a specific type of oligonucleotide for fragment assembly.

### Grouping of Fragments for Multiplex Assembly

When pooled oligonucleotides are components of multiple fragments, the pool may be split to produce either individual fragments or a group of fragments. When multiple fragments are produced in an oligonucleotide pool, individual fragments may be separated after a first assembly reaction by splitting up the volume and adding different primers suitable for amplification of one or more desired fragment. By such means, anywhere from one to twenty (e.g., from about two to about twenty, from about three to about twenty, from about four to about twenty, from about five to about twenty, from about two to about fifteen, from about two to about ten, from about two to about five, etc.) assembled nucleic acid molecules may be obtained from a single assembly mixture.

In instances where more than one nucleic acid fragment (for example three or more fragments) are assembled in a single reaction (such as, *e.g.,* from about 2 to about 20 or from about 3 to about 10), care needs to be taken that overlapping forward and reverse oligonucleotides belonging to the same fragment will efficiently anneal to each (without cross-hybridization with forward and reverse oligonucleotides belonging to another fragment) and that the assembly reaction of all fragments is unbiased (*i.e.,* the different fragments are produced in sufficient amounts at equal or comparable efficiency).

It has been found that for simultaneous assembly of multiple fragments in a single reaction (*i.e.,* a single "assembly pool") to occur at low bias, the fragments represented by a pooled oligonucleotide mixture are preferably within a predefined length range. It has been observed that multiplex assembly reactions (using oligonucleotides for two or more, preferably three or more fragments pooled together in the same assembly pool) may be increasingly biased if the fragments assembled in the same PCR reaction mixture exhibit substantial length differences. For purpose of illustration only, where three fragments having lengths of 287 bp, 243 bp or 216 bp, respectively, were PCR-assembled in the same reaction, the assembly of the longer 287-bp fragment was disadvantaged and therefore less efficient than the assembly of the two shorter 243-bp and 216-bp fragments. As a result, a smaller yield of the 287-bp fragment was obtained which may negatively affect all down-stream assembly or processing steps that require equal or similar amounts of multiplex fragments. In another example, the production of a 284-bp fragment showed to be less efficient when PCR-assembled together with a 256-bp and a 223-bp fragment. Of course, different results could be obtained under different experimental conditions.

Thus, in some instances, fragments (or the entirety of oligonucleotides from which they are assembled) in a given assembly pool may be selected to not deviate by more than 5, more than 10, more than 20, more than 30, more than 40, more than 50 or more than 60 base pairs (or bases) in length. Alternatively, fragments within a given assembly pool may be designed to not deviate by more than 2%, more than 5%, more than 10%, more than 15% or more than 20% in length. In an example, the length of all fragments (or the entirety of oligonucleotides from which they are assembled) in a given assembly pool may be within a range or "window" of about 40 base pairs. For purpose of illustration only a first fragment assembly pool may comprise all oligonucleotides for the assembly of two or more fragments having a length of between 240 and 280 bp, whereas a second assembly pool may comprise all oligonucleotides for the assembly of two or more fragments having a length of between 281 and 320 bp etc. Acceptable length ranges for a given fragment assembly pool may be determined based on the overall length distribution of all required fragments and/or the number of required assembly reactions and in view of other factors such as the particular reactions conditions.

The inventors have further found that for simultaneous assembly of multiple fragments in a single reaction or assembly pool to occur at low bias, the GC content of the fragments should be taken into account. The "GC content" (or guanine-cytosine content) as used herein defines the percentage of nitrogenous bases in a nucleic acid molecule that are either guanine ("G") or cytosine (C"). Whereas AT (adenine-thymine) and AU (adenine-uracil) pairs are bound by two hydrogen bonds, the GC pair is bound by three hydrogen bonds. Thus, nucleic acid molecules with a higher GC content exhibit a higher thermostability as compared to low GC content molecules.

In particular it has been observed that under certain conditions fragments with lower GC content may assemble less efficiently under certain conditions than those fragments in the same pool having a higher GC content. For purpose of illustration only, where three fragments each having a length of 304 bp and having GC contents of 57%, 45% and 56%, respectively, were PCR-assembled together in the same reaction, the assembly of the 45% GC fragment was disadvantaged and therefore less efficient than the assembly of the two fragments with higher GC content. As a result, a smaller yield of the 45% GC fragment was obtained which may negatively affect all down-stream assembly or processing steps that require equal or similar amounts of fragments. In another example, the production of a 40% GC fragment showed to be less efficient when PCR-assembled together with fragments having 53% and 48% GC content, respectively.

Thus, in some instances, fragments (or the entirety of oligonucleotides from which they are assembled) in a given assembly pool may be designed to not deviate by more than 1%, more than 2%, more than 3%, more than 4%, more than 5%, more than 10% or more than 20% in GC content.

In an example, 240 fragments for which the required oligonucleotides are produced may be grouped by GC content into 5% windows with a first fragment assembly pool comprising those fragments with a 41-45% GC content, a second fragment assembly pool comprising those fragments with a 46-50% GC content, a third fragment assembly pool comprising those fragments with a 51-55% GC content, etc.

In some instances, assembly pools may be grouped or sorted according to both, the length of the nucleic acid fragments as well as their GC content. Thus, in some instances, fragments in a given assembly pool may not deviate by more than 1%, more than 2%, more than 3%, more than 4%, more than 5%, more than 10% or more than 20% in GC content and at the same time may not deviate by more than 5, more than 10, more than 20, more than 30, more than 40, more than 50 or more than 60 base pairs (or bases) in length (or alternatively not deviate by more than 2%, more than 5%, more than 10%, more than 15% or more than 20% in length).

For purpose of illustration, 240 fragments for which the required oligonucleotides are produced may first be grouped by GC content into 5% windows (with a first fragment assembly pool comprising those fragments with a 41-45% GC content, a second fragment assembly pool comprising those fragments with a 46-50% GC content, a third fragment assembly pool comprising those fragments with a 51-55% GC content, etc.). The fragments may then be further sorted by length within a given GC window, (*e.g.,* grouping fragments within the 41-45% GC window by length such that those fragments having similar lengths can be combined in a single multiplex reaction) to obtain assembly pools defined according to the resulting order. A two-step sorting by GC content and length ensures that the GC content within a given assembly pool would always remain within a predefined window (such as, *e.g.,* a 5% window) and that in the vast majority of cases the fragments within a given assembly pool are in a predefined length range (such as, *e.g.,* a 10-bp length window). In some instances, the pools may first be sorted according to GC content and subsequently sorted by length, whereas in other instances the pools may first be sorted by length and subsequently by GC content.

Thus, in a first example, a method for multiplex nucleic acid assembly of a desired nucleic acid molecule may comprise the following steps: (i) dividing in silico, the desired nucleic acid molecule into a plurality of fragments, (ii) sorting in silico the fragments into two or more assembly pools according to fragment length, (iii) obtaining for each fragment a set of oligonucleotides, wherein each set of oligonucleotides comprises a plurality of forward and a plurality of reverse oligonucleotides having overlapping regions to allow hybridization in a desired order, (iv) pooling the sets of oligonucleotides for the two or more, preferably three or more fragments belonging to the same assembly pool as determined in (ii), and (v) assembling the pooled sets of oligonucleotides into two or more fragments.

In a second example, a method for multiplex nucleic acid assembly of a desired nucleic acid molecule may comprise the following steps: (i) dividing in silico, the desired nucleic acid molecule into a plurality of fragments, (ii) sorting in silico the fragments into two or more assembly pools according to fragment GC content, (iii) obtaining for each fragment a set of oligonucleotides, wherein each set of oligonucleotides comprises a plurality of forward and a plurality of reverse oligonucleotides having overlapping regions to allow hybridization in a desired order, (iv) pooling the sets of oligonucleotides for the two or more fragments belonging to the same assembly pool as determined in (ii), and (v) assembling the pooled sets of oligonucleotides into two or more fragments.

In a third example a method for multiplex nucleic acid assembly of a desired nucleic acid molecule may comprise the following steps: (i) dividing in silico, the desired nucleic acid molecule into a plurality of fragments, (ii) a) sorting in silico the fragments into two or more assembly pools according to fragment length and b) further sorting in silico the fragments into two or more assembly pools according to fragment GC content, (iii) obtaining for each fragment a set of oligonucleotides, wherein each set of oligonucleotides comprises a plurality of forward and a plurality of reverse oligonucleotides having overlapping regions to allow hybridization in a desired order, (iv) pooling the sets of oligonucleotides for the two or more fragments belonging to the same assembly pool as determined in (ii) a) and b), and (v) assembling the pooled sets of oligonucleotides into two or more fragments. In a fourth example, the in silico sorting step (ii) may comprise a) sorting the fragments into one or more assembly pools according to fragment GC content and b) further sorting the fragments into two or more assembly pools according to fragment length.

In one example a method for multiplex nucleic acid assembly of a desired nucleic acid molecule may comprise the following steps: (i) dividing in silico, the desired nucleic acid molecule into a plurality of fragments, (ii) a) sorting in silico the fragments into one or more assembly pools according to fragment GC content, wherein all fragments in a given assembly pool do not deviate by more than 10%, preferably not more than 5% in GC content, and b) further sorting in silico the fragments into one or more assembly pools according to fragment length, wherein all fragments in a given assembly pool do not deviate by more than 60 bp, preferably not more than 30 bp in length (or alternatively, wherein all fragments in a given assembly pool do not deviate by more than 2%, more than 5%, more than 10%, more than 15% or more than 20% in length), (iii) obtaining for each fragment a set of oligonucleotides, wherein each set of oligonucleotides comprises a plurality of forward and a plurality of reverse oligonucleotides having overlapping regions to allow hybridization in a desired order, (iv) pooling the sets of oligonucleotides for the two or more fragments belonging to the same assembly pool as determined in (ii) a) and b), and (v) assembling the pooled sets of oligonucleotides into two or more fragments. These methods allow for sorting of multiple fragments into two or more available pools or reaction compartments and simultaneous assembly of at least two, preferably at least three or more fragments at high efficiency in a single reaction compartment.

Thus, in some instances oligonucleotides or sets of oligonucleotides corresponding to fragments to be co-assembled may first be grouped according to their GC content and then scanned in respect of classes of GC content (*e.g*. from low to high GC content). In addition, fragments may then be sorted or grouped according to their length in those GC content classes and assigned to the next available pool or reaction compartment.

In addition, fragments may be sorted or grouped based on specific motifs or sequence regions, such as *e.g*. homopolymers (stretches of identical bases) or other complex DNA regions as described elsewhere herein. For example fragments having stretches of more than 5, more than 6, more than 8, more than 10 G and/ or C and/or A and/or T bases or repetitive sequences of a certain length may be assembled under specific conditions (*e.g*. specific PCR conditions) or may be excluded from multiple fragment pools or assigned to one or more separate pools. Thus, although the present disclosure generally provides methods for pooling of multiple fragments and multiplex assembly, it is understood that such methods do not exclude that individual fragments that may be difficult to multiplex are processed and/or assembled in isolated pools or separate compartments.

Whereas it has been demonstrated that less variation of fragment lengths or GC content in a single assembly pool (or reaction compartment) can increase assembly efficiency, it is less desirable to combine fragments with high "sequence similarity" into the same assembly pool (although they may be assigned to the same pool based on length and/or GC content). Co-assembly of fragments with high sequence similarity may result in cross-hybridization of the assembly oligonucleotides or intermediate assembly products belonging to different fragments. It is therefore another aim to take into account sequence similarity when assigning multiple fragments (or the respective assembly oligonucleotides) to a limited number of available reaction compartments.

The term "sequence similarity", as used herein, refers to the overall degree of sequence identity between two non-identical nucleic acid sequences. Two sequences may share identical internal or terminal regions or both. It has been shown that co-assembly of two fragments in the same reaction compartment that share identical terminal regions of a minimum length may result in problems if the identical regions are positioned at the 3'-ends of the respective assembly oligonucleotides that end up in the same assembly pool (for example, where assembly oligonucleotides belonging to different fragments share at least 5 base pairs or share between 10 and 15 base pairs at their 3'-ends). Thus, fragments having identical terminal regions may be defined as similar and may therefore be assigned to different assembly pools.

However, sequence similarity is not restricted to terminal fragment regions and may need to be considered taking full-length sequence properties of co-assembled fragments into account. This may become relevant for example, if multiple variants of a defined nucleic acid sequence are to be assembled simultaneously or in parallel to generate a library. For example, two nucleic acids sequences may share one or more regions of sequence identity, whereas other portions of the sequences may be different. Examples are libraries or variants of antibodies or antibody fragments (*e.g*. antibody sequences encoding an identical conserved region but comprising different variable regions), enzymes (*e.g.* variation of a domain, a catalytic residue or binding region etc.), receptors or ligands (*e.g*. variation of a domain, a binding site), RNAi molecules, aptamers, peptides, etc. Similarity of two or more sequences may also arise where variation is based on differences in codon usage (*e.g*. two identical amino acid sequences with deviating nucleotide sequences encoding them) as described elsewhere herein.

In such instance sequence similarity of two nucleic acid sequences (or fragments) can be determined by comparing the nucleotide positions in a pairwise alignment of both sequences over a predefined window of comparison (*e.g*. the length of a fragment, the assembly region of a fragment etc.). In some instances, two sequences (or fragments) may be defined as similar, if they share a minimum number (*e.g.* 2, 3, 4, 5, or more) of identical sequence regions of a minimum length referred to as "matching blocks" (wherein a matching block may have a minimum length of about 15, 16, 17, 18, 19, 20 bases in the alignment etc.)) that are interspersed with or flanked by one or more regions that are not a matching block and may be referred to herein as "irrelevant block" (wherein an irrelevant block can have a length of 1, 2, 3, 4, 5, 10 etc. bases in the alignment). In some instances, two sequences (or fragments) may be defined as non-similar, if they differ only in a few positions (*e.g.,* less than 10, less than 8, less than 5, less than 3 etc.) that are close to each other (*e.g.,* within a window of 2, 3, 4, 5, 6, 8, or 10 nucleotide positions). To determine sequence similarity, a pairwise alignment of two sequences may be generated and divided into matching blocks and irrelevant blocks as described in more detail below. The two sequences can be defined as similar, if the number of irrelevant blocks in the pairwise alignment exceeds a predefined threshold value (*e.g.* if the number of irrelevant blocks is at least 3). Alternatively, the two sequences can be defined as non-similar if the number of irrelevant blocks does not exceed the predefined threshold value (*e.g.* if the number of irrelevant blocks is smaller than 6, smaller than 5, smaller than 4 or smaller than 3).

An assembly pool of oligonucleotides for unbiased fragment assembly may be further defined by a "similarity score", which is a measure of similarity between all oligonucleotides of one fragment and all oligonucleotides of a second fragment to be assembled in the same pool. The similarity score may be used to predict possible cross-hybridization events between fragments in an assembly pool. Furthermore, the similarity score may be used to exclude two or more nucleic acid fragments that are too similar or are represented by oligonucleotides that are too similar from being assigned to the same assembly pool.

Thus, the grouping of fragments may be modified to avoid that multiple fragments with similar or homologous sequences or sequence regions will be assigned to the same pool.

By considering further criteria for optimal grouping, extra constraints can be introduced. This allows more flexibility with regard to the number of oligonucleotide sets that can be pooled together and thus, the number of fragments that can be co-assembled in a single pool.

An exemplary method for assigning fragments to multiple pools that takes into account sequence similarity may be described using the definitions indicated in Table 1 and as further specified below. However, additional definitions or parameters may be used. For simplicity of description, exemplary values are provided for some of the definitions in Table 1:

| Table 1 | | |
|---|---|---|
| **Definition** | **description** | **Example** |
| Pₘₐₓ | Number of available pools | 80 |
| S | fragment sequences | 240 |
| Sₘₐₓ | number of fragments allowed per pool | 3 |
| Lₘᵢₙ | Minimum length of fragments; base pairs | 250 |
| Lₘₐₓ | Maximum length of fragments; base pairs | 500 |
| L_{diffmax} | Maximum length difference of two fragments assigned to the same pool; measured relatively (%) or absolutely; base pairs | 8% |
| GC_{diffmax} | Maximum difference in GC percentage of two fragments assigned to the same pool; measured absolutely | 5 |
| P | Number of calculated pools | |
| L_{RMBmin} | Minimum length of relevant matching block; base pairs | 15 |
| NABₘₐₓ | Maximum number of irrelevant blocks | 2 |

As input the following parameters are provided: Up to Sₘₐₓ * Pₘₐₓ fragment sequences over the alphabet {A, C, G, T} (the alphabet indicating options for available bases may be extended as necessary, *e.g.* to include additional bases such as uracil etc.). The total number of input fragment sequences for distribution over a given set of pools may exceed Pₘₐₓ. However, because the number of fragment sequences that can be selected for distribution over the given set of pools is limited by Pₘₐₓ, any number of fragment sequences exceeding Pₘₐₓ must be allocated to the next pool set. All fragment lengths are between Lₘᵢₙ and Lₘₐₓ base pairs. A suitable length range will be chosen by the skilled person based on assembly conditions and/or downstream processes. For example, Lₘₐₓ may be limited by the read length of a given sequencing platform. In some examples fragment lengths may be from 100 base pairs to 500 base pairs or from 200 base pairs to 700 base pairs etc.

Further, the fragments *(i.e.* the oligonucleotides that are components of the fragments) are to be distributed over at most Pₘₐₓ available pools or reaction compartments such that: (i) each pool contains at most Sₘₐₓ fragments, and (ii) fragment sequences in the same pool "harmonize". Two sequences "harmonize" if
a) their lengths differ by at most L_{diffmax} (which can be a predefined percentage or absolute value);
b) their GC contents *(i.e.,* percentage of bases that are G or C) differ by at most GC_{diffmax} percentage points; and
c) they are "non-similar" (*i.e*. they do not have many identical sequence regions), as further explained below.

Regarding feature a), if L_{diffmax} indicates a relative length difference between two sequences or fragments (*e.g.* fragment 1 of length 11 and fragment 2 of length l2), then an exemplary parameter value of 8 means that max(l1/l2, l2/l1) is less than or equal to 1.08 for those two sequences of lengths 11 and l2, *i.e.,* their lengths differ by at most 8 percent.

By taking into account sequence similarity of the fragments the inventors surprisingly found that: (1) fragments with very similar sequences can be assigned to the same pool or reaction compartment if their sequences differ only in a few positions that are close to each other; and (2) fragments that have a large number of identical sequence regions of a certain length that are interspersed with non-identical sequence regions should not be combined into the same pool.

The above rules were derived from observations that pools comprising fragments according to category (2) generate a higher percentage of misassembled fragments. Without being bound by theory, this effect may be based on cross-hybridization *(i.e.* non-specific hybridization) of pooled oligonucleotides which combine in an "exponential" number of ways during PCR assembly, while sequence similarities of category (1) lead to a smaller number of cross-hybridizations which do not result in a significant amount of mis-assemblies or are diluted out in subsequent processing steps. To implement these rules, a pooling algorithm is applied to prevent that fragments of category (2) will be pooled together. The inventors observed that by using the described pooling method, at least 95% of the fragments assembled simultaneously in Pₘₐₓ pools were correctly assembled (see Example 1).

Different algorithms may be used to determine sequence similarity. For example, the similarity may be measured by forming pairwise alignments between sequences and evaluating the form of the obtained alignment (see *e.g. "*Understanding Bioinformatics", 1st edition, Marketa J Zvelebil, Jeremy O. Baum; Garland Science 2007).

Methods of alignment of sequences for comparison are well known in the art. Optimal alignment of sequences for comparison can be conducted, *e.g.,* by the local homology algorithm of Smith and Waterman (1970) Adv. Appl. Math. 2:482c, by the homology alignment algorithm of Needleman and Wunsch (1970) 1 Mol. Biol. 48:443, by the search for similarity method of Pearson and Lipman (1988) Proc. Nat'l. Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, e.g., Ausubel et al., Current Protocols in Molecular Biology (1995 supplement)). An example of an algorithm that is suitable for determining percent sequence identity and sequence similarity are the BLAST and BLAST 2.0 algorithms, which are described in Altschul et al. (1977) Nuc. Acids Res. 25:3389-3402, and Altschul et al. (1990) I Mol. Biol. 215:403-410, respectively. Software for performing BLAST analyses is publicly available through the National Center for Biotechnology Information.

Thus, to determine sequence similarity, nucleic acid fragments to be combined into a plurality of pools may be compared based on pairwise affine sequence alignments. Such an alignment may contain one or more blocks of matching bases referred to as "matching blocks" *(i.e.* a stretch of identical bases in both aligned sequences). A matching block is chosen to include a maximum number of consecutive identical bases between two aligned fragment sequences (such that the block cannot be extended in either direction by additional matching bases). As used herein, a matching block is deemed "relevant" if it has a minimum predetermined length L_{RMBmin}. For example, L_{RMBmin} could be at least 10, at least 12, at least 15, at least 17, at least 20 or more bases. Relevant matching blocks may be interspersed with or flanked by one or more "irrelevant blocks", *i.e.* blocks in the aligned fragment sequences that do not have sequence identity or matching blocks that do not have a minimum predetermined length L_{RMBmin}. An irrelevant block may consist of one or more bases. Thus, any pairwise sequence alignment can be divided into relevant matching blocks and irrelevant blocks.

For purpose of illustration only, a pairwise alignment for exemplary sequence 1 (5'-ACGTGGAAGCAGAGATAGACAGATAGACAGATAA -3'; (SEQ ID NO:1)) and sequence 2 (5'- TTCAGCAGAGATAGACAGATAGACAGATATA -3'; (SEQ ID NO:2)) is provided with L_{RMBmin} = 17. Sequences 1 and 2 may be represented by the following pairwise alignment with matching bases indicated by a vertical line (alternatively, also an alignment with a reverse complement sequence could be generated):

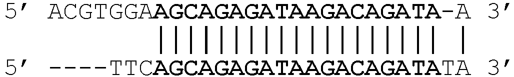

The above sequence alignment shows a total of one relevant matching block of length 19 (indicated in boldface) that is flanked by two irrelevant blocks.

Using the above approach, a similarity notion can be determined for two non-identical (fragment) sequences. The two sequences can be classified as "non-similar" if the number of irrelevant blocks is less than or equal to N_{ABmax}. Assuming that in the above example N_{ABmax} = 2, sequences 1 and 2 are deemed "non-similar" and thus fulfill harmonization requirement c). In some instances N_{ABmax} may be 2. In other instances, N_{ABmax} may be 3, 4 or 5. In general, N_{ABmax} may be determined *e.g*. based on data mining using multiple assembly production results.

Once the harmonization criteria a) - c) (L_{diffmax}, GC_{diffmax}, and non-similarity; N_{ABmax}) have been determined, the oligonucleotide sets for fragment assembly are assigned to the available number of pools Pₘₐₓ. This can be done in a number of ways, one of which is graph coloring using the common concept of "undirected graphs".

An undirected graph consists of a set of objects (called vertices or nodes) that are connected by edges. Each edge between two vertices or nodes represents a pairwise relation between those two objects, where the relation is symmetrical. Similarity is an example of such a symmetrical relation. (Undirected) graphs are a well-known concept in computer science, and the area provides a plethora of algorithms. A description of undirected graphs and some graph algorithms is provided *e.g.* in "Introduction to Algorithms", Third Edition, Cormen et al., The MIT Press 2009.

An exemplary graph coloring method for pooling sets of oligonucleotides, wherein each set of oligonucleotides represents a fragment may comprise the following steps:
(i) Define an undirected graph with nodes, wherein each node represents a fragment S and wherein an edge between two nodes is present if the two respective fragments do not harmonize;
(ii) Color the nodes of the undirected graph with as few colors as possible, wherein each color represents a pool and wherein each color is used at most Sₘₐₓ times. (Preferably, there should be no edge between the nodes in the undirected graph representing pooled fragments).
(iii) Determine the amount of used colors P; and
(iv) Assign the fragments to Pₘₐₓ pools.

Step (iv) may be carried out by applying one or more of the following rules:
(1) if P = Pₘₐₓ then for all colors i=1...P, assign all fragments with color i to pool i";
(2) if P < Pₘₐₓ, either proceed as in (1) (leaving one or more pools empty) or "redistribute" fragments from pools 1 to P into the empty pools P+1 to Pₘₐₓ. This can be done in a number of ways, for example by using random decisions. Alternatively, extra criteria may be defined for identifying fragments from the pools 1 to P that would prefer to be in a separate pool *(i.e.* determining an "isolation score"), and then redistribute those fragments until P = Pₘₐₓ or until respective fragments are isolated. Such criteria may include fragments with a homopolymer stretch exceeding a certain threshold length *(e.g,* a homopolymer stretch of length ≥ 8) or fragments having a "particular high GC" or a "particular low GC" *(e.g.* < 35% or > 60%).
(3) if P > Pₘₐₓ, relax the criteria for constructing the undirected graph, possibly generating a graph with less edges and possibly allowing a coloring with a number P_{new} < P, then proceed as in (1) or (2) with P_{new} replacing P. Relaxation means allowing larger length differences/or GC content differences. Alternatively or if step (3) does still not yield P ≤ Pₘₐₓ, redistribute the fragments that were assigned to pools > Pₘₐₓ, forcing them to be combined into pools 1 to P with S < Sₘₐₓ. This approach, however, is less preferred as it does no longer reflect the coloring principle, according to which there should be no edge between the nodes in the undirected graph representing pooled fragments. In case the latter approach would need to be chosen, the redistribution should at least take similar length or GC content (*e.g*. with GC_{diffmax} = 10%) into account.

An exemplary flowchart of the above pooling approach is shown in FIG. 6. In step 1. a set of input sequences S representing the desired nucleic acid fragments that are to be assembled is provided. Further, the maximum number of fragments Sₘₐₓ to be assembled in each reaction compartment or pool and the maximum number of available pools Pₘₐₓ are defined. These parameters may depend on various criteria including workflow capacity and automation level etc. In step 2. the "harmony criteria" for fragment pooling are defined as set out above. In many instances harmony criteria take at least into account fragment (non-) similarity, GC content and length. In the example shown in FIG. 6, GC_{diffmax} *(i.e.* the maximum difference in GC percentage of two fragments assigned to the same pool) is set at ≤ 5% and L_{diffmax} (maximum length difference of two fragments assigned to the same pool) is set to ≤ 25 bases (or base pairs). As discussed above, these parameters may also vary depending on the input sequences and may need to be determined separately for each assembly run. Based on the defined harmony criteria, a number of required pools P can be determined. If P = Pₘₐₓ *(i.e.* if the number of required pools is equal to the number of available pools), the fragments can be assigned to P pools using the harmony criteria determined in step 2. If P > Pₘₐₓ *(i.e.* if more pools would be required than pools are available) the harmony criteria could be relaxed. For example, GC_{diffmax} could be raised to ≤ 10% and L_{diffmax} could be set to ≤ 30 bp. If P = Pₘₐₓ, the fragments can be assigned to P pools using the relaxed harmony criteria determined in step 2.1. If P is still > Pₘₐₓ, some of the fragments could be redistributed individually (*e*.*g*. to pools having the closest GC_{diffmax}) until P = Pₘₐₓ. If using the harmony criteria of step 2. result in P < Pₘₐₓ *(i.e.* if less pools would be required than pools are available) the fragments can be assigned to P < Pₘₐₓ pools. Alternatively, to make use of all available pools, an "isolation score" could be determined in step 3. *(e.g.* based on sequences with a homopolymer stretch ≥ 8, or very high or low GC content) and fragments with a high "isolation score" would be redistributed or assigned to separate or "empty" pools until P = Pₘₐₓ.

Instead of using a coloring scheme as described above the skilled person may also select other algorithms to assign the oligonucleotides/fragments to Pₘₐₓ pools. The skilled person is well aware that the approach of using colorings can be reformulated in a number of ways, *e.g*., the color classes (fragments with the same color) can be seen as "independent sets" of size Sₘₐₓ or "cliques" in the "complement" of the undirected graph. The task may then be formulated as computing a set of ≤ Pₘₐₓ "independent sets" or "cliques" of size ≤ Sₘₐₓ.

In many instances a consecutive coloring approach may be used in step (ii). According to a consecutive approach (also known as a greedy approach), an uncolored node v is selected in a given iteration of step (ii) and the minimum color that is not yet exhausted (used less than Sₘₐₓ times) and has not been used for one of v's neighbors is assigned to it. As used herein, a neighbor of a given node is a node that is connected to it via an edge. In a consecutive iteration step, the next uncolored node may be selected using a "greedy" approach (*i.e*., choose the node of minimum / maximum degree) or completely randomly. Randomness may be exploited *e.g.* by performing a maximum number of iterations (*e.g.* Rₘₐₓ = 500) and choosing the best solution, preferably the one using the least number of pools. Although different algorithms may be applied, the above coloring method provides a reasonable approach also taking into account computation time which is critical in a high throughput setting.

FIG. 7 shows an exemplary coloring method that can be used for assigning fragments to multiple pools. For simplicity of illustration, 6 fragments *a, b, c, d, e* and *f* are to be distributed over 2 pools (Sₘₐₓ = 6; Pₘₐₓ = 2). Each of the 6 fragments is represented by an individual node (open circles) in the constructed undirected graph. An edge between two nodes indicates that respective sequences do not harmonize according to the criteria set out above and should therefore not be combined into the same pool. FIG. 7A shows an exemplary graph where *e.g.,* the edge between nodes *a* and c indicates that fragments *a* and *c* should not be assigned to the same pool. The coloring may start by choosing node *a* first, as it has the least number of neighbors, namely one. The first available color *1* is then assigned to node a. Assume the algorithm chooses node c next (*e.g*. using a random or greedy approach). The smallest number available for *c* is 2, as *1* has already been used for neighbor *a*. Thus, color 2 is assigned to node c (FIG. 7B). Next, the algorithm computes that colors {*1*,*2*} could be used at node *b,* color *1* at node *d,* colors {*1,2*} at node *f,* color *1* at node *e.* As shown in FIG. 7C, color *1* is assigned to nodes *e* and *d,* whereas color *2* is assigned to nodes c and *f,* respectively. The skilled person understands that different algorithms may be employed to achieve efficient coloring using the above principles.

In variations of the coloring method, less than Pₘₐₓ*Sₘₐₓ sequences for Pₘₐₓ pools may be provided, *i.e.,* there may be pools with less than the allowed number of Sₘₐₓ sequences assigned. In such case, the following observations may be taken into account: (i) sequences are more difficult to assemble if they have extremely low or high GC contents (*e.g*. a GC content close to or outside a range of about 35% to 60% GC); thus, these sequences may be "isolated" or combined with fewer fragments in the same pool; (ii) in general, fragments (sequences) prefer to be more isolated.

After the coloring has taken place, an optional additional step may comprise: determining if there are pools with S < Sₘₐₓ fragments and assigning "non-harmonizing" fragments to pools with S < Sₘₐₓ fragments. This redistribution could be achieved in a number of ways. For example, if there are empty pools, one can identify the sequences with very low GC content *(e.g.* a GC content below 35%), remove them from the pool they got assigned to in the previous coloring step and isolate them into one of the empty pools.

Alternatively, the graph coloring problem may be formulated as an "integer linear program" (ILP) and an ILP solver may be used for it. An ILP uses integer variables and linear constraints. As an example, one might use a variable *x*(*i,j*) for a node *i* and color *j* with the variable having value *1* indicating that node *i* gets color *j* and the variable having value *0* otherwise. For each edge between nodes *j* and *k* and color *m,* one would have constraints *x*(*j,m*) *+ x*(*k,m*) <= *1* meaning that color *m* cannot be used at node *i* and node *j* simultaneously. The other restrictions can be formulated accordingly.

Although the ILP approach may find the "optimum solution" for a given problem, it will in most instances be less efficient than the above described consecutive approach due to the high number of variables that have to be used. Based on the graph coloring problem, other algorithms known in graph theory may be used, as long as the colors will only be used a limited number of times (*i.e.* Pₘₐₓ times).

Another approach that may be used to define the similarity of sequences may be based on the mis-hybridization of oligonucleotides as disclosed elsewhere herein. However, the above approach has the advantage that it uses criteria that only depend on the (predefined) fragment sequences and do not need to take into account additional parameters that may vary depending on downstream processes.

Applying the above rules for grouping and assigning multiple fragments to a predefined number of reaction compartments increases throughput and parallelization of assembly reactions and decreases cost by avoiding a high number of single assembly reactions which would significantly decrease the overall capacity of array- or chip-based synthesis platforms.

### Multiplex Assembly

Following grouping and assignment of nucleic acid fragments to available assembly pools, the assembly oligonucleotides that are components of the grouped nucleic acid fragments can be selectively retrieved and pooled into the assigned assembly pools or reaction compartments *e.g*. using methods and devices as set out above. The pooled sets of oligonucleotides are then simultaneously assembled in a series of multiplex assembly reactions.

In some instances two or more, preferably three or more nucleic acid fragments (*e.g.* 3, 4, 5, 6, 7, 8, 9, 10, 12, 15, 20, 25, 30 or 50) are assembled simultaneously in the same reaction compartment (*e.g*. in wells of a microwell plate). In some instances a method comprises multiplex assembly of between about 3 and about 30 nucleic acid fragments in each of a plurality of between 10 and 100 reaction compartments. In some instances, three or more fragments may be assembled in a reaction volume of between about 0.01 µl and about 10 µl, between about 0.1 µl and about 1,000 µl or between about 0.1 µl and about 300 µl or between about 0.5 µl and about 50 µl, such as for example a 1-µl reaction volume.

In some instances, a prokaryotic or eukaryotic cell *(e.g.* a yeast cell, a mammalian cell, an *E. coli* cell or an engineered cell) may be used as compartment for assembly. In such instances, the two or more *(e.g.* three or more) nucleic acid fragments may be assembled in a reaction volume of between about 0.1 pl and about 10 pl.

One exemplary method for multiplex assembly of nucleic acid fragments is depicted in FIG. 2 and involves starting with oligonucleotides that will generally contain sequences that are overlapping at their termini which are "stitched" together via these complementary sequence regions or overlaps using PCR. A set of overlapping assembly oligonucleotides that are components of a defined nucleic acid fragment typically comprise a set of "forward oligonucleotides" that comprise at least a portion of the forward or sense strand of a double-stranded assembly product and a set of "reverse oligonucleotides" that comprise at least a portion of the reverse or antisense strand of the double-stranded assembly product. Each forward oligonucleotide is at least partially complementary to at least one reverse oligonucleotide such that hybridization of the forward and reverse oligonucleotides in a predetermined order can occur. The forward and reverse oligonucleotides may be designed to hybridize in a "nicked", "single gapped" or "double gapped" conformation. A "nicked" conformation is achieved if forward and reverse oligonucleotides are designed to cover the full-lengths of both the forward and reverse strands (*i.e.* they will hybridize only leaving nicks between adjacent oligonucleotides in both strands). A "single-gapped" conformation is achieved if either the forward or the reverse oligonucleotides are designed to cover the full-length of one of both strands, whereas the other oligonucleotides (forward or reverse, respectively) only cover a portion (*i.e.,* less than the full-length) of the other strand, such that when the forward and reverse oligonucleotides are hybridized there will be nicks in one of the strands and single-stranded "gaps" of one or more nucleotides between adjacent oligonucleotides in the other strand. A "double-gapped" conformation is achieved if the forward and the reverse oligonucleotides are designed to cover only a portion (*i.e.,* less than the full-length) of the forward and reverse strands, such that when the forward and reverse oligonucleotides are hybridized there will be single-stranded gaps of one or more nucleotides between adjacent oligonucleotides of both strands. FIGs. 2 and 3 represent examples of a double-gapped conformation, whereas oligonucleotides in FIG. 4 are hybridized in a single-gapped conformation. Gaps are subsequently filled up by PCR-mediated elongation. A "single gapped" conformation is achieved, for example, when the oligonucleotides that are components of either the forward or reverse strand are shorter than the oligonucleotides belonging to the other strand. For example, in a single gapped scenario, the forward oligonucleotides may have an average length of between about 25 to about 100 or between about 30 to about 60 or between about 36 to about 80 or between about 36 to about 120 nucleotides, whereas the reverse oligonucleotides may have an average length of between about 25 to about 100 or between about 30 to about 50 or between about 36 to about 44 nucleotides. In a double gapped conformation the oligonucleotides belonging to the forward or reverse strands may comprise oligonucleotides of similar or equal lengths which may in certain instances be shorter than the oligonucleotides used in a nicked conformation. Double-gapped conformation results in a minimal amount of oligonucleotides required to cover a desired nucleic acid sequence to be assembled, less phosphoramidite couplings limiting the amount of errors introduced via inefficient coupling events, and less hybridization events required for successful nucleic acid assembly, possibly facilitating oligonucleotide design considerations. Thus, in some examples, a single or double gapped conformation may be preferred over a nicked conformation, *e.g*., where the production of shorter oligonucleotides for one or both strands reduces oligonucleotide synthesis time and reagent consumption. In other examples a nicked conformation may be preferred, *e.g*., where PCR amplification of two sets of complementary oligonucleotides may fail due to certain sequence properties (such as, *e.g.,* GC-rich and/or highly repetitive sequence stretches). Under such circumstances, oligonucleotides provided in a nicked conformation can be used in a LCR reaction (ligation chain reaction) to directly ligate adjacent oligonucleotides prior to PCR extension as described, *e.g*., in U.S. Patent No. 6,472,184. This process is based on a combination of overlap extension and ligation-based assembly wherein only the oligonucleotides forming the first strand are provided in a "linkable" *(i.e.,* phosphorylated) form whereas the overlapping oligonucleotides forming part of the second strand are used as primers for PCR extension. In certain examples a LCR reaction may be desirable in particular where nucleic acid molecules with highly repetitive regions (*e.g.,* containing GC stretches) are to be assembled.

In some examples, the overlaps between forward and reverse oligonucleotides are approximately 10 base pairs; in other examples, the overlaps may be 15, 25, 30, 50, 60, 70, 80 or 100 base pairs, etc. *(e.g.,* from about 10 to about 120, from about 15 to about 120, from about 20 to about 120, from about 25 to about 120, from about 30 to about 120, from about 40 to about 120, from about 10 to about 40, from about 15 to about 50, from about 40 to about 80, from about 60 to about 90, from about 20 to about 50, from about 15 to about 35, etc. base pairs). In some examples, the overlaps may be at least about 15 bases or at least about 18 bases in length depending on the overall length of the nucleic acid molecules to be assembled. In other examples the overlaps may be between about 18 to about 25 bases or between about 20 to about 30 bases in length.

Melting temperatures of oligonucleotides typically depend on length and sequence requirements, but may typically be within a range of about 45 to about 72°C, such as about 55°C or as about 50°C. The complementary regions within oligonucleotides used for joining may have a minimum length to allow for efficient base pairing of overlapping oligonucleotides. For example, the complementary overlaps used for joining forward and reverse oligonucleotides of a nucleic acid fragment will generally be from about 8 to about 30, from about 10 to about 30, from about 15 to about 20 nucleotides in length.

In one example the assembly oligonucleotides are designed to foster the preferential assembly of oligonucleotides whose sequence is exactly correct and prevent or avoid the hybridization of correct assembly oligonucleotides with oligonucleotides whose sequence is incorrect (*e.g.* due to errors introduced during oligonucleotide synthesis). Any composition(s) and/or method(s) that is/are operable to produce hybridization of overlaps in such a way that hybridization of overlap segments having the correct sequence is favored over other possible hybridizations can be used in accordance with the present disclosure. Without limiting the generality of the foregoing, in various examples, hybridization of assembly oligonucleotides is performed at a temperature at which hybridization of pairs of overlaps that are exactly reverse complementary are able to occur more readily than hybridizations involving incorrect sequences. The ability to select against incorrect hybridizations can be improved to the extent that correct hybridizations of the overlaps of the assembly oligonucleotides anneal at a temperature distinctly higher than the temperature at which hybridizations with incorrect oligonucleotides anneal, so that it is possible to segregate correctly hybridized duplexes from incorrect hybridizations by setting the temperature used for hybridization during assembly of assembly oligonucleotides at a temperature that is low enough to allow the correct hybridizations to form but high enough to prevent incorrect hybridizations from forming.

It is well known to a skilled person that as nucleic acids are heated under appropriate conditions, melting takes place over a range of temperatures, so that at the nominal melting temperature of a given sequence, half of the duplexes of that sequence with its reverse complement will be melted and half will be annealed. As the temperature is raised or lowered above and below the nominal melting temperature, varying proportions of the hybridized duplexes will remain annealed. This phenomenon is described by the melting relation $θ = \frac{1}{{e}^{\frac{Δ H}{RT} + \frac{Δ S + Rln \left(\frac{C}{2}\right)}{R}}}$ where *θ* is the percent of duplexes remaining annealed at a temperature of T in degrees Kelvin, *C* is the concentration of duplexes present if all were annealed, *ΔH* is the change of enthalpy and *ΔS* the change in entropy, respectively, ongoing from the annealed to the melted state, and *R* is the ideal gas constant. At a temperature within a few degrees Celsius of the melting temperature in either direction, not all of the duplexes will be melted or annealed; rather, the proportion of the duplexes given by *θ* in the foregoing equation will remain annealed.

The melting temperature of a given hybridized duplex will depend upon the length, the sequence, and the nature and position of any differences between the sequence of the first strand and the reverse complement of the second strand. The melting temperature for a given sequence with respect to its exact reverse complement may be estimated according to the formula: ${T}_{m} = 64.9 + \frac{41 \left({N}_{G}+{N}_{C}-16.4\right)}{L}$ where *N_{G}* is the number of *G* residues in the overlaps, *N_{C}* is the number of *C* residues in the overlaps, and *L* is the number of all residues in the overlaps.

To the extent that the overlaps of the assembly oligonucleotides in an assembly pool differ in sequence in ways that affect their respective melting temperatures and melting curves, the result will be that, at a given temperature at which hybridization of assembly oligonucleotides is carried out, the various pairs of reverse complementary overlap segments will be melted in differing proportions. An assembly oligonucleotide pool may comprise, in addition to the correctly synthesized assembly oligonucleotides, a distribution of other oligonucleotides having sequences that differ from the correct sequences in length and/or composition. The extent to which these will introduce error by hybridizing with correct assembly oligonucleotides at the temperature used for assembly of the assembly oligonucleotides will depend upon the specific incorrect sequences present and the concentrations at which they are present. In principle, if the exact sequences and concentrations of all oligonucleotides present were known, it would be possible to make a close estimate of the relative quantities of various correct and incorrect hybridizations that would form at a given temperature, and thereby select a temperature that optimally excludes the unwanted hybridizations from forming. As a practical matter, the sequences and concentrations of the incorrect oligonucleotides will not usually be known or determinable, so it may be useful to base the temperature design of the overlap segments upon mismatch-exclusion temperature characteristics reflecting reasonable estimates or heuristics.

In various examples, the melting temperatures of all overlaps of all assembly oligonucleotides (1) present in an assembly pool, or (2) corresponding to the same nucleic acid fragment, or (3) corresponding to the two or more nucleic acid fragments in a single pool or reaction compartment are within a range not exceeding about 10, 9, 8, 7, 6, 5, 4, 3, 2, or 1 degree Celsius.

In other examples, the single-mismatch annealing temperature of an overlap is taken as conservatively representative of the population of incorrect sequences. Other factors being equal, sequences that differ from the exact reverse complement of an overlap segment at only a single position will, in general, hybridize with the overlap segment more readily and at a higher temperature than sequences that differ at more than one position. In various examples, the melting temperature of the overlap having the lowest melting temperature of all overlaps of all assembly oligonucleotides (1) present in the assembly pool, or (2) corresponding to the same nucleic acid fragment, or (3) corresponding to the two or more nucleic acid fragments in a single pool or reaction compartment is at least about 10, 9, 8, 7, 6, 5, 4, 3, 2, or l degree Celsius higher than the single-mismatch annealing temperature of the overlap having the highest single-mismatch annealing temperature of all overlaps of the said assembly oligonucleotides.

Assembly oligonucleotides are typically assembled into larger nucleic acid fragments by hybridizing two or more assembly oligonucleotides at their mutually reverse complementary overlap regions. Any method for assembling oligonucleotides into nucleic acid fragments can be used, including but not limited to ligation chain reaction (LCR) and self-priming PCR. In instances where fully overlapping assembly oligonucleotides are used, the oligonucleotides can be hybridized and ligated together by LCR according to any protocol known in the art. Exemplary methods for ligase chain reaction assembly of nucleic acid molecules are set out in U.S. patent Nos. 6,472,184 and 5,869,252 or in Wiedmann et al, 1994 ("Ligase Chain Reaction (LCR) -Overview and Applications"; Genome Res. 1994 (3), S51-S64*).* Alternatively, nucleic acid fragments may be assembled by PCR, or by combination of the above methods. In embodiments where partially or redundantly overlapping assembly oligonucleotides are used, fragments may be assembled by overlap PCR as illustrated in FIG. 2. Upon incubation of assembly oligonucleotides under the chosen hybridizing conditions, intermediate fragments may form, comprising two or more assembly oligonucleotides but less than the number of assembly oligonucleotides sufficient to assemble an entire nucleic acid fragment; these may be further amplified and assembled in additional cycles of melting and annealing so as to assemble complete nucleic acid fragments.

In one non-limiting example, hybridization of fully overlapping assembly oligonucleotides to form intermediate nucleic acid molecules, and assembly of intermediate nucleic acid molecules into a nucleic acid fragment, can be accomplished in a single reaction mixture by ligase chain reaction (LCR). Any LCR protocol operable for assembling and ligating assembly oligonucleotides of the length, temperature characteristics, and composition present in the assembly oligonucleotide pool may be used. By way of example only, LCR assembly may comprise performing hybridization/LCR ligation on an assembly oligonucleotide pool using exemplary conditions: 95°C for 2 minutes, followed by 40 cycles 55°C for 5 minutes, and 75°C for 1 minute, wherein assembly oligonucleotides are phosphorylated and present at 50 pM concentration, in 1 x Taq ligase buffer with 20 U Taq ligase, optionally supplemented with 20% PEG in total volume 10 µl. It will be apparent to persons having ordinary skill in the art that other cycle times and temperatures, concentrations, volumes, buffer compositions, and enzymes may be used. LCR assembly results in the hybridization and ligation of a plurality of overlapping assembly oligonucleotides into intermediate nucleic acid molecules and intermediate nucleic acid molecules into desired nucleic acid fragments. The use of stringent hybridization conditions and a hybridization temperature determined in accordance with the disclosures hereof favors hybridization of adjacent assembly oligonucleotides when no mismatches are present, and disfavors hybridization when mismatches are present. For LCR, the ligase used is unable to ligate two assembly oligonucleotides unless they are hybridized to a complementary sequence in a manner that exactly aligns the ends to be conjoined so that the termini of adjacent assembly oligonucleotides abut; thus, any oligonucleotides having errors in their sequences will be selected against for incorporation in the intermediate nucleic acid molecule, nucleic acid fragment, or other ligated product. This helps ensure that a high proportion of the nucleic acid fragment will be error-free, even when assembled from an oligonucleotide pool mixture containing many oligonucleotides having erroneous sequences. These methods can be used with an oligonucleotide pool to produce a plurality of nucleic acid fragments in a single reaction compartment.

In another non-limiting example, assembling nucleic acid fragments comprises using self-priming or overlap PCR, in which chain extension takes place using the single strand regions of hybridized overlaps as templates and the hybridized complementary overlaps as primer, with successive amplification steps assembling increasingly larger polynucleotides until each full nucleic acid fragment is assembled. Nucleic acid fragment assembly by self-priming PCR is illustrated schematically in FIGs. 2 and 3. As with LCR assembly increasing the stringency of the annealing conditions by maintaining annealing temperature at 1, 2, 3, 4, 5, 6 degrees above the calculated Tm generally results in a lower expected probability of hybridization of mismatches resulting from any errors in overlaps, and is expected to lead to a higher proportion of error-free nucleic acid fragments in the assembly pool.

It will be apparent to a skilled person that various combinations of LCR and PCR assembly are possible, and that, whether assembly is performed by LCR, PCR, or a combination of the two, assembly oligonucleotides will hybridize in many possible combinations forming many species of intermediate nucleic acid molecules, and that the number of cycles required to assemble complete nucleic acid fragments will vary. Use of PCR for assembly of nucleic acid fragments is preferred when adjacent sense or (antisense) assembly oligonucleotides are or can be designed to have gaps between them when hybridized to the complementary antisense (or sense) assembly oligonucleotides. However, PCR can also be used for assembly of nucleic acid fragments where fully overlapping assembly oligonucleotides are used. Techniques and specific conditions for PCR are known to those of skill in the art, and involve performing PCR on the pool of assembly oligonucleotides with overlaps, where the assembly oligonucleotides alternate between sense and antisense directions, and the overlaps serve to order the PCR fragments so that they selectively produce the desired nucleic acid fragments. By way of example only, PCR assembly may comprise performing PCR on an assembly oligonucleotide pool using exemplary conditions: 95°C for 1 min, followed by 40 cycles 95°C for 10 sec, 58° C for 20 sec, and 72°C for 15 sec, wherein assembly oligonucleotides present at 50 pM concentration, in 1 x iProof DNA polymerase buffer supplemented with 200 µM dNTP and 2 units of enzyme in total volume 100 µl.

In various preferred embodiments, the incubation of assembly oligonucleotides for hybridization is performed at a temperature that fosters exclusion of incorrect sequences from hybridizing with the overlaps of assembly oligonucleotides as outlined above.

It will be apparent that the stringency of nucleic acid hybridization conditions can be affected or adjusted by other factors, including without limitation and by way of example only, the choice of ligase or polymerase used in, for example, an LCR or PCR reaction, and enzyme cofactors such as magnesium and other composition of the ligase or polymerase buffer. Incubation for hybridization of assembly oligonucleotides is done under hybridizing conditions so as to result in hybridization of the overlaps segments providing a plurality of double stranded intermediate fragments. The determination of appropriate hybridization conditions is well known to those of ordinary skill in the art and will be based, at least in part, on the specific composition, sequence, distribution, and quantities of assembly oligonucleotide species present, and their overlaps and overlap temperature characteristics.

In various embodiments, the result of LCR or PCR assembly is a nucleic acid fragment pool, comprising at least 2, 3, 5, 10, 20, 40 or more distinct nucleic acid fragments, which can be standardized for efficient and unbiased parallel amplification to allow the nucleic acid fragments present in a reaction compartment to be amplified together in a single reaction mixture. Prior to amplification the nucleic acid fragments are at least partially double-stranded but may have single-stranded overhangs.

### Multiplex Fragment Amplification

In various examples where nucleic acid fragments are assembled from array- or chip-synthesized oligonucleotides, the oligonucleotide concentrations are as low as femtomolar or attomolar, resulting in similarly low concentrations of assembled nucleic acid fragments. Therefore, in many instances following multiplex assembly the resulting nucleic acid fragments may be further amplified to obtain larger amounts of each assembly product for downstream processes.

A nucleic acid fragment pool can be amplified in whole or part, by any of the nucleic acid amplification techniques known to persons of ordinary skill in the art. In some examples nucleic acid fragments may be amplified by PCR using primers complementary to fragment terminal regions. Amplification of assembled nucleic acid fragments with terminal primers further increases the amount of full-length assembly products while smaller assembly intermediates that do not comprise the terminal oligonucleotides with universal primer binding sites are excluded from amplification.

In some instances, each nucleic acid fragment in a pool may have substantially identical terminal regions such as universal primer binding sites or "linkers" added during multiplex oligonucleotide assembly as described elsewhere herein. Such linker sequences present at the 5'- and 3'-ends of the fragments may be used for co-amplification of all fragments in a given pool, or to link or attach flanking sequence regions such as adaptor sequences and/or barcodes for downstream sequencing or hybridization sites for analysis or purification etc. Such flanking regions providing desired functionality are more generally referred to as "tags" herein.

Where universal linkers are used for multiplex amplification of nucleic acid fragments having mutual overlaps (*i.e.* complementary sequence regions), it is preferred that no pair of nucleic acid fragments that share an overlap have the same pair of linkers in both of the nucleic acid fragments comprising the pair; otherwise, amplification of the short overlap segment may occur in preference to amplification of the entire fragment. It is possible to ensure that adjacent fragments that share an overlap will not have identical linker pairs by employing two distinct linker pairs in an alternating fashion, so that, for example, where nucleic acid fragment 1 overlaps with nucleic acid fragment 2, which overlaps with nucleic acid fragment 3 and nucleic acid fragment 3 overlaps with nucleic acid fragment 2 and 4, and so forth, amplification of fragment overlaps in preference to complete nucleic acid fragments can be avoided by employing one pair of amplification primers for "odd" fragments and a different pair of amplification primers for "even" fragments. Such distinction between odd and even fragments is typically not required if mutual overlaps between adjacent fragments are very short *(e.g.* if adjacent fragments are assembled into larger nucleic acid molecules by restriction enzyme cleavage and ligation rather than exonuclease-based seamless cloning or fusion PCR-based methods that require longer overlaps). Also, by using the grouping methods described above that take fragment similarity into account, the co-amplification of assembled fragments with large mutual overlaps in the same reaction compartment can be avoided.

An exemplary workflow for multiplex assembly and subsequent multiplex amplification of fragments with universal linkers is shown in FIG. 8(A). In this example in step (1) fragments 1 to 3 are simultaneously assembled in a multiplex assembly reaction from three sets of oligonucleotides having specific overlapping assembly sequence regions. In each set of oligonucleotides those oligonucleotides designed to assemble at the 5'- and 3' termini of a respective fragment share identical sequence regions (referred to as 5' and 3' linkers, respectively) to provide universal primer binding sites that allow for simultaneous amplification of the assembled fragments with a common primer pair in step (2). Post-assembly amplification with terminal primers yields sufficient amounts of assembled fragments for downstream applications. In some instances, step (2) may be conducted with terminal primers that comprise tags. For example, following multiplex assembly, the assembly products may be directly used in an amplification reaction with terminal primers that comprise barcodes and / or sequencing adaptors as described elsewhere herein.

For purpose of illustration, an exemplary multiplex amplification reaction of a mixed nucleic acid fragment pool could comprise: performing PCR on an aliquot of the assembly reaction mixture diluted 10 fold with water, to which has been added 1 x iProof polymerase buffer, 200 µM dNTP, 500 nM primer, and 0.02 u/µl iProof polymerase, 40 cycles each at 95°C for 10 seconds, 55°C for 20 seconds, and 72°C for 15 seconds. It will be apparent to persons having ordinary skill in the art that the volumes, buffer composition, polymerase, temperatures, and other parameters may vary depending upon the compositions of the nucleic acids being amplified, their temperature characteristics, and any other relevant factors.

Amplified fragments may then be subject to further processing steps such as size selection or purification, tagging for downstream analysis or sequencing reactions. In some instances tagging may be conducted in a PCR reaction as shown in step (3) of FIG. 8(A) using "linker"-specific primers with flanking tag sequences to obtain a mixed pool of tagged nucleic acid fragments (step (4) of FIG. 8; flanking tags not shown).

The use of universal primers for multiplex amplification of a mixture of assembled fragments may, however, result in unequal distribution of the different fragments due to assembly and/or amplification bias. For example, some of the fragments may assemble more efficiently than other fragments in the same pool which results in assembly products being present at different concentration. To overcome this problem, the 5'- and/or 3'-linkers may be flanked by fragment-specific primer binding sites which may differ for some or all fragments in a pool, as illustrated in step (1) of FIG. 8B. In this example, each set of oligonucleotides for assembly of a specific fragment comprises a pair of terminal oligonucleotides with fragment-specific primer binding sites (f1 and f1' for the first fragment, f2 and f2' for the second fragment, f3 and f3' for the third fragment etc.). The fragment-specific primer binding sites may be designed to flank the 5'- universal linkers of the terminal oligonucleotides such that they are positioned at the fragment termini following oligonucleotide assembly.

By using fragment-specific primers, the result of a multiplex amplification reaction can be equilibrated. Thus, multiple assembly products can be amplified in the same reaction compartment without competing for the same terminal primers. With reference to the example in FIG. 8B primer pairs f1/f1', f2/f2' and f3/f3' may be used at equal but limited concentrations to avoid amplification bias. Thus, every fragment can be amplified in a similar number of amplification cycles before the primers are exhausted. Alternatively, to equilibrate assembly bias (*i.e.* where fragments are co-assembled at different efficiencies and are therefore present at different concentrations in a mixed pool) primer pairs (*e.g.* f1/f1', f2/f2', f3/f3' etc.) may be added at different concentrations. For example, a first fragment-specific primer pair for amplification of a first fragment may be provided at a lower concentration than one or more fragment-specific primer pairs for a second and optionally further fragment to restrict the amplification of the first fragment as compared to the one or more other fragments in the mixed pool. In another example, a first fragment-specific primer pair for amplification of a first fragment may be provided at a higher concentration than one or more fragment-specific primer pairs for second and optionally further fragments to increase the amplification of the first fragment as compared to the one or more other fragments in the mixed pool. In some instances, a lower concentration of a fragment-specific primer pair may be used for a fragment expected to amplify more efficiently in a mixed pool (*e.g.* a fragment with an "optimal" GC content, and/or average length etc.). In contrast, a higher concentration of a fragment-specific primer pair may be used for a fragment expected to amplify less efficiently in a mixed pool according to the criteria described herein. In some instances a fragment-specific primer pair may be used at a relative amount of between 1 and 1/1,000 (*e.g.* 1/2, 1/5, 1/10, 1/20, 1/50, 1/100). Alternatively, a fragment-specific primer pair may be used at a concentration of between 2 nM and 2,000 nM or between 100 nM and 1,000 nM (*e.g.* 10 nM, 50 nM, 100 nM, 200 nM, 500 nM etc.).

Using fragment-specific primers of the current disclosure allows for increased control of the amplification of a pool of fragments diverse in composition and concentration, thereby increasing the success of downstream processes that rely on equal distribution or representation of assembled and amplified nucleic acid fragments. In some instances, fragment-specific primers may be used to preferably amplify specific fragments in a pool. For example, one or more primer pairs may be added at higher amounts than other primer pairs to over-amplify those fragments that are expected to be out-competed by other fragments in the same pool in downstream process steps such as size selection, purification, amplification, modification, tagging, higher order assembly, enzymatic treatment, error correction, sample preparation and/or sequencing workflows as described elsewhere herein.

In addition, fragment-specific primer binding sites may be useful to selectively amplify or retrieve fragments in a rescue workflow where one or more fragments are "lost" or out-competed in downstream processing steps. For example, in instances where multiple fragments are pooled and sequenced in a single sequencing run to identify error-free molecules, some of the fragments may be underrepresented in the entirety of sequencing reads (due to bias in assembly, amplification, tagging and/or sequencing steps) such that no error-free molecules representing a desired fragment sequence can be found. Where a specific fragment cannot be found, a rescue cycle may require re-amplification of the entire mixed pool in which the respective fragment was assembled. This can be avoided by using fragment-specific primers to selectively amplify only the desired one or more fragments from a given pool which decreases complexity in subsequent processing steps thereby increasing the success rate of identifying the desired fragment in the rescue cycle.

Fragment-specific primer binding sites of terminal oligonucleotides may comprise between about 8 and about 50, between about 10 and about 30 or between about 12 and about 25 bases in length and may be designed sufficiently diverse to avoid cross-hybridization between primer pairs used to amplify different nucleic acid fragments in a multiplex reaction.

In some instances a diverse library of defined fragment-specific primer binding sites and corresponding primer pairs can be provided that can be re-used in different amplification pools. For example, the same f1/f1' primer pair may be used to amplify a first fragment in a first pool of mixed fragments and a second fragment in a second pool of mixed fragments and optionally further fragments in further mixed pools. Likewise, the same f2/f2', f3/f3' and optionally further primer pairs may be used to amplify specific fragments in different mixed pools.

In some examples a library of fragment-specific primer binding sites and/or a library of fragment-specific primer pairs may comprise between about 2 and about 50 or between about 3 and about 30 different sequences or sequence combinations for fragment-specific amplification. A library of fragment-specific primer binding sites for a limited number of targets can be obtained in multiple ways. For purpose of illustration only, in one example a set of random nucleic acid sequences (*e.g*. between about 50 and about 500) of approximate length 17-30 base pairs may be created *in silico* and melting temperature Tm be calculated for all sequences. Those sequences with a Tm outside a predefined Tm-window (*e.g.* any window of ~5°C within 48-72°C) could then be discarded from the set. In addition, in some instances all sequences with a minimum distance of < 3 within 6 base pairs of the 3' end, forming hairpins or loops, or sequences that self-dimerize etc. could be excluded from the set. In other instances, it may be sufficient if sequences with a minimum distance of < 2 within 8 base pairs of the 3' end are excluded. Since the number of fragments that are co-amplified in a given reaction compartment is limited, exclusion criteria for suitable fragment-specific primer binding sites and corresponding primer pairs could be even more rigorous.

Fragment-specific primer binding sites may be removed by various ways after multiplex amplification of the mixed fragments. In one example, fragment-specific primer binding sites can be removed in a nested PCR reaction by using universal linker-specific primers that bind to universal primer binding sites located internal of the fragment-specific primer binding sites in the fragments as illustrated in step (3) of FIG. 8(B). As shown in FIG. 8(A) and (B), amplification with universal primers can be combined with a tagging step to attach tags at one or both termini of the amplified fragments (Note that tags may be present but are not shown in step (4) of FIG. 8). This can be achieved by using tagged universal primers as described elsewhere herein. Alternatively, where fragment-specific primer binding sites are flanked by specific restriction enzyme cleavage sites *(e.g.* type IIs cleavage sites), they may be removed using enzymatic cleavage. In such instance, subsequent tagging may occur by ligation.

Processing of amplified fragments (such as removal of fragment-specific primer binding sites and/or tagging) may occur in parallel in the various fragment pools or may be conducted in a single reaction mixture after pooling the assembly reaction products from two or more reaction compartments.

### Fragment Pooling and Processing

Nucleic acid fragments may be obtained by any of the multiplex assembly methods described herein. In some instances, the plurality of assembly pools comprises between about 10 and about 1,000, or between about 3 and about 100 or between about 5 and about 500 or between about 50 and about 5,000 pools. In some instances the assembly products in each pool comprise a mixture of correctly assembled nucleic acid fragments and incorrectly assembled nucleic acid fragments. In some instances the assembled (and amplified) fragments may be optionally purified, *e.g.,* by using "Solid Phase Reversible Immobilisation" selection methods or other methods described herein or known in the art.

Some or all of the fragments assembled (and amplified) in one or more multiplex reactions according to methods disclosed herein may be pooled into a single target reaction compartment for further processing. For example, multiple sets of between 2 and 30 fragments assembled in separate multiplex reactions may be pooled together. In some instances a target reaction compartment may comprise fragments from 10 to about 100 assembly pools with each pool comprising one or more (*e.g*. between 3 and 30) distinct nucleic acid assembly products. In some instances, a target reaction compartment may comprise fragments from about 20 to about 500 assembly pools with each pool comprising one or more, preferably two or three or more (*e.g*. between 2 and 50, between 3 and 100) distinct nucleic acid assembly products. In some instances, a fragment pool may comprise between about 30 and about 3,000 nucleic acid fragments. In some examples, the reaction products of between about 20 and about 500 multiplex assembly reactions may be pooled together to obtain a fragment pool. A fragment pool may have between about 10⁷ and 10¹⁰ different nucleic acid molecules taking sequence errors *(i.e.,* sequences that deviate from the sequence of a desired nucleic acid molecule) into account. Pooled fragments may then be subject to further processing steps. Such processing steps may include size selection, purification, amplification, modification, tagging, higher order assembly, enzymatic treatment, error correction, sample preparation and/or sequencing as described in more detail below.

The success of a downstream processing step may depend on the distribution and/ or representation of the various assembly products in the target reaction compartment. In some instances equal amounts of each fragment mixture may be retrieved from the multiple assembly pools. This can be done, for example, by quantifying the nucleic acid assembly products in each assembly pool and pipetting respective volumes representing equal amounts of the assembly products from each desired assembly pool into the target pool compartment.

However, not every fragment may be assembled with the same efficiency and be present at equal or similar amounts in a given assembly pool. In particular, there are certain biases within the multiplex assembly workflow that favor fragments with some characteristics, and disfavor fragments with other characteristics. In addition, such sequence-specific biases may also affect downstream sample preparation and sequencing processes. Examples include: (1) mean GC-content of a fragment sequence *(i.e.* low GC content and very high GC content may be more difficult to assemble or may cause bias in downstream high throughput sequencing); (2) mean fragment length *(i.e.* longer fragments may contain more errors and more of these may need to be screened to find correct ones); (3) concentration of each assembly product in a mixed pool (a quantitative concentration measurement for every assembled fragment would be required); (4) "complex DNA regions" and (5) "rescue" workflows (*e.g*. re-synthesis, re-assembly or re-sequencing of fragments that failed to assemble or could not be identified or isolated in downstream steps.

"Complex DNA regions" as used herein comprise any sequence region of a DNA template that may affect the efficiency and/or correctness of one or more of assembly, amplification or processing steps in a fragment assembly workflow including multiplex assembly, amplification and tagging for sequencing sample preparation and/or a sequencing reaction. Complex DNA regions may include sequences that comprise one or more of the following:
(i) homopolymers: stretches of identical bases of a certain length *(e.g.* more than 4, 5, 6, 7, 8, 9, 10 etc.) have been shown to be problematic in certain sequencing workflows.
(ii) GC-rich sequence stretches: for example, sequences with > 60-65% GC content; GC-rich nucleic acids often have unique secondary structures and melting temperatures due to the greater proportion of nucleotides with three hydrogen bonds which makes such nucleic acids more difficult to denature and process, and may lead to a more challenging sequencing reaction.
(iii) sequence repeats such as di- and trinucleotide, direct, inverted and Alu repeats. Examples are: AG, CA, CT, GT, AGG, ACC, CCG, CCT, CTT, GCC, GGA, CCCTTT, and any other combination of these repeats; it has been shown *e.g*. that long stretches of repeats (> 40) containing only two nucleotides that do not necessarily form repeats may also be difficult to sequence.
(iv) hairpin structures: such structures occur when two regions of the same strand, (usually complementary in nucleotide sequence when read in opposite directions), base-pair to form a double helix that ends in an unpaired loop. A hairpin may consist *e.g.* of two inverted repeats, separated by at least three nucleotides.

The above problems can be solved by analysis of these biases (*e.g*. by analyzing large sets of data from previous reaction cycles) and using the obtained data to optimize fragment pooling and processing to counter-act these biases.

In some aspects, analysis of biases may comprise a prediction of error rates associated with certain fragments. Assembly pools comprising fragments that are predicted to possess higher error rates may then be added at higher concentration (*e.g*. by pipetting a higher volume of said pools) to subsequent reactions than those ones with a lower predicted error rate. For example, pools with GC-rich fragments that are expected to have a higher error rate *(e.g.* due to transversions G>T, C>A or transitions G>A, C>T during oligonucleotide synthesis) may be added at higher concentration/volume to downstream reactions than pools comprising fragments with low GC content. Alternatively, lower concentrations/volumes of those pools expected to have less errors may be added to the mixture. Thus, methods of pooling assembly products disclosed herein may comprise a step of adjusting pooling dilutions to predicted error rates.

In an exemplary workflow according to FIG. 1, nucleic acid fragments co-assembled in 80 multiplex assembly reactions (with 3 fragments simultaneously assembled in each pool) were combined into a single pool, tagged with barcodes and sequencing adaptors and sequenced using a sequencing-by-synthesis platform. In this example, the analysis of the total number of sequencing reads demonstrated a significant bias against nucleic acid fragments comprising a GC content below 35% (see FIG. 9(A) where the peak of sequencing reads correlates with a mean fragment GC content between about 35% and about 65%). The analysis of the sequencing reads to identify correct *(i.e.* error-free) fragments yielded a large fraction of sequences with GC contents below 40% or above 65% for which no reads could be obtained (see FIG. 9(B) indicating the number of non-identified fragments weighted for the number of input sequences). Thus, in this exemplary setting the success rate for finding a correctly assembled/processed nucleic acid fragment clearly depended on the fragment GC content. This affect may be mitigated by increasing the number of molecules from "difficult" fragments in a target pool for downstream processing.

For example, in instances where one or more assembly pools comprise fragments with a GC content below or above the optimum range, a higher quantity of those one or more pools comprising fragments with a GC content outside the "optimum" range could be added to the target reaction compartment. For example, the added quantity could be 2-, 3-, 4-, 5-, or 10-times etc. the quantity of those one or more assembly pools comprising fragments with optimum GC content (*e.g*. between about 40% and about 65%). Alternatively, lower quantities of those pools comprising fragments with optimum GC content could be added *(e.g.* 90%, 80%, 70%, 60%, 50%, 40%, 30%, 20%, 10%, 5% etc. of the quantity added from one or more pools comprising fragments with a GC content outside the "optimum" range). A similar analysis can be performed for other criteria (such as fragment length, homopolymers etc.) and pooling quantities can be adjusted accordingly.

In many instances the fragments or mixtures of fragments obtained by multiplex assembly will vary in length which may affect the efficiency of downstream processing steps. First, subsequent PCR amplification reactions often used to attach barcodes and/or sequencing adaptors, which is also referred to herein as "tagging" may be less efficient for longer fragments. Second, errors accumulate with fragment length. In some instances it may therefore be desired to have longer fragments overrepresented in downstream processing steps (such as amplification, tagging and/or sequencing) such that more of the longer fragments can be sequenced or analyzed to identify error-free molecules. Third, it has been shown that sequencing signals typically decrease with increased read length, which makes it more difficult to obtain evaluable sequencing data (including consensus sequences) for longer fragments. To address the above biases, the concentration of fragments in a target reaction compartment may be adjusted according to the fragment length differences. Thus, in many instances longer fragments may be added at higher quantities than shorter fragments to balance or optimize processing and readout in downstream sample preparation and/or sequencing workflows.

Further, it has been observed that under some circumstances bead-based purification of nucleic acid fragments prior to pooling may result in losses of portions of shorter fragments. Thus, where appropriate, the pooling of assembly products may comprise adjusting the pooled quantities to generate a fragment mixture having higher molar amounts of the shortest and the longest fragments. In some instances the group of shortest fragments may comprise the 10% or 20% shortest fragments in the pool, whereas the group of longest fragments may comprise the 10% or 20% longest fragments in the pool. In some instances the shortest and/or the longest fragments may be added to the mixed pool at a ratio of 10: 1. In other instances the shortest and/or the longest fragments may be added to the mixed pool at a ratio of between 10:1 and 50:1.

By combining different volumes or quantities of assembly pools with certain characteristics, more (correctly) processed nucleic acid fragments can be screened and/or identified in downstream analysis, even where fragments may be difficult to assemble or difficult to sequence based on the above mentioned biases. Analysis results and data from a given production cycle can then be used to further optimize fragment grouping and/or fragment pooling criteria for a subsequent production cycle as illustrated by the "feedback loop" arrows in FIG. 1.

Thus, the present disclosure further provides methods for combining and processing of nucleic acid assembly products from multiple assembly pools comprising the following steps:
(i) providing a target reaction compartment and a plurality of assembly pools wherein each assembly pool comprises one or more distinct nucleic acid assembly products. (In some examples, most of the assembly pools may comprise at least two distinct nucleic acid assembly products and some assembly pools may comprise only one distinct nucleic acid assembly product),
(ii) assigning a dilution factor to each assembly pool, wherein the dilution factor indicates the degree of dilution (*e.g.* x-fold dilution) of an assembly pool in the target reaction compartment and wherein the dilution factor is determined based on one or more sequence parameters of the nucleic acid assembly products present in a pool,
(iii) optionally determining the concentration of the nucleic acid assembly products in each pool and optionally adjusting the nucleic acid concentration of one or more of the nucleic acid assembly pools to obtain equimolar nucleic acid solutions of each assembly pool;
(iv) combining a first quantity of assembly products from a first assembly pool and at least a second quantity of assembly products from at least a second assembly pool into the target reaction compartment, wherein the first and at least second quantities are determined based on the dilution factors assigned to the first and at least second assembly pools,
(v) optionally purifying the combined assembly products;
(vi) tagging the combined assembly products;
(vii) sequencing the tagged assembly products or a portion thereof to obtain sequencing reads;
(viii) analyzing the obtained sequencing reads to identify error free assembly products, and
(ix) retrieving the error-free assembly products.

FIG. 10 shows an exemplary workflow for combining and processing of nucleic acid assembly products from multiple assembly pools. In step (1) multiple nucleic acid fragment pools *(e.g.* between about 30 and about 500) each comprising one or more *(e.g.* between 2 and about 20) nucleic acid assembly products (that may have been amplified) are provided. Within the assembly pools the fragments are grouped according to GC content, fragment length and similarity as described above. As exemplified by the Table in FIG. 10, an overall dilution factor is determined for each assembly pool based on the sequence parameters represented by the nucleic acid fragments in each pool. The sequence parameters may be selected from one or more of fragment length, GC content, complex DNA regions (as described above), number of distinct nucleic acid assembly products per pool, assembly product concentration per pool, and/or number of screening repetitions. In some instances the dilution factor of each pool is determined by weighting the one or more sequence parameters according to downstream processing requirements or biases as specified above. For example, GC content may be weighted against fragment length and/or homopolymers etc. Optionally, prior to the combining step the nucleic acid concentration in each assembly pool may be determined (*e.g*. by OD measurement) and concentrations of one or more of the assembly pools may be adapted to obtain equimolar nucleic acid solutions. In step (2) different quantities or volumes of two or more of the assembly pools are combined into a target reaction compartment for subsequent processing, wherein the quantities are determined based on the assigned dilution factors. The dilution factor assigned to a specific pool indicates the relative quantity of a given assembly pool that needs to be added to the target reaction compartment to achieve the desired dilution/distribution of distinct assembly products in the target pool mixture. For example, where the dilution factor assigned to a first assembly pool is higher than the dilution factor assigned to a second assembly pool, a higher quantity of the first assembly pool would be added and a lower quantity of the second assembly pool would be added to the target reaction compartment. Thus, in this example, the first assembly pool would be diluted less in the target reaction compartment (*e.g*. 2-fold) than the second assembly pool *(e.g.* 5-fold). In some instances, one or more assembly pools comprising assembly products with similar sequence characteristic (*e.g*. all pools comprising fragments with low GC content) may be combined and then added to a target reaction compartment in more quantity. In one example, those assembly products assembled in separate pools *(e.g.* fragments with long homopolymers or specific sequence characteristics) may be combined and then added to the target compartment at higher quantity. In another example an assembly pool comprising only one desired assembly product may be added at a lower quantity to the target compartment than an assembly pool comprising two or more assembly products to ensure that assembly products derived from the first and second pools are represented at equal amounts in the target compartment. In yet another example, an assembly pool with longer assembly products and/or assembly products with a very low or very high GC content may be added at higher quantities to the target compartment than assembly pools with shorter assembly products and/or assembly products with an optimum GC content as described above.

For purpose of illustration only, the Table in FIG. 10 shows an exemplary pool 1 with an overall dilution factor of 3.3. In this example, the overall factor is expressed as the sum of factors determined for each weighted sequence parameter in a given pool, where GC content is assigned factor 1, concentration is assigned factor 1.3, fragment length is assigned factor 1.5, screening repetition for rescue is assigned factor 1, respectively. Pool 2 has an overall dilution factor of 5.1. The assembly products from both pools can be combined such that in the target reaction compartment pool 1 is diluted by factor 3.3 and pool 2 is diluted by factor 5.1. This would result in a final mixture comprising 39% (= 3.3/(3.3+5.1)) of assembly products of pool 1 and 61% (= 5.1/(3.3+5.1)) of assembly products of pool 2. The skilled person understands that dilution factors may be determined and expressed in multiple ways and that the above example is only of illustrative nature. In step (3) the pooled fragments are optionally purified, tagged and prepared for sequencing. After sequencing the obtained sequencing reads are analyzed in respect of key sequence parameters (GC content, length, homopolymers, successful rescues/number of screening repetitions needed etc. with an optimum target indicated as dotted line in the plots to the right. The target lines in this example represent an optimum solution, wherein the sum of all distances from all dots to the lines of all plots is minimal. In step (4) the analysis results can be used to adjust dilution factors and thus, the quantities of assembly reactions to be combined to improve downstream processing and sequence analysis as indicated by multiple arrows. In addition the analysis results can be used to improve and fine-tune the criteria for grouping and distributing the fragments (or the respective sets of oligonucleotides) into the plurality of assembly pools. In some instances, an artificial intelligence and/or machine learning algorithm can be employed to determine and fine-tune grouping and pooling criteria.

Intelligent pooling of assembly products based on the above criteria can increase speed and success rate of downstream processing workflows (such as sequencing and retrieval of correctly assembled fragments) which in turn leads to a higher predictability of the sequences that can be synthesized and assembled using workflows described herein.

Once nucleic acid fragments from multiple assembly reactions have been combined they may be subject to further processing steps including steps of purifying, tagging, sequencing and analyzing as described in more detail below.

In some instances, a purification step may be performed to separate assembly products from reaction mixture components (e.g., dNTPs, primers, truncated fragments, buffers, salts, enzymes, etc.) to facilitate a downstream process. This may be done in a number of ways, such as, *e.g.,* by enzymatically removing undesired nucleic acid side-products with an exonuclease, restriction enzyme or UNG glycosylase. In some instances, nucleic acid fragments may be filtered, precipitated or bound to a solid support such as magnetic beads (*e.g*. Ampure XP beads, ChargeSwitch^{™} PCR Clean-Up Kit, Thermo Fisher Scientific) to separate assembled fragments of a desired length from truncated products, assembly oligonucleotides or other reaction components. In some examples nucleic acid fragments may be purified by size-exclusion, electrophoresis or any other purification method known to persons of ordinary skill in the art. Purification of nucleic acid fragments may be performed at various stages of the workflows disclosed herein such as *e.g.* prior to pooling *(e.g.* after multiplex nucleic acid fragment amplification) or after pooling. Purified nucleic acid fragment may then be used in additional reactions (*e.g.,* assembly reactions, amplification, cloning etc.).

In some examples, pooled nucleic acid fragments may be tagged with additional sequences (such as adaptors and/or barcodes) required for downstream analysis, selection or sequencing workflows. Nucleic acid fragments may be tagged at one end or at both ends. Tagging may be achieved by various means. In some examples tagging may be achieved by PCR amplification of the nucleic acid fragments with universal or fragment-specific primers In some examples, barcodes and/or sequencing adaptors may be conjugated to each fragment by fusion PCR via overlapping ends as described in standard protocols. In many instances the tagging is achieved by using modified PCR primers that comprise the required adaptors and/or barcodes at their 5' ends. The primers which further comprise a target-specific region at their 3' ends that is capable of hybridizing to the respective 5' ends of the template strands of a given fragment are then extended in a PCR reaction resulting in copies of the template flanked by the sequencing adaptors and/or barcode(s).

Alternatively, barcodes and/or sequencing adaptors can be blunt-end ligated to both ends of each fragment in the presence of a ligase *(e.g.,* T4 DNA ligase, Thermo Fisher Scientific). Ligation-based tagging may be preferred in certain instances as barcodes and/or adaptors can be designed without overlaps for fusion PCR resulting in shorter tags and thus, reduced synthesis efforts. In case of ligation, the barcodes are provided as double-stranded molecules.

Barcodes may either be degenerate or comprise defined sequences and may be derived from a barcode library. Depending on the read length, the sequencing platform used and the method used for tagging such library may comprise barcodes that are about 10 to about 60 bases or about 30 to about 50 based in length. Barcodes and/or sequencing adaptors and/or linkers may further be designed to comprise at least one recognition site for a restriction endonuclease (*e.g.,* a type IIs restriction endonuclease) and/or a cleavage site for said restriction enzyme. Such cleavage site can be used to remove undesired sequence segments that are not required for the assembly of a desired nucleic acid molecule. In examples, the cleavage and recognition sites do not overlap. For example, the restriction site may be a type IIs restriction enzyme cleavage site. Alternatively a restriction enzyme recognition site may be contained in the nucleic acid fragments and respective cleavage sites may be positioned to allow for complete removal of barcode and/or sequencing adaptors prior to subsequent assembly with other fragments as laid down below.

In some examples, the barcode library may be designed such that each barcode is sufficiently different from another barcode in the library so that the library of barcodes can be used as primers to specifically anneal to a sequence-verified desired target sequence to allow for extension to produce a double-stranded nucleic acid molecule.

In some instances, each molecule within the mixed population of nucleic acid fragments may receive an individual barcode. This may be achieved by using a barcode library with a diversity that exceeds the diversity of nucleic acid fragments in the pool. In other instances it may not be required to tag each molecule with an individual barcode.

In some examples, tagged nucleic acid fragments may be clonally amplified prior to sequencing. Clonal amplification may be conducted in solution and the amplified nucleic acid fragments may be attached to a solid support *(e.g.,* a bead). In other examples, clonal amplification may be conducted on a support, for example on beads. Clonal amplification of nucleic acid templates can be achieved by any means known to those of skill in the art. In some instances, clonal amplification can comprise spatially separating individual fragments and performing amplification of the separated fragments. One method that may be used to obtain multiple copies of spatially separated individual nucleic acid molecules is by emulsion PCR. Emulsion polymerase chain reaction (PCR) is a single-molecule PCR that occurs in "cell-like" compartments. In simple terms, emulsion PCR is used to amplify individual DNA molecules in aqueous droplets within an oil phase. Further, these amplified nucleic acid molecules may be localized on beads. Emulsion PCR methods are set out in U.S. Patent Nos. 7,947,477 and 9,121,047.

Another method allowing for monoclonal amplification of nucleic acid templates on solid support(s) is referred to as "bridge amplification" which can be conducted under isothermal conditions. Examples of compositions and methods for bridge amplification are found in U.S. Pat. Nos. 7,790,418, 7,985,565, 8,143,008 and 8,895,249.

Yet another amplification method for generating monoclonal nucleic acid populations is known as "template walking" typically performed with only the forward primer immobilized on the support whereas a reverse primer may be provided in solution. Examples of compositions and methods for nucleic acid template walking are found in U.S. Patent Public. Nos. 2012/0156728, 2013/0203607 and in PCT Publication WO 2013/158313. Partial strand separation in template walking may be achieved by using low melt primers and/or adaptor sequences or alternatively, by using recombinase-polymerase amplification (RPA) under isothermal conditions. Examples of compositions and methods for RPA reactions are found in U.S. Patent Public. Nos. 2013/0225421 and 2014/0080717, and in U.S. Pat. Nos. 7,399,590, 7,666,598, 8,637,253, 8,809,021, and 9,057,097.

Clonal amplification technique may be selected according to the amount or volume of amplicons required for downstream processing. For example, isothermal amplification on beads may be chosen to obtain sufficient amounts of bead-bound amplicons that can be further divided into sub-pools for differential treatment. Following clonal amplification, amplicon-carrying beads may be enriched *(i.e.,* separated from those beads not carrying amplicons). This may be achieved by various means, such as, *e.g*., biotinylating the amplicons and capturing the biotinylated amplicons with streptavidin-coated beads *(e.g.,* MyOne^{™} Streptavidin beads; Thermo Fisher Scientific), or by using beads coated with a probe that specifically hybridizes to the free ends of the bead-bound amplicons. SPRI beads (as described, *e.g.,* in DeAngelis et al. "Solid-phase reversible immobilization for the isolation of PCR products", 1995 23(22):4742-3) may likewise be used for purifying clonal amplicons. The skilled person may chose whatever purification or enrichment method is compatible with the used sequencing platform. Furthermore, a denaturation step may be required to render the amplicons single-stranded for subsequent sequencing. This can be achieved, for example by treating the amplicons with an NaOH solution or any other method recommended by suppliers of the respective sequencing platform.

Sequence verification prior to full-length or multi-fragment nucleic acid assembly may be useful to avoid the carry-over of errors from chemically synthesized nucleic acid molecules into amplification reactions thereby multiplying the errors in assembled polynucleotides. This may be done for example by sequencing a pool of amplified nucleic acid molecules to determine if any errors are present and retrieving one or more error-free molecules for downstream processing. Thus, sequencing techniques may be applied to identify and select error-free nucleic acid molecules for amplification and subsequent assembly.

Sequencing of nucleic acid fragments to verify sequence correctness may be conducted by any sequencing method known in the art including Maxam-Gilbert sequencing, chain-termination sequencing (*e.g.,* Sanger sequencing), pyrosequencing, sequencing by synthesis and sequencing by ligation. Methods of the disclosure may use any type of sequencing platform suitable for the intended purpose, including any next-generation sequencing platform such as: sequencing by oligonucleotide probe ligation and detection (*e.g.,* SOLiD^{™} from Thermo Fisher Scientific, (see *e.g.,* PCT Publication No. WO 2006/084131), probe-anchor ligation sequencing (*e.g.,* Complete Genomics^{™} or Polonator^{™}), sequencing-by-synthesis (*e.g.,* Genome Analyzer^{™} and HiSeq^{™}, from lllumina), pyrophosphate sequencing (*e.g.,* Genome Sequencer FLX from 454 Life Sciences), single molecule sequencing platforms (*e.g.,* HeliScope^{™} from Helicos^{™}), and ion-sensitive sequencing (*e.g.,* Personal Genome Machine, Proton and Ion S5 from Ion Torrent^{™} Systems, Inc.) as set out, *e.g.,* in PCT Publication WO 2012/044847 or U.S. Patent No. 7,948,015. For an overview, see, *e.g*., Mardis E.R., "Next-Generation Sequencing Platforms", Annu. Rev. Anal. Chem. 6:287-303 (2013).

The obtained sequencing reads can then be used to identify correctly assembled or error-free nucleic acid fragments and their corresponding barcodes. This may be achieved by aligning reads with identical barcodes to determine a consensus sequence for each given barcode. Sequences with identical barcodes are aligned to determine if a consensus sequence of the region of interest can be identified. If a consensus sequence can be obtained based on the alignment the consensus sequence can be compared to a reference sequence to identify correct nucleic acid fragments. If there is a 100% match against one of the reference sequences, the fragment is identified as error-free fragment and can be retrieved. Sequence-verified nucleic acid fragments may be retrieved by various means.

One exemplary method for retrieving a sequence-verified nucleic acid molecule from a mixture based on its individual barcode signature is referred to as "dial-out PCR" and is described *e.g*., in U.S. Patent Publication No. 2012/0283110, 2012/0322681 or 2014/0141982. For dial-out PCR, the desired nucleic acid fragment needs to be amplified from a complex mixture of nucleic acid fragments wherein the generated clonal populations are not kept separate during the amplification. Once the individual barcode signature of an error-free nucleic acid fragment has been identified, complementary barcode primers (*i.e.,* oligonucleotides designed to specifically hybridize to individual barcodes of the desired amplicons) can be added to the fragment mixture or portions thereof to selectively amplify the fragment carrying the specific barcodes. Such barcode primers may either be synthesized individually or retrieved from a barcode primer library. For example, barcode primers may be synthesized on a microfluidic chip or microarray platform as described elsewhere herein.

In instances, where larger nucleic acid molecules are to be assembled from two or more nucleic acid fragments, more than one correct fragment can be targeted simultaneously by adding the required number of specific barcode primers to the reaction compartment (*e.g.,* a specific well of a multiwell plate).

To avoid re-introduction of errors a dial-out PCR reaction should be conducted in the presence of a high fidelity polymerase. A non-exhaustive list of suitable polymerases includes Pfu DNA polymerase (Thermo Fisher Scientific), Deep Vent^{®} DNA polymerase (New England Biolabs), Q5^{®} High-Fidelity DNA Polymerase (New England Biolabs), Phusion^{®} (New England Biolabs), Phusion^{®} Hot Start Flex (New England Biolabs), PrimeSTAR^{®} HS (TAKARA), PrimeSTAR^{®} GXL (TAKARA), PrimeSTAR^{®} Max (TAKARA), AccuPrime^{™} Pfx (Thermo Fisher Scientific), Platinum^{™} DNA Polymerase High Fidelity (Thermo Fisher Scientific), Phusion^{®} Flash II DNA Polymerase (Thermo Fisher Scientific), Phusion^{®} Hot Start II High-Fidelity DNA Polymerase (Thermo Fisher Scientific), Accura^{®} High-Fidelity polymerase (Lucigen), iProof^{™} High-Fidelity polymerase (Bio-Rad), PAN PowerScript DNA Polymerase (PAN Biotech), Platinum^{™} SuperFi^{™} DNA Polymerase (Thermo Fisher Scientific) or TrueScript^{™} DNA polymerase (PAN Biotech).

In some examples, released or dialed-out fragments may be collected from one or more reaction chambers and mixed with other fragments for further assembly. In examples, where multiple different fragments are present in the same reaction chamber, assembly of released or dialed-out fragments may be directly affected in said chamber by adding required assembly reagents.

Another method of selective retrieval of desired nucleic acids is referred to as "laser catapulting" which relies on the use of high-speed laser pulses to eject selected clonal nucleic acid populations from a sequencing plate. This method is described, for example, in U.S. Patent Publication No. 2014/0155297

Other methods for retrieving sequence-verified nucleic acid molecules are set out in U.S. Patent No. 8,173,368. The described method comprises the steps of monoclonizing nucleic acids from a mixture of different nucleic acid molecules, parallel sequencing of the monoclonized nucleic acids, identifying and localizing an individual nucleic acid with a desired sequence, and isolating the individual nucleic acid with the desired sequence for further processing. Localization of the desired sequence is effected by immobilization of the support during sequencing and molecules having the desired sequence are then removed directly from the sequencing reaction support. Retrieval of the selected nucleic acids may be accomplished by isolating of beads comprising the selected nucleic acid, cleaving off the nucleic acids from the respective support on which they are immobilized, selective amplification by spatially-resolved addition of PCR reagents or elution by a laser capture method.

### Error Correction

The disclosure further includes compositions and methods for multiplex assembly of nucleic acid molecules with high sequence fidelity. High sequence fidelity can be achieved by several means, including sequencing of oligonucleotides, nucleic acid fragments or final assembly products assembled from multiple nucleic acid fragments to identify and retrieve ones with correct sequences. Alternatively, error correction may be performed at one or more steps along the workflow as indicated in FIG. 1. Errors may find their way into nucleic acid molecules in a number of ways. Examples of such ways include chemical synthesis errors, amplification/polymerase mediated errors (especially when non-proof reading polymerases are used), and assembly mediated errors (usually occurring at nucleic acid fragment junctions). Errors may be removed or prevented by methods, such as, the selection of nucleic acid molecules having correct sequences, error correction, and/or improved chemical synthesis methods.

In some instances, methods of the disclosure will combine error removal and prevention methods to produce nucleic acid molecules with relative low numbers of errors. Thus, assembled nucleic acid molecules produced by methods of the disclosure may have error rates from about 1 base in 1,500 to about 1 base in 30,000, from about 1 base in 2,000 to about 1 base in 30,000, from about 1 base in 4,000 to about 1 base in 30,000, from about 1 base in 8,000 to about 1 base in 30,000, from about 1 base in 10,000 to about 1 base in 30,000, from about 1 base in 15,000 to about 1 base in 30,000, from about 1 base in 10,000 to about 1 base in 20,000, etc.

In most instances, regardless of the method by which a larger nucleic acid molecule is generated from chemically synthesized oligonucleotides, errors from the chemical synthesis process will be present. While sequencing of individual nucleic acid molecules may be performed to identify and select error-free nucleic acid molecules as described above, alternative approaches may comprise one or more error correction or removal steps. Thus, in many instances, error correction will be desirable. Error correction can be achieved by any number of means. Typically, such error removal steps will be performed after a first round of assembly. Thus, in one aspect, methods of the disclosure involve the following (in this order or different orders): (i) fragment amplification and/or assembly (*e.g.,* according to the methods described herein), (ii) error correction, (iii) final assembly (*e.g.,* according to the *in vitro* or in vivo methods described herein,).

In many instances, one or more ligase may be present in reaction during error correction. It is believed that some endonucleases used in error correction processes have nickase activity. The inclusion of one or more ligase is believed to seal nicks caused by such enzymes and increase the yield of error corrected nucleic acid molecules after amplification. Exemplary ligases that may be used are T4 DNA ligase, Taq ligase, and PBCV-1 DNA ligase. Ligases used in the practice of the disclosure may be thermolabile or thermostabe (*e.g.,* Taq ligase). If a thermolabile ligase is employed, it will typically need to be added to a reaction mixture for each error correction cycle. Thermostable ligases will typically not need to be re-added during each cycle, so long as the temperature is kept below their denaturation point.

One exemplary process for error correction that may be used in methods disclosed herein is also set out in U.S. Patent No. 7,704,690. Another process for effectuating error correction in chemically synthesized nucleic acid molecules that may be used in methods of the disclosure is by a commercial process referred to as ERRASE^{™} (Novici Biotech). Yet another process for reducing errors during nucleic acid synthesis that may be used in aspects of the disclosure is referred to as Circular Assembly Amplification and described in PCT Publication WO 2008/112683.

Synthetically generated nucleic acid molecules typically have error rate of about 1 base in 300-500 bases. Conditions can be adjusted so that synthesis errors are substantially lower than 1 base in 300-500 bases. Further, in many instances, greater than 80% of errors are single base frame shift deletions and insertions. Also, less than 2% of errors result from the action of polymerases when high fidelity PCR amplification is employed. Therefore, error-correction processes using PCR-based assembly steps as described above may be combined with one or more error-correction methods not involving polymerase activity. In many instances, mismatch endonuclease (MME) correction will be performed using fixed protein:DNA ratio. Non-PCR-based error correction may, *e.g.,* be achieved by separating nucleic acid molecules with mismatches from those without mismatches by binding with a mismatch binding agent in a number of ways. For example, mixtures of nucleic acid molecules, some having mismatches, may be (1) passed through a column containing a bound mismatch binding protein or (2) contacted with a surface *(e.g.,* a bead (such as a magnetic bead), plate surface, etc.) to which a mismatch binding protein is bound.

Exemplary formats and associated methods involve those using surfaces or supports (*e.g.,* beads) to which a mismatch binding protein is bound. For example, a solution of nucleic acid molecules may be contacted with beads to which is bound a mismatch binding protein. One mismatch binding protein that may be used in various aspects of the disclosure is MutS from Thermus aquaticus the gene sequence of which is published in Biswas and Hsieh, J. Biol. Chem. 271:5040-5048 (1996) and is available in GenBank, accession number U33117. Furthermore, mismatch cleavage endonucleases such as T7 endonuclease I or Cel I from, for example, celery may be genetically engineered to inactivate the cleavage function for use in error filtration processes based on mismatch binding. Nucleic acid molecules that are bound to a mismatch binding protein may either be actively removed from a pool of nucleic acid molecules (*e.g.,* via magnetic force where magnetic beads coated with mismatch binding proteins are used) or may be immobilized or linked to a surface such that they remain in the sample whereas unbound nucleic acids are removed or transferred (*e.g.,* by pipetting, acoustic liquid handling etc.) from the sample. Such examples are set out, for example, in PCT Publication WO 2016/094512.

Error correction methods and reagents suitable for use in methods of the disclosure are set out in U.S. Patents Nos. 7,838,210 and 7,833,759, U.S. Patent Publication No. 2008/0145913 (mismatch endonucleases), PCT Publication WO 2011/102802, and in Ma et al., Trends in Biotechnology, 30(3):147-154 (2012). Furthermore, the skilled person will recognize that other methods of error correction and/or error filtration (*i.e.*, specifically removing error-containing molecules) may be practiced in certain examples of the disclosure such as those described, for example, in U.S. Patent Publication Nos. 2006/0127920, 2007/0231805, 2010/0216648, or 2011/0124049.

### Multi-Fragment Assembly

Where larger nucleic acid molecules are needed two or more selected nucleic acid fragments may be combined and assembled using any of the assembly methods known in the prior art. In some instances, the selected nucleic acid fragments may be purified prior to or after combination by standard methods (such as by bead-based extraction, gel extraction, column purification and/or alcohol precipitation) before they are assembled into larger nucleic acid molecules.

In some aspects, larger nucleic acid molecules assembled from two or more nucleic acid fragments may have lengths from about 500 base pairs to about 100,000 base pairs, from about 1,000 base pairs to about 50,000 base pairs, from about 2,000 base pairs to about 10,000 base pairs, , etc.

Any number of methods may be used for nucleic acid amplification and higher order assembly. In some instances multi-fragment assembly may be accomplished by using PCR or LCR methods as set out above. In some examples, an exonuclease based seamless cloning strategy may be used. One exemplary method is described in Yang et al., Nucleic Acids Research 21:1889-1893 (1993) and U.S. Patent No. 5,580,759. In the process described in Yang *et al.,* a linear vector is mixed with double-stranded nucleic acid molecules which share sequence homology at the termini. An enzyme with exonuclease activity *(i.e.,* T4 DNA polymerase, T5 exonuclease, T7 exonuclease, etc.) is added which generates single-stranded overhangs of all termini present in the mixture. The nucleic acid molecules having single stranded overhangs are then annealed and incubated with a DNA polymerase and deoxynucleotide triphosphates under condition which allow for the filling in of single-stranded gaps. Nicks in the resulting nucleic acid molecules may be repaired by introduction of the molecule into a cell or by the addition of ligase. Of course, depending on the application and workflow, the vector may be omitted. Further, the resulting nucleic acid molecules, or sub-portions thereof, may be amplified by polymerase chain reaction.

Exonucleases suitable for use in such methods may either have 5' activity (such as T5 exonuclease or T7 exonuclease) or 3' activity (such as 3' exo activity of T4 polymerase).

To allow for optimal hybridization of the compatible ends, the homologous regions should be at least 12 nucleotides in length (such as, *e.g.,* from 12 to about 30 or from 15 to about 50 bp). In some instances the assembly efficiency may be enhanced by shortening the overhangs outside the homologous regions, *e.g*., via cleavage of restriction sites included in the linker regions. In some instances, the annealing efficiency of parts and vector through the digested single stranded homologous regions can be increased by using a crowding agent such as polyethylene glycol and/or by including a single strand binding protein (*e.g.,* RecA, E. coli SSB, T7 SSB, T4 gene 32 protein).

The annealed fragments may then either be transformed directly into E. coli or optionally be treated with a ligase prior to transformation. Where it may be difficult to control exonuclease activity, a polymerase (*e.g.,* a Taq polymerase in the presence of dNTPs) may be added for gap repair after exonuclease treatment and prior to ligation and/or transformation. In some instances, exonuclease-based assembly protocols may be used to assemble between 2 and 10 fragments into a target vector in directed order, wherein each fragment may be between 200 and 600 bp in length. In some instances the desired nucleic acid molecule obtained with exonuclease-based assembly protocols may be up to 15 kb in length.

Other methods of seamless nucleic acid assembly include those described in U.S. Patent Publication Nos. 2010/0062495; 2007/0292954; 2003/0152984; 2006/0115850; 2010/184187; 2016/0122792; and in U.S. Patents Nos. 6,083,726; 6,110,668; 5,624,827; 6,521,427; 5,869,644; 6,495,318; 7,575,860; 8,815,600; 8,968,999; 9,951,327 and 9,447,445.

A method for the isothermal assembly of nucleic acid molecules is set out in U.S. Patent Publication No. 2012/0053087. In one aspect of this method, nucleic acid molecules for assembly are contacted with a thermolabile protein with exonuclease activity (*e.g.,* T5 polymerase) and optionally, a thermostable polymerase, and/or a thermostable ligase under conditions where the exonuclease activity decreases with time (*e.g.,* 50°C). The exonuclease "chews back" one strand of the nucleic acid molecules and, if there is sequence complementarity, nucleic acid molecules will anneal with each other. In one example, a thermostable polymerase may be used to fill in gaps and a thermostable ligase may be provided to seal nicks.

In another example, multiple single-stranded nucleic acid fragments with overlapping termini can be annealed in directed order wherein the termini of 5' and 3' fragments overlap the 5' and 3' termini of a linearized target vector. Following overlap extension PCR the assembly product may be directly used to transform a host cell and gaps and nicks will be repaired *"in vivo"* by endogenous enzymatic activities of the transformed cell as described for example in PCT application No. PCT/EP2018/067575.

Single-stranded binding proteins, such as T4 gene 32 protein and RecA, as well as other nucleic acid binding or recombination proteins known in the art, may be included, for example, to facilitate the annealing of nucleic acid molecules.

In some instances, standard ligase based joining of partially and fully assembled nucleic acid fragments may be employed. For example, assembled nucleic acid molecule may be generated with restriction enzyme sites near their termini. These nucleic acid molecules may then be treated with one of more suitably restriction enzymes to generate, for example, either one or two "sticky ends". Flanking cleavage sites may be designed to be cut by any suitable type of nuclease, such as type II or type IIs restriction enzymes, nickases or other types of engineered nucleases (*e.g.,* a Cas protein and a guide RNA (gRNA) (gRNA-Cas complex), a zinc finger nuclease, or a Transcription Activator-Like Effector Nuclease (TALEN) etc.) to produce overhangs that are compatible with termini or overhangs of a linearized or digested target vector and/or another nucleic acid fragment. These sticky end molecules may then be introduced into a vector by standard restriction enzyme-ligase methods. In instances where the insert nucleic acid molecules have only one sticky end, ligases may be used for blunt end ligation of the "non-sticky" terminus.

Constructs of larger sizes may also be obtained using for example, type IIs restriction site mediated assembly methods to simultaneously and directionally assemble multiple fragments (*e.g.,* two, three, five, eight, ten, etc.) in a single reaction. One suitable cloning system is referred to as Golden Gate cloning which is described in Engler et al. ("A One Pot, One Step, Precision Cloning Method with High Throughput Capability") PLoS One, 2008 and set out in various forms in U.S Patent Publication No. 2010/0291633 and PCT Publication WO 2010/040531.

Golden Gate assembly is a one-tube efficient cloning method based on type IIs restriction enzymes (such as *BsmBI, BbsI, Bsa*I, *AarI, SapI, BtgZI,* etc.) that cleave outside their recognition sites and typically leave 4-base overhangs. Unlike standard type II restriction enzymes (*e.g. BamHI, EcoRI* etc.) these type IIs enzymes cut DNA outside of their recognition sites and therefore can create non-palindromic overhangs. Since 256 potential overhang sequences are possible, multiple nucleic acid fragments can be assembled in directed order by using defined combinations of overhang sequences. Using this approach fragments can be assembled in a sequence-oriented and scar-less manner (*e.g*. upon removal of flanking tags). Also, because the final assembly product does not have a type IIs restriction enzyme recognition site, correctly-ligated nucleic acid products cannot be cleaved again by the restriction enzyme, thereby leading to accumulation of the desired assembly product.

In one example, following selective retrieval two or more (*e.g.* 2, 3, 4, 5, 6, 7, 8, 9 or 10 etc.) nucleic acid fragments comprising terminal type IIs restriction enzyme recognition sites may be combined (optionally in the presence of a target vector carrying the same type IIs restriction enzyme recognition sites) and cleaved with respective one or more type IIs restriction enzymes to generate compatible single-stranded overhangs between the fragments (and optionally between the fragments and the termini of the vector). The digested fragments (and vector) are then assembled under suitable conditions in the presence of a ligase (such as *e.g.* a T4 ligase, a T3 ligase, a T7 DNA ligase, *E. coli* DNA ligase, Taq ligase etc. or any combinations thereof). The reaction mixture may also comprise a ligase *(e.g.,* a T4 DNA ligase or a Taq ligase) for ligation of the assembled parts. Optionally, correctly assembled parts ligated together in a closed circle may further be treated with an exonuclease (*e.g.,* T5 exonuclease) to degrade all noncircular nucleic acid fragments, leaving intact only the complete circular ligation products for subsequent transformation.

It has been demonstrated that an exonuclease treatment prior to transformation increases transformation efficiency by removing unwanted non-circular nucleic acid molecules from the sample (such as cleaved vector backbone, partial assembly products, cleaved parts etc.) which may compete with the circular ligation products in transformation. Without an exonuclease treatment the correct ligation product would need to be gel--purified before proceeding. The combination of Golden Gate digestion-ligation and exonuclease treatment therefore reduces the overall hands-on time required for fragment assembly. In some instances type IIs-based assembly methods may be used to assemble up to 10 parts having sizes of between about 200 and about 600 base pairs in directed order. The reaction mixture comprising the assembled construct may then optionally be heated (*e.g.,* 10 min. at 65°C) to inactivate any residual enzyme activity and may then be transformed into *E. coli.*

Positive clones of assembled nucleic acid can be selected according to standard procedures. Alternatively, the assembled construct may be combined with another assembled construct in a further hierarchical assembly reaction to generate even longer constructs.

The various assembly methods disclosed above can be applied to efficiently assemble between about 2 and about 20 or between about 4 and about 10 parts depending on the overall size of the assembled parts. In some instances the assembly methods may be used to assemble desired nucleic acid molecules of between about 3,000 and about 6,000 base pairs in length or between about 5,000 and about 15,000 base pairs in length.

Parameters that may be varied to optimize efficiency of assembly methods described herein include the concentration of nucleic acid fragments present and the ratio of these nucleic acids. In many instances, the nucleic acid concentration will be adjusted in combination with the concentration of other components, such as enzymatic activities (*e.g.,* a nuclease, an enzyme with ligase activity, a polymerase etc.). Further, the ratio of nucleic acid fragments within the assembly mixture may vary for particular applications and depending on the length of the nucleic acid fragments. For example, longer nucleic acids (*e.g.* a target vector) may be present at a lower concentration than shorter nucleic acids (typically the fragment(s) to be inserted).

Assembled nucleic acid molecules may also include functional elements which confer desirable properties. These elements may either be provided by the plurality of oligonucleotides or nucleic acid fragments or by a target vector. Examples of such elements include origins of replication, long terminal repeats, resistance markers (such as antibiotic resistance genes), selectable markers and antidote coding sequences (*e.g.,* ccdA coding sequences for counter-acting toxic effects of ccdB), promoters, enhancers, polyadenylation signal coding sequences, 5' and 3' UTRs and other components suitable for the particular use(s) of the nucleic acid molecules (*e.g.,* enhancing mRNA or protein production efficiency). In examples where nucleic acid molecules are assembled to form an operon, the assembled nucleic acid products will often contain promoter and terminator sequences. Furthermore, assembled nucleic acid molecules may contain multiple cloning sites, such as, *e.g.,* type II or type IIs cleavage sites and/or GATEWAY^{®} recombination sites, as well as other sites for the connection of nucleic acid molecules to each other.

In instances were multi-fragment assembly comprises PCR-based assembly reactions, PCR reactions may be performed using one of the high fidelity polymerases disclosed above, optionally having 3'-5' exo proofreading activity to limit the amount of errors introduced during PCR. Following higher order assembly and optionally cloning into a target vector, the assembly product may be used to transform suitable competent host cells such as, *e.g.,* a common E. coli strain according to standard protocols. The skilled person can select suitable host cells depending on construct size and nucleotide composition, plasmid copy number, selection criteria etc. Useful strains are available through the American Type Culture Collection (http://www.atcc.org) and the E. coli Genetic Stock Center at Yale (cgsc.biology.yale.edu), as well as from commercial suppliers such as Agilent, Promega, Merck, Thermo Fisher Scientific, and New England Biolabs, respectively.

In many instances, nucleic acid molecules prepared by methods of the disclosure will be replicable. Further, many of these replicable nucleic acid molecules will be circular (*e.g.*, plasmids). Replicable nucleic acid molecules, regardless of whether they are circular, will generally be formed from the assembly of two or more (*e.g.,* three, four, five, eight, ten, twelve, etc.) nucleic acid fragments. In some instances, methods of the disclosure employ selection based upon the reconstitution of one or more (*e.g*., two, three, four, etc.) selection marker or one or more (*e.g.*, two, three, four, etc.) origin of replication resulting from the linking of different nucleic acid fragments. Further selection may result from the formation of a circular nucleic acid molecule, in instances where circularity is required for replication.

Functional elements may be used to select for correctly assembled constructs. For example, a target vector may provide one or more truncated and therefore inactive versions of certain elements (such as, *e.g.,* a portion of an origin of replication or a portion of a selection marker) which need to be completed by flanking sequences provided by complementary oligonucleotides during the assembly procedure. Such positive selection approach which renders only clones comprising correctly assembled functional elements is described in Baek et al., "Positive selection improves the efficiency of DNA assembly", Analytical Biochemistry 476 (2015), p. 1-4. In one example, the disclosure includes methods for selecting assembled nucleic acid molecules through a combination of the generation of replicable vectors (*e.g.*, re-circularized vectors) and one or more selectable marker. For example, some of the nucleic acid molecules to be assembled into a target vector may contain one or more resistance markers, one or more selectable markers or have functionalities that are otherwise required for replication (*e.g*., contain an origin of replication).

Larger nucleic acid molecules may also be assembled *in vivo.* In *in vivo* assembly methods, a mixture of all of the fragments to be assembled is often used to transfect the host cell using standard transfection techniques. The ratio of the number of molecules of fragments in the mixture to the number of cells in the culture to be transfected should be high enough to permit at least some of the cells to take up more molecules of fragments than there are different fragments in the mixture. Thus, in most instances, the higher the efficiency of transfection, the larger number of cells will be present which contain all of the nucleic acid fragments required to form the final desired assembly product. Technical parameters along these lines are set out in U.S. Patent Publication No. 2009/0275086 A1.

Large nucleic acid molecules are relatively fragile and, thus, shear readily. One method for stabilizing such molecules is by maintaining them intracellularly. Thus, in some aspects, the disclosure involves the assembly and/or maintenance of large nucleic acid molecules in host cells. Large nucleic acid molecules will typically be 20 kb or larger (*e.g.,* larger than 25 kb, larger than 35 kb, larger than 50 kb, larger than 70 kb, larger than 85 kb, larger than 100 kb, larger than 200 kb, larger than 500 kb, larger than 700 kb, larger than 900 kb, etc.).

As suggested above, one group of organisms known to perform homologous recombination fairly efficient is yeasts. Thus, host cells used in the practice of the disclosure may be yeast cells (*e.g*., Saccharomyces cerevisiae, Schizosaccharomyces pombe, Pichia, pastoris, etc.).

Exemplary of the yeast host cells are yeast strain VL6-48N, developed for high transformation efficiency parent strain: VL6-48 (ATCC Number MYA-3666TM)), the W303a strain, the MaV203 strain (Thermo Fisher Scientific, cat. no. 11281-011), and recombinationdeficient yeast strains, such as the RAD54 gene-deficient strain, VL6-48- Δ 54G (MAT*α* his3- Δ 200 trp1- Δ 1 ura3-52 lys2 ade2-101 met14 rad54- Δ 1::kanMX), which can decrease the occurrence of a variety of recombination events in yeast artificial chromosomes (YACs).

Once nucleic acid molecules are assembled and optionally error-corrected, their sequences may be verified to confirm that "junction" sequences are correct and that no other nucleotide sequence "errors" are located within assembled nucleic acid molecules.

Gene assembly workflows are typically completed by identifying a clone comprising the desired polynucleotide (referred to as "Clone Selection" in FIG. 1). For this purpose assembled polynucleotides are often transformed into *E. coli* followed by several hours (often overnight) of incubation to allow for growth of transformed bacteria to colonies of visible size. Colony PCR may then be performed to identify clones comprising correctly assembled polynucleotides having the desired length and isolated clones may further be sequenced (*e.g*., by Sanger sequencing techniques or next generation sequencing as outlined above) to verify the sequence of a desired nucleic acid product.

Methods described herein that combine high throughput oligonucleotide synthesis followed by intelligent pooling for efficient multiplex nucleic acid fragment assembly and processing provide optimal tools for library production and parallel testing of multiple genetic variants. This can include the evaluation of several natural homologs, the systematic screening of protein variants with site saturation libraries (comprising one amino acid substitution at a time), or the selection of best candidates from complex nucleic acid libraries with ambiguities at several nucleotide positions. All these approaches have in common, that it requires specimen of genetic material (*e.g*., an ORF) which are mostly similar to each other but comprise defined differences. The disclosed methods allow for exact synthesis of a gene as designed *in silico* including bundles of distinct nucleic acid variants and also synthetic DNA libraries for directed evolution.

### Example

To demonstrate multiplex assembly of six different nucleic acid fragments from overlapping oligonucleotides and subsequent multiplex amplification of the fragments with fragment-specific primer pairs in a single reaction compartment, nucleic acid sequences for 864 fragments with lengths from 283 bp to 359 bp were designed. The fragments were grouped for multiplex assembly taking fragment harmony criteria (GC content, length and fragment similarity) into account. Grouping of the 864 fragments resulted in a total of 144 pools with six fragments per pool. Furthermore, each fragment was designed with universal linker regions of 18 base pairs flanking both fragment ends and fragment-specific primer binding sites of 17 base pairs flanking the linker regions. Every pair of 5'- and 3'- fragment-specific primer binding sites (f1/f1' - f6/f6', respectively) was only assigned to one fragment per pool such that a specific primer pair could be used to amplify only that specific fragment in each pool. Fragment-specific primer sequences used in this example are listed in Table 2:

| Table 2 | | |
|---|---|---|
| Primer | Sequence | SEQ ID NO.: |
| f1 | 5'-CTAGCCCGCCCCGTATG-3' | 3 |
| f1' | 5'-CACTGACGCTTACGCCC-3' | 4 |
| f2 | 5'-TCAAAGGCCGCATCATC-3' | 5 |
| f2' | 5'-ATAGTTCCTTGGCGCGG-3' | 6 |
| f3 | 5'-TCGGGCGAGACACTACC-3' | 7 |
| f3' | 5'-AGTGATTCCGCTCCCCT-3' | 8 |
| f4 | 5'-GCGCCGCACCATTTTCT-3' | 9 |
| f4' | 5'-GTCAATCGGTCATCGCC-3' | 10 |
| f5 | 5'-CGGCCCAGGGAGTATTA-3' | 11 |
| f5' | 5'-ACACCGCGAGAGTCACA-3' | 12 |
| f6 | 5'-GCTTCCGCGACCCACTA-3' | 13 |
| f6' | 5'-AGTCTGAGTGCGCCGGC-3' | 14 |

The assembly oligonucleotides for the 864 fragments were synthesized on beads in wells of a microfluidic chip using phosphoramidite chemistry. Each oligonucleotide sub-set comprising all assembly oligonucleotides for six defined nucleic acid fragments was selectively retrieved and processed and pooled into a well of a 384-well plate. The fragments were then assembled in multiplex assembly reactions as illustrated in FIG. 8B. In each pool, six double stranded nucleic acid fragments were assembled in a first PCR reaction from the multiple overlapping oligonucleotides.

Each well comprising an assembly reaction was then supplied with an amplification mix containing a proof read polymerase and an equimolar mix of the 12 fragment-specific amplification primers f1/f1'-f6/f6' and nucleic acid fragments were amplified in a second multiplex PCR reaction.

Amplified fragments from all 144 reaction compartments were then pooled, purified and prepared for sequencing. Successful assembly of the desired fragments was assessed using an Ion Torrent S5XL sequencer. 839 of the 864 fragments were identified by sequencing indicating correct multiplex assembly and processing. In 124 of the 144 pools, all fragments were assembled; in 16 pools, 5 of 6 fragments were assembled; in 3 pools, 4 of 6 fragments were assembled; and in 1 pool, 3 of 6 fragments were assembled. Table 3 shows the pool distribution of the 25 "missing" fragments (including assigned primer pairs) that were not detected by sequencing after the first assembly cycle.

| Table 3 | | | |
|---|---|---|---|
| Missing fragment | Pool No. | primer pair | Rescued |
| Frag105 | 04 | f1/f1' | YES |
| Frag16 | 14 | f2/f2' | |
| Frag694 | 23 | f2/f2' | YES |
| Frag790 | 24 | f6/f6' | YES |
| Frag838 | 25 | f3/f3' | YES |
| Frag92 | 27 | f5/f5' | |
| Frag696 | 30 | f3/f3' | YES |
| Frag12 | 34 | f1/f1' | YES |
| Frag65 | 35 | f2/f2' | YES |
| Frag206 | 35 | f3/f3' | YES |
| Frag176 | 36 | f4/f4' | YES |
| Frag185 | 36 | f5/f5' | YES |
| Frag107 | 39 | f1/f1' | YES |
| Frag79 | 39 | f2/f2' | YES |
| Frag88 | 39 | f3/f3' | YES |
| Frag279 | 42 | f4/f4' | |
| Frag262 | 50 | f1/f1' | |
| Frag550 | 54 | f2/f2' | YES |
| Frag333 | 58 | f3/f3' | YES |
| Frag46 | 61 | f2/f2' | |
| Frag83 | 86 | f1/f1' | YES |
| Frag56 | 100 | f1/f1' | YES |
| Frag64 | 116 | f2/f2' | YES |
| Frag685 | 116 | f5/f5' | YES |
| Frag250 | 142 | f2/f2' | YES |

To obtain the missing fragments, a second assembly cycle ("rescue cycle") was performed using only the 20 pools comprising the assembly oligonucleotides for the missing fragments. Whereas the first multiplex assembly PCR was performed as described above for the first reaction cycle, the second amplification PCR was performed using only the fragment-specific primer pairs (1-µM equimolar mixes of each primer pair) to amplify only the missing fragments in each pool (*e.g.,* using only primer pair f2/f2' for pool 14 from which only 1 fragment was missing and using primer pairs f1/f1', f2/f2' and f3/f3', respectively, for pool 39 from which 3 fragments were missing). Based on this approach, 20 of the 25 missing fragments were "rescued" in the second cycle as shown in Table 3, resulting in a total of 859 out of 864 fragments (= 99.4%) that were successfully assembled in 6-plex assembly reactions.

Throughout the description and claims or clauses of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise. "a", "an", and "the" include plural reference unless the context clearly dictates otherwise.

It will be appreciated that where an "about" is used prior to the temperatures, concentrations, amounts, times, numbers, ranges, coverage, etc. discussed in the present teachings, slight and insubstantial deviations are within the scope of the present teachings.

While specific embodiments of the present disclosure have been shown and described herein, it will be obvious to those skilled in the art that such embodiments are provided by way of example only. It is intended that the following claims define the scope of the invention.

All publications, patents, and patent applications mentioned in this specification include following patent documents: U.S. Patent Publication Nos. 2008/0281466; 2008/0113361; 2012/0156728; 2013/0203607; 2012/0283110; 2012/0322681; 2014/0141982; 2014/0155297; 2006/0127920; 2007/0231805; 2010/0216648; 2011/0124049; 2008/0145913; 2010/0062495; 2007/0292954; 2003/0152984; 2006/0115850; 2010/184187; 2016/0122792; 2012/0053087; 2010/0291633, 2009/0275086; 2013/0225421 and 2014/0080717; U.S Patent Nos. 4,458,066; 8,224,578; 7,164,992; 6,887,431; 7,347,975; 7,384,606; 6,472,184; 5,869,252; 7,947,477; 9,121,047; 7,790,418, 7,985,565, 8,143,008; 8,895,249; 7,399,590, 7,666,598, 8,637,253, 8,809,021; 9,057,097; 7,948,015; 8,173,368; 7,704,690; 7,838,210; 7,833,759; 5,580,759; 6,083,726; 6,110,668; 5,624,827; 6,521,427; 5,869,644; 6,495,318; 7,575,860; 8,815,600; 8,968,999; 9,951,327; 8,808,989; 7,948,015 and 9,447,445; PCT Publications: WO 2016/094512, WO 2013/049227, WO 2017/176541; WO 2017/223517; WO 2017/100283; WO 2013/158313; WO 2006/084131; WO 2012/044847; WO 2008/112683; WO 2016/094512; WO 2011/102802; WO 2010/040531; and PCT application No. PCT/EP2018/067575.

## Claims

1. A method for multiplexed assembly of two or more predefined nucleic acid fragments in multiple reaction compartments comprising the following steps:
(a) designing a plurality of oligonucleotide sequences together comprising the sequences of the two or more predefined nucleic acid fragments and synthesizing the oligonucleotides to obtain a plurality of single-stranded oligonucleotides,
(b) selectively retrieving a first and a second and optionally one or more further sub-set of the plurality of single-stranded oligonucleotides, wherein the oligonucleotides of each sub-set are components of two or more predefined nucleic acid fragments, and wherein all oligonucleotides belonging to one of the two or more predefined nucleic acid fragments share a region of sequence complementarity with at least one other oligonucleotide belonging to the same nucleic acid fragment,
(c) pooling the oligonucleotides of the first sub-set into a first reaction compartment, the second sub-set into a second reaction compartment and optionally pooling the one or more further sub-sets into one or more further reaction compartments, and
(d) simultaneously assembling under suitable conditions the two or more nucleic acid fragments in the first, second and optionally further reaction compartments,
wherein the designing the plurality of oligonucleotide sequences comprises conducting the following steps on a computer:
(i) dividing *in silico* a predefined nucleic acid sequence into a plurality of fragments,
(ii) generating *in silico,* multiple sets of oligonucleotide sequences for each fragment, wherein each set of oligonucleotide sequences comprises a plurality of forward sequences and a plurality of reverse sequences, wherein the forward and reverse sequences of each set are intended to hybridize in a predetermined order,
(iii) assigning *in silico* a score to each pair of oligonucleotide sequences within the multiple sets of oligonucleotide sequences generated *in silico,* wherein the score indicates the probability of hybridization between two oligonucleotides,
(iv) determining *in silico* a total score for each set of oligonucleotide sequences by (a) summing up the individual scores assigned to all pairs of oligonucleotide sequences which are intended to hybridize in the predetermined order and subtracting the individual scores assigned to all pairs of oligonucleotide sequences which are not intended to hybridize in the predetermined order, or by (b) selecting the maximum scores of all pairs of oligonucleotide sequences that are not intended to hybridize, and
(v) selecting for each fragment at least one set of oligonucleotide sequences from the plurality of generated sets of oligonucleotide sequences that together yield the highest total score according to step (iv) (a) or together yield the lowest total score according to step (iv) (b); or
wherein the designing the plurality of oligonucleotide sequences comprises conducting the following steps on a computer:
(i) generating *in silico,* multiple sets of oligonucleotide sequences for a predefined nucleic acid sequence, wherein each set of oligonucleotide sequences comprises a plurality of forward sequences and a plurality of reverse sequences, wherein forward and reverse sequences are intended to hybridize in a predetermined order,
(ii) assigning *in silico,* a score to each pair of oligonucleotide sequences within the multiple sets of oligonucleotide sequences generated in silico, wherein the score indicates the probability of hybridization between two oligonucleotides,
(iii) determining *in silico* a total score for each set of oligonucleotide sequences by (a) summing up the individual scores assigned to all pairs of oligonucleotide sequences which are intended to hybridize in the predetermined order and subtracting the individual scores assigned to all pairs of oligonucleotide sequences which are not intended to hybridize in the predetermined order, or by (b) selecting the maximum scores of all pairs of oligonucleotide sequences that are not intended to hybridize, and
(iv) dividing *in silico,* the predefined nucleic acid sequence into a plurality of oligonucleotide sets, each oligonucleotide set defining a fragment of the predefined nucleic acid sequence, wherein each oligonucleotide set is selected based on the highest total score determined according to step (iii) (a) or based on the lowest total score determined according to step (iii) (b).

2. The method of claim 1, further comprising:
assigning *in silico,* a score to each combination of selected sets of oligonucleotide sequences within the pool of selected sets, wherein the score indicates the probability of cross-hybridization between forward and/or reverse oligonucleotides of a first set of oligonucleotide sequences with forward and/or reverse oligonucleotides of a second set of oligonucleotide sequences, and
selecting two or more sets of oligonucleotide sequences with a desired score for simultaneous assembly of two or more fragments in the same reaction compartment.

3. The method of any previous claim, wherein pooling the sub-sets of oligonucleotides comprises: grouping the first, second and optionally further sub-sets of oligonucleotides to minimize cross-hybridization between oligonucleotides belonging to different nucleic acid fragments.

4. The method of any previous claim, wherein pooling the sub-sets of oligonucleotides comprises: grouping the first, second and optionally further sub-sets of oligonucleotides according to fragment GC content and/or fragment length and/or according to fragment similarity.

5. The method of claim 4, wherein the two or more nucleic acid fragments assembled in each reaction compartment do not deviate by more than 2%, more than 5%, more than 10%, more than 15% or more than 20% , more than 40% or more than 50% in length and/or wherein the two or more nucleic acid fragments assembled in each reaction compartment do not deviate by more than 1%, more than 2%, more than 3%, more than 4%, more than 5%, more than 10% or more than 20% in GC content .

6. The method of any previous claim, wherein the two or more nucleic acid fragments assembled in a reaction compartment are non-similar and wherein similarity of two nucleic acid fragments is determined based on a pairwise alignment of the fragment sequences, division of the aligned sequences into matching blocks and irrelevant blocks and counting of the irrelevant blocks, optionally wherein the number of irrelevant blocks is <6 or <5 or <4 or <3.

7. The method of any previous claims, wherein the pooling of oligonucleotides for assembling S fragments into Pₘₐₓ reaction compartments comprises:
(i) determining whether two fragments harmonize taking into account fragment length, fragment GC content and fragment similarity,
(ii) defining an undirected graph with nodes, wherein each node represents a fragment S and wherein an edge between two nodes is present if the two fragments do not harmonize;
(iii) coloring the nodes of the undirected graph with as few colors P as possible, wherein each color represents a reaction compartment and wherein each color is used at most Sₘₐₓ times,
(iv) determining the amount of used colors P;
(v) assigning the Sₘₐₓ fragments to Pₘₐₓ reaction compartments, wherein all fragments S with the same color are assigned to the same reaction compartment, and
(vi) pooling the oligonucleotides into the assigned reaction compartments.

8. The method of any previous claim, wherein each nucleic acid fragment is assembled from 3 to about 100 overlapping oligonucleotides.

9. The method of any previous claim, wherein the number of different oligonucleotides in a sub-set is between 6 and 200.

10. The method of any previous claim, wherein the number of nucleic acid fragments simultaneously assembled in two or more, reaction compartments is between about 2 and about 400, or between about 5 and about 200.

11. The method of any previous claim, wherein the total number of nucleic acid fragments assembled in the first, second and optionally further reaction compartments is between 50 and 500 or between 100 and 1,000.

12. The method of any previous claim, wherein at least one of the two or more nucleic acid fragments assembled in the first reaction compartment has a region with sequence complementarity to at least one of the two or more fragments assembled in the second or further reaction compartments.

13. The method of any previous claim, wherein the number of different reaction compartments is between 10 and 100 or between 50 and 500.

## Patentansprüche

1. Verfahren zur gemultiplexten Zusammenstellung von zwei oder mehreren vordefinierten Nukleinsäurefragmenten in multiplen Reaktionskompartimenten, das die folgenden Schritte umfasst:
(a) Entwerfen einer Vielzahl von Oligonukleotidsequenzen, die zusammen die Sequenzen der zwei oder mehr vordefinierten Nukleinsäurefragmente umfassen, und Synthetisieren der Oligonukleotide, um eine Vielzahl von einzelsträngigen Oligonukleotiden zu erhalten,
(b) selektives Entnehmen einer ersten und einer zweiten und gegebenenfalls einer oder mehrerer weiterer Untergruppe der Vielzahl von einzelsträngigen Oligonukleotiden, wobei die Oligonukleotide jeder Untergruppe Komponenten von zwei oder mehr vordefinierten Nukleinsäurefragmenten sind und wobei alle Oligonukleotide, die zu einem der zwei oder mehr vordefinierten Nukleinsäurefragmente gehören, eine Region von Sequenzkomplementarität mit mindestens einem anderen Oligonukleotid teilen, das zu dem gleichen Nukleinsäurefragment gehört,
(c) Zusammenführen der Oligonukleotide der ersten Untergruppe in ein erstes Reaktionskompartiment, der zweiten Untergruppe in ein zweites Reaktionskompartiment und gegebenenfalls Zusammenführen der einen oder der mehreren weiteren Untergruppen in ein oder mehrere weitere Reaktionskompartimente, und
(d) gleichzeitiges Assemblieren der zwei oder mehr Nukleinsäurefragmente unter geeigneten Bedingungen in dem ersten, zweiten und gegebenenfalls weiteren Reaktionskompartimenten,
wobei das Entwerfen der Vielzahl von Oligonukleotidsequenzen die Durchführung der folgenden Schritte auf einem Computer umfasst:
(i) Unterteilen *in silico* einer vordefinierten Nukleinsäuresequenz in eine Vielzahl von Fragmenten,
(ii) Erzeugen in silico von multiplen Sätze von Oligonukleotidsequenzen für jedes Fragment, wobei jeder Satz von Oligonukleotidsequenzen eine Vielzahl von Vorwärtssequenzen und eine Vielzahl von Rückwärtssequenzen umfasst, wobei die Vorwärts- und Rückwärtssequenzen jedes Satzes in einer vorgegebenen Reihenfolge hybridisieren sollen,
(iii) Zuweisen in silico einer Punktzahl für jedes Paar von Oligonukleotidsequenzen innerhalb multipler Sätze von Oligonukleotidsequenzen, die in silico erzeugt wurden, wobei die Punktzahl die Wahrscheinlichkeit der Hybridisierung zwischen zwei Oligonukleotiden angibt,
(iv) Bestimmen in silico einer Gesamtpunktzahl für jeden Satz von Oligonukleotidsequenzen durch (a) Aufsummieren der einzelnen Punktzahlen, die allen Oligonukleotidsequenzpaaren zugewiesen wurden, die in der vorgegebenen Reihenfolge hybridisieren sollen, und Subtrahieren der einzelnen Punktzahlen, die allen Oligonukleotidsequenzpaaren zugewiesen wurden, die nicht in der vorgegebenen Reihenfolge hybridisieren sollen, oder durch (b) Auswahl der maximalen Punktzahlen aller Oligonukleotidsequenzpaare, die nicht hybridisieren sollen, und
(v) Auswählen von mindestens einem Satz von Oligonukleotidsequenzen aus der Vielzahl der erzeugten Sätze von Oligonukleotidsequenzen für jedes Fragment, die zusammen die höchste Gesamtpunktzahl gemäß Schritt (iv) (a) ergeben oder zusammen die niedrigste Gesamtpunktzahl gemäß Schritt (iv) (b) ergeben; oder
wobei das Entwerfen der Vielzahl von Oligonukleotidsequenzen die Durchführung der folgenden Schritte auf einem Computer umfasst:
(i) Erzeugen in silico von multiplen Sätze von Oligonukleotidsequenzen für eine vorgegebene Nukleinsäuresequenz, wobei jeder Satz von Oligonukleotidsequenzen eine Vielzahl von Vorwärtssequenzen und eine Vielzahl von Rückwärtssequenzen umfasst, wobei die Vorwärts- und Rückwärtssequenzen in einer vorgegebenen Reihenfolge hybridisieren sollen,
(ii) Zuweisen in silico einer Punktzahl für jedes Paar von Oligonukleotidsequenzen innerhalb multipler Sätze von Oligonukleotidsequenzen, die in silico erzeugt wurden, wobei die Punktzahl die Wahrscheinlichkeit der Hybridisierung zwischen zwei Oligonukleotiden angibt,
(iii) Bestimmen *in silico* einer Gesamtpunktzahl für jeden Satz von Oligonukleotidsequenzen durch (a) Aufsummieren der einzelnen Punktzahlen, die allen Oligonukleotidsequenzpaaren zugewiesen wurden, die in der vorbestimmten Reihenfolge hybridisieren sollen, und Subtrahieren der einzelnen Punktzahlen, die allen Oligonukleotidsequenzpaaren zugewiesen wurden, die nicht in der vorbestimmten Reihenfolge hybridisieren sollen, oder durch (b) Auswahl der maximalen Punktzahlen aller Oligonukleotidsequenzpaare, die nicht hybridisieren sollen, und
(iv) Unterteilen *in silico* der vordefinierten Nukleinsäuresequenz in eine Vielzahl von Oligonukleotidsätzen, wobei jeder Oligonukleotidsatz ein Fragment der vordefinierten Nukleinsäuresequenz definiert, wobei jeder Oligonukleotidsatz basierend auf der höchsten Gesamtpunktzahl, die gemäß Schritt (iii) (a) bestimmt wurde, oder basierend auf der niedrigsten Gesamtpunktzahl, die gemäß Schritt (iii) (b) bestimmt wurde, ausgewählt wird.

2. Verfahren nach Anspruch 1, ferner umfassend:
Zuweisen *in silico* einer Punktzahl für jede Kombination ausgewählter Sätze von Oligonukleotidsequenzen innerhalb der Gruppe ausgewählter Sätze, wobei die Punktzahl die Wahrscheinlichkeit einer Kreuzhybridisierung zwischen Vorwärts- und/oder Rückwärts-Oligonukleotiden eines ersten Satzes von Oligonukleotidsequenzen mit Vorwärts- und/oder Rückwärts-Oligonukleotiden eines zweiten Satzes von Oligonukleotidsequenzen angibt, und Auswählen von zwei oder mehr Sätzen von Oligonukleotidsequenzen mit einer gewünschten Punktzahl für die gleichzeitige Zusammenstellung von zwei oder mehr Fragmenten im gleichen Reaktionskompartiment.

3. Verfahren nach einem der vorhergehenden Ansprüche, wobei das Zusammenführen der Untergruppen von Oligonukleotiden Folgendes umfasst: Gruppieren der ersten, zweiten und gegebenenfalls weiteren Untergruppen von Oligonukleotiden, um eine Kreuzhybridisierung zwischen Oligonukleotiden, die zu verschiedenen Nukleinsäurefragmenten gehören, zu minimieren.

4. Verfahren nach einem der vorhergehenden Ansprüche, wobei das Zusammenführen der Untergruppen von Oligonukleotiden Folgendes umfasst: Gruppieren der ersten, zweiten und gegebenenfalls weiteren Untergruppen von Oligonukleotiden gemäß dem GC-Gehalt des Fragments und/oder der Fragmentlänge und/oder gemäß der Fragmentähnlichkeit.

5. Verfahren nach Anspruch 4, wobei die zwei oder mehr Nukleinsäurefragmente, die in einem Reaktionskompartiment zusammengestellt wurden, in der Länge nicht um mehr als 2 %, mehr als 5 %, mehr als 10 %, mehr als 15 % oder mehr als 20 %, mehr als 40 % oder mehr als 50 % abweichen und/oder wobei die zwei oder mehr Nukleinsäurefragmente, die in jedem Reaktionskompartiment zusammengestellt wurden, beim GC-Gehalt nicht um mehr als 1 %, mehr als 2 %, mehr als 3 %, mehr als 4 % oder mehr als 5 %, mehr als 10 % oder mehr als 20 % abweichen.

6. Verfahren nach einem der vorhergehenden Ansprüche, wobei sich die zwei oder mehr Nukleinsäurefragmente, die in einem Reaktionskompartiment zusammengestellt wurden, nicht ähneln und wobei die Ähnlichkeit zweier Nukleinsäurefragmente basierend auf einer paarweisen Anordnung der Fragmentsequenzen, einer Unterteilung der angeordneten Sequenzen in übereinstimmende Blöcke und irrelevante Blöcke und einer Zählung der irrelevanten Blöcke bestimmt wird, wobei die Anzahl der irrelevanten Blöcke gegebenenfalls < 6 oder < 5 oder < 4 oder < 3 beträgt.

7. Verfahren nach einem der vorhergehenden Ansprüche, wobei das Zusammenführen von Oligonukleotiden zum Zusammenstellen von S Fragmenten in Pₘₐₓ Reaktionskompartimente Folgendes umfasst:
(i) Bestimmen, ob zwei Fragmente unter Berücksichtigung der Fragmentlänge, des Fragment-GC-Gehalts und der Fragmentähnlichkeit harmonieren,
(ii) Definieren eines ungerichteten Graphen mit Knoten, wobei jeder Knoten ein Fragment S darstellt und wobei ein Rand zwischen zwei Knoten vorhanden ist, wenn die zwei Fragmente nicht harmonieren;
(iii) Einfärben der Knoten des ungerichteten Graphen mit so wenig Farben P wie möglich, wobei jede Farbe ein Reaktionskompartiment darstellt und wobei jede Farbe höchstens Sₘₐₓ-mal verwendet wird,
(iv) Bestimmen der Menge von verwendeten Farben P;
(v) Zuweisen der Sₘₐₓ-Fragmente zu PₘₐₓReaktionskompartimenten, wobei alle Fragmente S mit der gleichen Farbe dem gleichen Reaktionskompartiment zugewiesen werden, und
(vi) Zusammenführen der Oligonukleotide in den zugewiesenen Reaktionskompartimenten.

8. Verfahren nach einem der vorhergehenden Ansprüche, wobei jedes Nukleinsäurefragment aus 3 bis etwa 100 überlappenden Oligonukleotiden assembliert ist.

9. Verfahren nach einem der vorhergehenden Ansprüche, wobei die Anzahl der verschiedenen Oligonukleotide in einer Untergruppe zwischen 6 und 200 beträgt.

10. Verfahren nach einem der vorhergehenden Ansprüche, wobei die Anzahl der Nukleinsäurefragmente, die gleichzeitig in zwei oder mehr Reaktionskompartimenten assembliert wurden, zwischen etwa 2 und etwa 400 oder zwischen etwa 5 und etwa 200 beträgt.

11. Verfahren nach einem der vorhergehenden Ansprüche, wobei die Gesamtzahl der Nukleinsäurefragmente, die im ersten, zweiten und gegebenenfalls weiteren Reaktionskompartimenten zusammengestellt wurden, zwischen 50 und 500 oder zwischen 100 und 1.000 beträgt.

12. Verfahren nach einem der vorhergehenden Ansprüche, wobei mindestens eines der zwei oder mehr Nukleinsäurefragmente, die im ersten Reaktionskompartiment zusammengestellt wurden, eine Region mit Sequenzkomplementarität zu mindestens einem der zwei oder mehr Fragmente, die im zweiten oder weiteren Reaktionskompartimenten zusammengestellt wurden, aufweist.

13. Verfahren nach einem der vorhergehenden Ansprüche, wobei die Anzahl der verschiedenen Reaktionskompartimente zwischen 10 und 100 oder zwischen 50 und 500 beträgt.

## Revendications

1. Procédé d'assemblage multiplexé de deux fragments d'acide nucléique prédéfinis ou plus dans des compartiments de réaction multiples, comprenant les étapes suivantes :
(a) concevoir une pluralité de séquences d'oligonucléotide comprenant ensemble, les séquences des deux fragments d'acide nucléique prédéfinis ou plus et la synthèse des oligonucléotides pour obtenir une pluralité d'oligonucléotides monocaténaires,
(b) récupérer sélectivement un premier et un deuxième et éventuellement un ou de plusieurs autres sous-ensembles de la pluralité d'oligonucléotides monocaténaires, où les oligonucléotides de chaque sous-ensemble sont des composants de deux fragments d'acide nucléique prédéfinis ou plus, et dans lequel tous les oligonucléotides appartenant à l'un parmi les deux fragments d'acide nucléique prédéfinis ou plus partagent une région de complémentarité de séquence avec au moins un autre oligonucléotide appartenant au même fragment d'acide nucléique,
(c) mettre en commun des oligonucléotides du premier sous-ensemble dans un premier compartiment de réaction, du deuxième sous-ensemble dans un deuxième compartiment de réaction et, le cas échéant, mettre en commun un ou plusieurs autres sous-ensembles dans un ou plusieurs autres compartiments de réaction, et
(d) assembler simultanément, dans des conditions appropriées, les deux fragments d'acide nucléique ou plus dans les premier, deuxième et éventuellement d'autres compartiments de réaction,
dans lequel la conception de la pluralité des séquences d'oligonucléotide comprend la réalisation des étapes suivantes sur un ordinateur :
(i) diviser *in silico* une séquence d'acide nucléique prédéfinie en une pluralité de fragments,
(ii) générer in silico, plusieurs ensembles de séquences d'oligonucléotide pour chaque fragment, dans lequel chaque ensemble de séquences d'oligonucléotide comprend une pluralité de séquences sens et une pluralité de séquences antisens, les séquences sens et antisens de chaque ensemble étant destinées à s'hybrider dans un ordre prédéterminé,
(iii) attribuer in silico, un score à chaque paire de séquences d'oligonucléotide au sein des multiples ensembles de séquences d'oligonucléotide générés in silico, le score indiquant la probabilité d'hybridation entre deux oligonucléotides,
(iv) déterminer in silico, un score total pour chaque ensemble de séquences d'oligonucléotide en (a) additionnant les scores individuels attribués à toutes les paires de séquences d'oligonucléotide, qui sont destinées à s'hybrider dans l'ordre prédéterminé et en soustrayant les scores individuels attribués à toutes les paires de séquences d'oligonucléotide, qui ne sont pas destinées à s'hybrider dans l'ordre prédéterminé, ou en (b) sélectionnant les scores maximaux de toutes les paires de séquences d'oligonucléotide, qui ne sont pas destinées à s'hybrider, et
(v) sélectionner, pour chaque fragment, au moins un ensemble de séquences d'oligonucléotide parmi la pluralité d'ensembles générés de séquences d'oligonucléotide, qui donnent ensemble le score total le plus élevé selon l'étape (iv) (a) ou donnent ensemble le score total le plus bas selon l'étape (iv) (b) ; ou
dans lequel la conception de la pluralité des séquences d'oligonucléotide comprend la réalisation des étapes suivantes sur un ordinateur :
(i) générer in silico, plusieurs ensembles de séquences d'oligonucléotide pour une séquence d'acide nucléique prédéfinie, dans lequel chaque ensemble de séquences d'oligonucléotide comprend une pluralité de séquences sens et une pluralité de séquences antisens, les séquences sens et antisens étant destinées à s'hybrider dans un ordre prédéterminé,
(ii) attribuer in silico, un score à chaque paire de séquences d'oligonucléotide au sein des multiples ensembles de séquences d'oligonucléotide générés in silico, le score indiquant la probabilité d'hybridation entre deux oligonucléotides,
(iii) déterminer *in silico*, un score total pour chaque ensemble de séquences d'oligonucléotide en (a) additionnant les scores individuels attribués à toutes les paires de séquences d'oligonucléotide, qui sont destinées à s'hybrider dans l'ordre prédéterminé et en soustrayant les scores individuels attribués à toutes les paires de séquences d'oligonucléotide, qui ne sont pas destinées à s'hybrider dans l'ordre prédéterminé, ou en (b) sélectionnant les scores maximaux de toutes les paires de séquences d'oligonucléotide, qui ne sont pas destinées à s'hybrider, et
(iv) diviser *in silico*, la séquence d'acide nucléique prédéfinie en une pluralité d'ensembles d'oligonucléotides, chaque ensemble d'oligonucléotides définissant un fragment de la séquence d'acide nucléique prédéfinie, dans lequel chaque ensemble d'oligonucléotides est sélectionné en fonction du score total le plus élevé déterminé selon l'étape (iii) (a) ou en fonction du score total le plus bas déterminé selon l'étape (iii) (b).

2. Procédé selon la revendication 1, comprenant en outre l'étape consistant à :
attribuer *in silico*, un score pour chaque combinaison d'ensembles sélectionnés de séquences d'oligonucléotide au sein de la mise en commun d'ensembles sélectionnés, le score indiquant la probabilité d'hybridation croisée entre les oligonucléotides sens et/ou antisens d'un premier ensemble de séquences d'oligonucléotide avec des oligonucléotides sens et/ou antisens d'un second ensemble de séquences d'oligonucléotide, et sélectionner deux ensembles de séquences d'oligonucléotide ou plus avec un score souhaité pour l'assemblage simultané de deux fragments ou plus dans le même compartiment de réaction.

3. Procédé selon l'une quelconque des revendications précédentes, dans lequel la mise en commun des sous-ensembles d'oligonucléotides comprend l'étape consistant à : regrouper les premier, deuxième et éventuellement d'autres sous-ensembles d'oligonucléotides afin de minimiser l'hybridation croisée entre les oligonucléotides appartenant à différents fragments d'acide nucléique.

4. Procédé selon l'une quelconque des revendications précédentes, dans lequel la mise en commun des sous-ensembles d'oligonucléotides comprend l'étape consistant à : regrouper les premier, deuxième et éventuellement d'autres sous-ensembles d'oligonucléotides selon la concentration en GC des fragments et/ou la longueur des fragments et/ou la similarité des fragments.

5. Procédé selon la revendication 4, dans lequel les deux fragments d'acide nucléique ou plus assemblés dans chaque compartiment de réaction ne diffèrent pas de plus de 2 %, plus de 5 %, plus de 10 %, plus de 15 % ou plus de 20 %, plus de 40 % ou plus de 50 % concernant la longueur et/ou dans lequel les deux fragments d'acide nucléique ou plus assemblés dans chaque compartiment de réaction ne diffèrent pas de plus de 1 %, plus de 2 %, plus de 3 %, plus de 4 %, plus de 5 %, plus de 10 % ou plus de 20 % concernant la concentration en GC.

6. Procédé selon l'une quelconque des revendications précédentes, dans lequel les deux fragments d'acide nucléique ou plus assemblés dans un compartiment de réaction ne sont pas similaires et dans lequel la similarité de deux fragments d'acide nucléique est déterminée sur la base de l'alignement par paires des séquences de fragment, de la division des séquences alignées en blocs appariés et en blocs sans rapport, et du comptage des blocs sans rapport, le nombre de blocs sans rapport étant éventuellement < 6 ou < 5 ou < 4 ou < 3.

7. Procédé selon l'une quelconque des revendications précédentes, dans lequel la mise en commun des oligonucléotides pour l'assemblage de S fragments dans Pₘₐₓ compartiments de réaction comprend les étapes suivantes :
(i) déterminer si deux fragments sont compatibles en tenant compte de la longueur des fragments, de la concentration en GC des fragments et de la similarité des fragments,
(ii) définir un graphe non orienté avec des nœuds, dans lequel chaque nœud représente un fragment S et dans lequel une arête entre deux nœuds est présente si les deux fragments ne sont pas compatibles ;
(iii) colorer les nœuds du graphe non orienté avec le moins de couleurs P possible, chaque couleur représentant un compartiment de réaction et chaque couleur étant utilisée au maximum Sₘₐₓ fois,
(iv) déterminer la quantité de couleurs P utilisées ;
(v) affecter les Sₘₐₓ fragments aux Pₘₐₓ compartiments de réaction, tous les fragments S ayant la même couleur étant affectés au même compartiment de réaction, et
(vi) mettre en commun les oligonucléotides dans les compartiments de réaction affectés.

8. Procédé selon l'une quelconque des revendications précédentes, dans lequel chaque fragment d'acide nucléique est assemblé à partir de 3 à environ 100 oligonucléotides qui se chevauchent.

9. Procédé selon l'une quelconque des revendications précédentes, dans lequel le nombre d'oligonucléotides différents dans un sous-ensemble se situe dans l'intervalle allant de 6 à 200.

10. Procédé selon l'une quelconque des revendications précédentes, dans lequel le nombre de fragments d'acide nucléique assemblés simultanément dans deux compartiments de réaction ou plus se situe dans l'intervalle allant d'environ 2 à environ 400, ou d'environ 5 à environ 200.

11. Procédé selon l'une quelconque des revendications précédentes, dans lequel le nombre total de fragments d'acide nucléique assemblés dans les premier, deuxième et éventuellement d'autres compartiments de réaction se situe dans l'intervalle allant de 50 à 500 ou de 100 à 1 000.

12. Procédé selon l'une quelconque des revendications précédentes, dans lequel au moins un des deux fragments d'acide nucléique ou plus assemblés dans le premier compartiment de réaction présente une région ayant une complémentarité de séquence avec au moins un des deux fragments ou plus assemblés dans le deuxième ou d'autres compartiments de réaction.

13. Procédé selon l'une quelconque des revendications précédentes, dans lequel le nombre de compartiments de réaction différents se situe dans l'intervalle allant de 10 à 100 ou de 50 à 500.
